# EUROPEAN PATENT APPLICATION

(11) **EP 2 394 998 A1**
(43) Date of publication of application: **14.12.2011**
(21) Application number: 10382155.9
(22) Date of filing: 31.05.2010
(51) Int. Cl.: C07D 401/12, C07D 401/14, A61K 31/501, A61P 29/00, A61P 11/00

(54) **3-(5-Amino-6-oxo-1,6-dihydropyridazin-3-yl)-biphenyl derivatives as PDE4 inhibitors**

(71) Applicant: Almirall, S.A., 08022 Barcelona (ES)
(72) Inventor: Gracia Ferrer, Jordi, 08980, Sant Feliu de Llobregat (ES); Pages Santacana, Lluis Miquel, 08980, Sant Feliu de Llobregat (ES)
(74) Representative: Elzaburu Marquez, Alberto

(57) **Abstract**

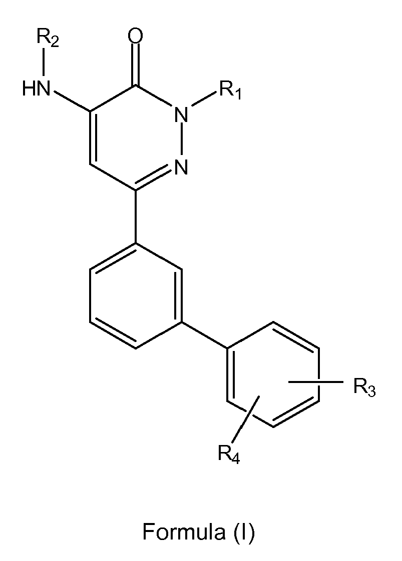

New pyridazin-3(2*H*)-one derivatives having the chemical structure of formula (I) are disclosed; as well as process for their preparation, pharmaceutical compositions comprising them and their use in therapy as inhibitors of the phosphodiesterase IV (PDE4).

## Description

The present invention relates to new therapeutically useful pyridazin-3(2H)-one derivatives, to processes for their preparation and to pharmaceutical compositions comprising them. These compounds are potent and selective inhibitors of phosphodiesterase 4 (PDE4) and are thus useful in the treatment, prevention or suppression of pathological conditions, diseases and disorders known to be susceptible of being improved by inhibition of PDE4.

Phosphodiesterases (PDEs) comprise a superfamily of enzymes responsible for the hydrolysis and inactivation of the second messenger cyclic adenosine monophosphate (cAMP) and cyclic guanosine monophosphate (cGMP). Eleven different PDE families have been identified to date (PDE1 to PDE11) which differ in substrate preference, catalytic activity, sensitivity to endogenous activators and inhibitors, and encoding genes.

The PDE4 isoenzyme family exhibits a high affinity for cyclic AMP but has weak affinity for cyclic GMP. Increased cyclic AMP levels caused by PDE4 inhibition are associated with the suppression of cell activation in a wide range of inflammatory and immune cells, including lymphocytes, macrophages, basophils, neutrophils, and eosinophils. Moreover, PDE4 inhibition decreases the release of the cytokine Tumor Necrosis Factor α (TNFα). The biology of PDE4 is described in several recent reviews, for example M. D. Houslay, P. Schafer, K. Y. Zhang, Drug Discov Today 2005, 10, 1503-19.

In view of these physiological effects, PDE4 inhibitors of varied chemical structures have been recently disclosed for the treatment or prevention of chronic and acute inflammatory diseases and other pathological conditions, diseases and disorders known to be susceptible to amelioration by inhibition of PDE4. See, for example, US 5449686, US 5710170, WO 98/45268, WO 99/06404, WO 01/57025, WO 01/57036, WO 01/46184, WO 97/05105, WO 96/40636, WO03/097613, US 5786354, US 5773467, US 5753666, US 5728712, US 5693659, US 5679696, US 5596013, US 5541219, US 5508300, US 5502072 or H. J. Dyke and J. G. Montana, Exp. Opin. Invest. Drugs 1999, 8, 1301-1325.

A few compounds having the capacity to selectively inhibit phosphodiesterase 4 are in active development. Examples of these compounds are roflumilast, GSK-256066, apremilast, tetomilast, rolipram, MK-0873 and oglemilast.

It is known that the clinical developement in man of early PDE4 inhibitors such as rolipram has been hampered by the appearance of side effects such as nausea and vomiting at therapeutic plasma levels (Curr. Pharm. Des. 2002, 8, 1255-96). The compounds described in the present invention are potent and selective PDE4 inhibitors. This property makes them useful for the treatment or prevention of pathological conditions or diseases such as respiratory diseases, skin disease, inflammatory diseases, diseases of the central or peripheral nervous system and cancer. These diseases include but are not limited to asthma, chronic obstructive pulmonary disease (COPD), allergic rhinitis, rheumatoid arthritis, multiple sclerosis, atopic dermatitis, psoriasis and inflammatory bowel disease.

PDE4 inhibitors, such as the compounds of the present invention referred to below, can also be used in combination with other drugs known to be effective in the treatment of these diseases. For example, they can be used in combination with bronchodilators such as β2-adrenergic agonists, antagonists of M3 muscarinic receptors or dual acting molecules combining β2 agonism with M3 antagonism, anti-allergics such as anti-histamines, mast cell stabilizers, CRTH2 antagonists, anti-inflammatory agents such as corticosteroids, LTD4 receptor antagonists, leukotriene synthesis inhibitors, COX inhibitors and PDE inhibitors, immunosuppressive agents such as calcineurin inhibitors, cyclosporin A, rapamycin, T-cell receptor blockers, B cell receptor blockers, and/or antiinfectives such as antibiotics, antimycotics or antiviral agents.

Accordingly, the present invention provides a compound of formula (I), or a pharmaceutically acceptable salt, or solvate, or N-oxide, or stereoisomer or deuterated derivate thereof: 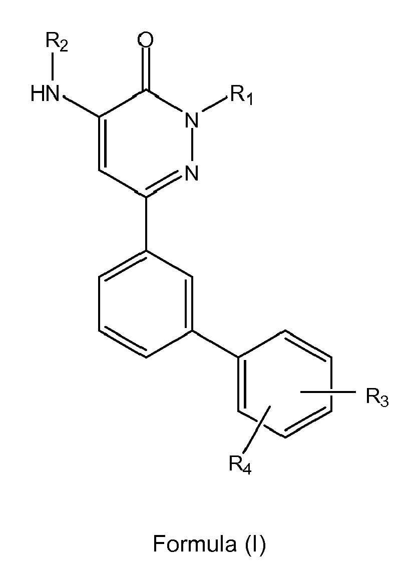 wherein
R₁ represents a linear or branched C₁-C₄ alkyl group;
R₂ represents a quinolyl or isoquinolyl group; wherein the quinolyl or isoquinolyl group is unsubstituted or substituted by one or more substituents selected from a halogen atom, a hydroxyl group, a cyano group, a linear or branched C₁-C₄ alkyl group, a C₁-C₄ haloalkyl group, a C₁-C₄ hydroxyalkyl group or a C₁-C₄ alkoxy group;
R₃ represents a -(CH₂)ₙC(O)OH group, a -(CH₂)ₙC(O)-R₅ group, a -(CH₂)ₙC(O)NH₂ group, a -(CH₂)ₙC(O)NH-(CH₂)ₘ-R₅ group, a -(CH₂)ₙC(O)NH-(CH₂)ₘ-C(O)R₆ group or a -(CH₂)ₙC(O)NH-(CH₂)ₘ-NR₅R₇ group;
R₄ represents a hydrogen atom, a hydroxyl group, a linear or branched C₁-C₄ alkyl group or a -(CH₂)ₙ,C(O)NH-(CH₂)ₘ,-R₈ group;
R₅ and R₈ independently represent a linear or branched C₁-C₄ alkyl group, a C₁-C₄ hydroxyalkyl group, a (C₁-C₄ alkoxy)(C₁-C₂ alkylene) group, a C₃-C₁₀ cycloalkyl group, a monocyclic or polycyclic C₅-C₁₄ aryl group or a 5- to 14- membered heterocyclyl group containing at least one heteroatom selected from O, S and N, wherein the cycloalkyl and heterocyclyl groups are unsubstituted or substituted by one or more substituents selected from a halogen atom, a hydroxyl group, a cyano group, an oxo group, a linear or branched C₁-C₄ alkyl group, a C₁-C₄ haloalkyl group, a C₁-C₄ hydroxyalkyl group, a C₁-C₄ alkoxy group, a (C₁-C₄ alkoxy)(C₁-C₂ alkylene) group, a carbamoyl group, a sulfamoyl group, an amino group, a mono-(C₁C₄ alkyl)amino group or a di-(C₁-C₄ alkyl)amino group; and the aryl groups are unsubstituted or substituted by one or more substituents selected from a halogen atom, a hydroxyl group, a cyano group, a linear or branched C₁-C₄ alkyl group, aC₁-C₄ haloalkyl group, a C₁-C₄ hydroxyalkyl group, a C₁-C₄ alkoxy group, a (C₁-C₄ alkoxy)(C₁-C₂ alkylene) group, a carbamoyl group, a sulfamoyl group, an amino group, a mono-(C₁-C₄ alkyl)amino group or a di-(C₁-C₄ alkyl)amino group;
R₆ represents a hydroxyl group, an amino group, a linear or branched C₁-C₄ alkyl group or a C₁-C₄ hydroxyalkyl group;
R₇ represents a linear or branched C₁-C₄ alkyl group or a C₁-C₄ hydroxyalkyl group;
n and n' are independently 0 or an integer from 1 to 10; and
m and m' are independently 0 or an integer from 1 to 10.

The invention further provides synthetic processes and intermediates described herein, which are useful for preparing said compounds.

The invention also provides a pharmaceutical composition comprising at least a compound of the invention and a pharmaceutically acceptable diluent or carrier.

The invention also provides a compound of the invention for use in the treatment of the human or animal body by therapy.

The invention is also directed to the compounds of the invention as described herein, for use in the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention also provides the use of the compounds of the invention as described herein, for the manufacture of a medicament for the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention is also directed to a method of treatment of a pathological condition or disease susceptible to amelioration by inhibiton of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease; comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more active ingredients selected from the group consisting of β2-adrenergic agonist, anti-cholinergic, an anti-allergic agent, an anti-inflammatory agent, an immunosuppressant and an anti-infective agent,
for simultaneous, separate or sequential use in the treatment of the human or animal body.

As used herein the term C₁-C₄ alkyl embraces linear or branched radicals having 1 to 4 carbon atoms. Examples include methyl, ethyl, n-propyl, i-propyl, n-butyl, sec-butyl and t-butyl radicals.

As used herein, the term C₁-C₄ haloalkyl group is an alkyl group, for example a C₁-C₄ or C₁-C₂ alkyl group, which is substituted by one or more, preferably 1, 2 or 3 halogen atoms. Preferably, said haloakyl group is chosen from -CCl₃ and -CF₃.

As used herein, the term C₁-C₄ hydroxyalkyl embraces linear or branched alkyl radicals having 1 to 4 carbon atoms, any one of which may be substituted by one or more, preferably 1 or 2, more preferably 1 hydroxyl radicals. Examples of such radicals include hydroxymethyl, hydroxyethyl, hydroxypropyl, 2,3-dihydroxypropyl and hydroxybutyl.

As used herein, the term C₁-C₄ alkoxy (or alkyloxy) embraces linear or branched oxy-containing radicals each having alkyl portions of 1 to 4 carbon atoms. Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, the term C₃-C₁₀ cycloalkyl embraces saturated monocyclic or polycyclic carbocyclic radicals having from 3 to 10 carbon atoms, preferably from 3 to 7 carbon atoms, more preferably from 3 to 6 carbon atoms. Polycyclic cycloalkyl radicals contain two or more fused cycloalkyl groups, preferably two cycloalkyl groups. Typically, polycyclic cycloalkyl radicals are selected from decahydronaphthyl (decalyl), bicyclo[2.2.2]octyl, adamantyl, camphyl or bornyl groups.Examples of monocyclic cycloalkyl groups include cyclopropyl, cyclobutyl, cyclopentyl, cyclohexyl, cycloheptyl, cyclooctyl, cyclononyl and cyclodecyl.

As used herein, the term C₅-C₁₄ aryl radical embraces typically a C₅-C₁₄, preferably C₆-C₁₄, more preferably C₆-C₁₀ monocyclic or polycyclic aryl radical such as phenyl, naphthyl, naphthalenyl, anthranyl and phenanthryl. Phenyl is preferred. A C₅-C₁₄ aryl radical is typically unsubstituted or substituted by 1, 2 or 3 substituents which may be the same or different. When a C₅-C₁₄ aryl radical carries 2 or more substituents, the substituents may be the same or different. Unless otherwise specified, the substituents on a C₅-C₁₄ aryl group are typically themselves unsubstituted.

As used herein, the term 5- to 14-membered heterocyclyl radical embraces typically a non-aromatic, saturated or unsaturated C₅-C₁₄ carbocyclic ring system, preferably C₅-C₁₀ carbocyclic ring system, more preferably C₅-C₆ carbocyclic ring system, in which one or more, for example 1, 2, 3 or 4 of the carbon atoms preferably 1 or 2 of the carbon atoms are replaced by a heteroatom selected from N, O and S. A heterocyclyl radical may be a single ring or two or more fused rings wherein at least one ring contains a heteroatom.

A 5- to 14-membered heterocyclyl radical is typically unsubstituted or substituted by 1, 2 or 3 substituents which may be the same or different. Where a 5- to 14-membered heterocyclyl radical carries 2 or more substituents, the substituents may be the same or different. Typically, the substituents on a 5 to 14-membered heterocyclyl radical are themselves unsubstituted.

Examples of 5- to 14-membered heterocyclyl radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, 4,5-dihydro-oxazolyl, 2-benzofuran-1 (3H)-one, 1,3-dioxol-2-one and 3-aza-tetrahydrofuranyl radicals.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

Typically when a cyclic radical is bridged by an alkylene or alkylenedioxy radical, the bridging alkylene radical is bonded to the ring at non-adjacent atoms.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclyl amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Typically, in the compound of formula (I) R₁ represents a methyl group or an ethyl group; preferably R₁ represents an ethyl group.

Typically, in the compound of formula (I) R₂ represents an unsubstituted quinolyl or isoquinolyl group.

Typically R₃ represents a -(CH₂)ₙC(O)OH group, a -(CH₂)ₙC(O)-R₅ group, a -(CH₂)ₙC(O)NH₂ group, a -(CH₂)ₙC(O)NH-(CH₂)ₘ-R₅ group, a -(CH₂)ₙC(O)NH-(CH₂)ₘ-C(O)R₆ group or a -(CH₂)ₙC(O)NH-(CH₂)ₘ-NR₅R₇ group.

Typically R₅ represents a linear or branched C₁-C₃ alkyl group, a C₁-C₃ hydroxyalkyl group, a C₃-C₇ cycloalkyl group, a phenyl group or a 5- to 10-membered heterocyclyl group containing 1, 2 or 3 heteroatoms selected from O, S and N, wherein: the cycloalkyl and heterocyclyl groups are unsubstituted or substituted by one or two substituents selected from a hydroxyl group, an oxo group, a linear or branched C₁-C₃ alkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group; and the phenyl groups are unsubstituted or substituted by one or two substituents selected from a hydroxyl group, a linear or branched C₁-C₃ alkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group.

Preferably R₅ represents a C₃-C₅ cycloalkyl group, a piperidinyl group, a morpholinyl group or a pyrrolidinyl group; wherein said cyclic groups are unsubstituted or substituted by one or two substituents selected from a methyl group, a methoxy group or a methoxy(methylene) group

Typically n is 0 or an integer from 1 to 3, preferably n is 0, 1 or 2, most preferably n is 0 or 1.

Typically m is 0 or an integer from 1 to 8, preferably 0 or an integer from 1 to 4, most preferably 0, 1 or 2.

Typically, R₆ represents a hydroxyl group or an amino group.

Typically, R₇ represents a linear or branched C₁-C₃ alkyl group or a C₁-C₃ hydroxyalkyl group.

Preferably, in the compound of formula (1) R₃ represents a -(CH₂)ₙC(O)OH group,
a -(CH₂)ₙC(O)-R₅ group, a -(CH₂)ₙC(O)NH₂ group,
a -(CH₂)ₙC(O)NH-(CH₂)ₘ-R₅ group, a -(CH₂)ₙC(O)NH-(CH₂)ₘ-C(O)R₆ group or a -(CH₂)ₙC(O)NH-(CH₂)ₘ-NR₅R₇ group; wherein
R₅ represents a linear or branched C₁-C₃ alkyl group, a C₁-C₃ hydroxyalkyl group, a C₃-C₇ cycloalkyl group, a phenyl group or a 5- to 10-membered heterocyclyl group containing 1, 2 or 3 heteroatoms selected from O, S and N,
wherein the cycloalkyl and heterocyclyl groups are unsubstituted or substituted by one or two substituents selected from a hydroxyl group, an oxo group, a linear or branched C₁-C₃ alkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group; and the phenyl groups are unsubstituted or substituted by one or two substituents selected from a hydroxyl group, a linear or branched C₁-C₃ alkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group;
R₆ represents a hydroxyl group, an amino group,
R₇ represents a linear or branched C₁-C₃ alkyl group or a C₁-C₃ hydroxyalkyl group;
n is 0 or an integer from 1 to 3; and
m is 0 or an integer from 1 to 8.

More preferably, in the compound of formula (I) R₃ represents a -(CH₂)ₙC(O)OH group, a -(CH₂)ₙC(O)NH₂ group, or a -(CH₂)ₙC(O)NH-(CH₂)ₘ-R₅ group, wherein n is 0 or an integer from 1 to 3; and m is 0 or an integer from 1 to 4; and wherein R₅ represents a C₃-C₅ cycloalkyl group, a piperidinyl group, a morpholinyl group or a pyrrolidinyl group; wherein said cyclic groups are unsubstituted or substituted by one or two substituents selected from a methyl group, a methoxy group or a methoxy(methylene) group.

Typically, R₄ represents a hydrogen atom, a hydroxyl group or a
-(CH₂)ₙ,C(O)NH-(CH₂)ₘ,-R₈ group, preferably a hydrogen atom, a hydroxyl group or a -C(O)NH-R₈ group.

Typically, R₈ represents a C₃-C₇ cycloalkyl group, wherein the cycloalkyl group is unsubstituted or substituted by one or two substituents selected from a hydroxyl group, a linear or branched C₁-C₃ alkyl group, a C₁-C₃ hydroxyalkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group R₈ is an unsubstituted C₃-C₇ cycloalkyl group. Preferably R₈ represents a cyclopropyl group, a cyclobutyl group, a cyclopentyl group, a cyclohexyl group or a cycloheptyl group. Most preferably R₈ represents a cyclopropyl group.

Typically, n' represents 0, 1 or 2, preferably 0.

Typically, m' represent 0, 1 or 2, preferably 0.

Preferably, in the compound of formula (I) R₄ represents a hydrogen atom, a hydroxyl group or a -(CH₂)ₙ,C(O)NH-(CH₂)ₘ,-R₈ group, wherein n' and m' are 0, 1 or 2, and wherein R₈ is a C₃-C₇ cycloalkyl group, wherein the cycloalkyl group is unsubstituted or substituted by one or two substituents selected from a hydroxyl group, a linear or branched C₁-C₃ alkyl group, a C₁-C₃ hydroxyalkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group; more preferably R₄ represents a hydrogen atom, a hydroxyl group or a -C(O)NH-R₈ group wherein R₈ is an unsubstituted C₃-C₇ cycloalkyl group; more preferably R₄ represents a hydrogen atom, a hydroxyl group or a -C(O)NH-R₈, wherein R₈ is a cyclopropyl group, a cyclobutyl group, a cyclopentyl group, a cyclohexyl group or a cycloheptyl group; most preferably R₄ represents a hydrogen atom, a hydroxyl group or a -C(O)NH-R₈ group, wherein R₈ is a cyclopropyl group.

Typically, in the compound of formula (I) R₃ is at the position 3 or position 4 of the phenyl group.

Typically, in the compound of formula (I) R₄ is at the position 3 or position 5 of the phenyl group.

Typically, when the cycloalkyl and heterocyclyl moieties which R₅ may represent are substituted by an oxo group, said cycloalkyl and heterocyclyl moieties are only substituted by one such oxo group.

For the avoidance of doubt, it is to be understood that (C₁-C₄ alkoxy)(C₁-C₂ alkylene) groups are typically bonded to the residue of the compound of formula (I) via the alkylene portions of the (C₁-C₄ alkoxy)(C₁-C₂ alkylene) group.

In a particularly preferred embodiment, in the compound of formula (I) or a pharmaceutically acceptable salt, or solvate, or N-oxide, or stereoisomer or deuterated derivate thereof:
R₁ represents an ethyl group;
R₂ represents an unsubstituted quinolyl or isoquinolyl group;
R₃ represents a -(CH₂)ₙC(O)OH group, a -(CH₂)ₙC(O)-R₅ group, a -(CH₂)ₙC(O)NH₂ group, a -(CH₂)ₙC(O)NH-(CH₂)ₘ-R₅ group, a -(CH₂)ₙC(O)NH-(CH₂)ₘ-C(O)R₆ group or a -(CH₂)ₙC(O)NH-(CH₂)ₘ-NR₅R₇ group; wherein n is 0 or an integer from 1 to 3, and m is 0 or an integer from 1 to 8; and wherein
   R₅ represents a methyl group, a C₁-C₂ hydroxyalkyl group, a C₃-C₅ cycloalkyl group, a phenyl group, a piperidinyl group, a piperidin-4-yl group, a morpholinyl group, a pyrrolidinyl group, a pyrrolidin-2-yl group or a pyrrolidin-2-one-yl group;
   wherein the C₃-C₅ cycloalkyl, piperidinyl, piperidin-4-yl, morpholinyl group, a pyrrolidinyl, a pyrrolidin-2-yl or a pyrrolidin-2-one-yl groups are unsubstituted or substituted by one or two substituents selected from a hydroxyl group, an oxo group, a methyl group, a methoxy group, a methoxy(methylene) group, a carbamoyl group, a sulfamoyl group or a dimethyl amino group; and the phenyl groups are unsubstituted or substituted by one or two substituents selected from a hydroxyl group, a methyl group, a methoxy group, a methoxy(methylene) group, a carbamoyl group, a sulfamoyl group or a di-methyl amino group;
   R₆ represents a hydroxyl group or an amino group,
   R₇ represents a methyl group or a C₁-C₂ hydroxyalkyl group;
R₄ represents a hydrogen atom, a hydroxyl group or a -C(O)NH-cyclopropyl group;

Particular individual compounds of the invention include:
3'-[1-ethyl-6-oxo-5-(quinolin-5-ylamino)-1,6-dihydropyridazin-3-yl]biphenyl-3-carboxylic acid;
3'-[1-ethyl-5-(isoquinolin-4-ylamino)-6-oxo-1,6-dihydropyridazin-3-yl]biphenyl-3-carboxylic acid;
3'-[1-ethyl-5-(isoquinolin-4-ylamino)-6-oxo-1,6-dihydropyridazin-3-yl]biphenyl-3-carboxamide;
3'-[1-ethyl-6-oxo-5-(quinolin-5-ylamino)-1,6-dihydropyridazin-3-yl]biphenyl-3-carboxamide;
3'-[1-ethyl-5-(isoquinolin-4-ylamino)-6-oxo-1,6-dihydropyridazin-3-yl]biphenyl-4-carboxylic acid;
{3'-[1-ethyl-6-oxo-5-(quinolin-5-ylamino)-1,6-dihydropyridazin-3-yl]biphenyl-4-yl}acetic acid;
3'-{1-ethyl-5-[(1-oxidoquinolin-5-yl)amino]-6-oxo-1,6-dihydropyridazin-3-yl}biphenyl-3-carboxamide;
{3'-[1-ethyl-5-(isoquinolin-4-ylamino)-6-oxo-1,6-dihydropyridazin-3-yl]biphenyl-3-yl}acetic acid;
3'-[1-ethyl-6-oxo-5-(quinolin-5-ylamino)-1,6-dihydropyridazin-3-yl]biphenyl-4-carboxylic acid;
{3'-[1-ethyl-6-oxo-5-(quinolin-5-ylamino)-1,6-dihydropyridazin-3-yl]biphenyl-3-yl}acetic acid;
3'-f 1 -ethyl-5-[(l -oxidoquinolin-5-yl)amino]-6-oxo-1,6-dihydropyridazin-3-yllbiphenyl-3-carboxylic acid;
N-[2-(dimethylamino)ethyl]-3'-[1-ethyl-6-oxo-5-(quinolin-5-ylamino)-1,6-dihydropyridazin-3-yl]biphenyl-3-carboxamide;
N-cyclopropyl-3'-[1-ethyl-6-oxo-5-(quinolin-5-ylamino)-1,6-dihydropyridazin-3-yl]biphenyl-3-carboxamide;
N-cyclopropyl-3'-[1-ethyl-5-(isoquinolin-4-ylamino)-6-oxo-1,6-dihydropyridazin-3-yl]biphenyl-3-carboxamide;
3'-[1-ethyl-6-oxo-5-(quinolin-5-ylamino)-1,6-dihydropyridazin-3-yl]-N-(2-morpholin-4-ylethyl)biphenyl-3-carboxamide;
3'-[1-ethyl-6-oxo-5-(quinolin-5-ylamino)-1,6-dihydropyridazin-3-yl]-N-(2-piperidin-1-ylethyl)biphenyl-3-carboxamide;
3'-[1-ethyl-5-(isoquinolin-4-ylamino)-6-oxo-1,6-dihydropyridazin-3-yl]-N-(2-morpholin-4-ylethyl)biphenyl-3-carboxamide;
3'-[1-ethyl-5-(isoquinolin-4-ylamino)-6-oxo-1,6-dihydropyridazin-3-yl]-N-(2-piperidin-1-ylethyl)biphenyl-3-carboxamide;
N-cyclobutyl-3'-[1-ethyl-5-(isoquinolin-4-ylamino)-6-oxo-1,6-dihydropyridazin-3-yl]biphenyl-3-carboxamide;
3'-[1-ethyl-5-(isoquinolin-4-ylamino)-6-oxo-1,6-dihydropyridazin-3-yl]-N-(2-morpholin-4-ylethyl)biphenyl-4-carboxamide;
N-cyclopropyl-3'-[1-ethyl-6-oxo-5-(quinolin-5-ylamino)-1,6-dihydropyridazin-3-yl]-5-hydroxybiphenyl-3-carboxamide;
3'-[l -ethyl-5-(isoquinolin-4-ylamino)-6-oxo-1,6-dihydropyridazin-3-yl]-N-(2-piperidin-1 - ylethyl)biphenyl-4-carboxamide;
N-cyclobutyl-3'-[1-ethyl-5-(isoquinolin-4-ylamino)-6-oxo-1,6-dihydropyridazin-3-yl]biphenyl-4-carboxamide;
N-cyclopentyl-3'-[1-ethyl-5-(isoquinolin-4-ylamino)-6-oxo-1,6-dihydropyridazin-3-yl]biphenyl-4-carboxamide;
N-cyclobutyl-3'-[1-ethyl-6-oxo-5-(quinolin-5-ylamino)-1,6-dihydropyridazin-3-yl]biphenyl-3-carboxamide;
3'-[1-ethyl-6-oxo-5-(quinolin-5-ylamino)-1,6-dihydropyridazin-3-yl]-N-(3-hydroxybenzyl)biphenyl-3-carboxamide;
N-cyclopentyl-3'-[1-ethyl-6-oxo-5-(quinolin-5-ylamino)-1,6-dihydropyridazin-3-yl]biphenyl-3-carboxamide;
1-({3'-[1-ethyl-6-oxo-5-(quinolin-5-ylamino)-1,6-dihydropyridazin-3-yl]biphenyl-3-yl}carbonyl)piperidine-4-carboxamide;
3'-[1-ethyl-6-oxo-5-(quinolin-5-ylamino)-1,6-dihydropyridazin-3-yl]-N-{7-[(2-hydroxyethyl)(methyl)amino]heptyl}biphenyl-3-carboxamide;
N-cyclopentyl-3'-[1-ethyl-5-(isoquinolin-4-ylamino)-6-oxo-1,6-dihydropyridazin-3-yl]biphenyl-3-carboxamide;
3'-[1-ethyl-5-(isoquinolin-4-ylamino)-6-oxo-1,6-dihydropyridazin-3-yl]-N-(3-hydroxybenzyl)biphenyl-3-carboxamide;
3'-[1-ethyl-5-(isoquinolin-4-ylamino)-6-oxo-1,6-dihydropyridazin-3-yl]-N-{7-[(2-hydroxyethyl)(methyl)amino]heptyl}biphenyl-3-carboxamide;
3-{3'-[1-ethyl-6-oxo-5-(quinolin-5-ylamino)-1,6-dihydropyridazin-3-yl]biphenyl-4-yl}propanoic acid;
3-{3'-[1-ethyl-6-oxo-5-(quinolin-5-ylamino)-1,6-dihydropyridazin-3-yl]biphenyl-3-yl}propanoic acid;
N-cyclopropyl-3'-[1-ethyl-5-(isoquinolin-4-ylamino)-6-oxo-1,6-dihydropyridazin-3-yl]-5-hydroxybiphenyl-3-carboxamide
3'-[1-ethyl-5-(isoquinolin-4-ylamino)-6-oxo-1,6-dihydropyridazin-3-yl]-N-(1-methylpiperidin-4-yl)biphenyl-3-carboxamide;
N-cyclopropyl-3-{3'-[1-ethyl-6-oxo-5-(quinolin-5-ylamino)-1,6-dihydropyridazin-3-yl]biphenyl-4-yl}propanamide;
N-cyclopropyl-3-{3'-[1-ethyl-6-oxo-5-(quinolin-5-ylamino)-1,6-dihydropyridazin-3-yl]biphenyl-3-yl}propanamide;
N-cyclopentyl-3'-[1-ethyl-6-oxo-5-(quinolin-5-ylamino)-1,6-dihydropyridazin-3-yl]biphenyl-4-carboxamide;
2-{3'-[1-ethyl-5-(isoquinolin-4-ylamino)-6-oxo-1,6-dihydropyridazin-3-yl]biphenyl-3-yl}-N-(2-pyrrolidin-1-ylethyl)acetamide;
2-{3'-[1-ethyl-6-oxo-5-( qui nolin-5-ylamino )-1 ,6-di hyd ropyridazin-3-yl]biphenyl-3-yl}-N-(2-pyrrolidin-1-ylethyl)acetamide;
3'-[1-ethyl-6-oxo-5-(quinolin-5-ylamino)-1,6-dihydropyridazin-3-yl]-N-(2-morpholin-4-ylethyl)biphenyl-4-carboxamide;
3'-[1-ethyl-6-oxo-5-(quinolin-5-ylamino)-1,6-dihydropyridazin-3-yl]-N-(2-piperidin-1-ylethyl)biphenyl-4-carboxamide;
N-(3,4-dimethoxybenzyl)-3'-[1-ethyl-5-(isoquinolin-4-ylamino)-6-oxo-1,6-dihydropyridazin-3-yl]biphenyl-3-carboxamide;
N-cyclobutyl-3'-[1-ethyl-6-oxo-5-(quinolin-5-ylamino)-1,6-dihydropyridazin-3-yl]biphenyl-4-carboxamide;
N-[4-(aminosulfonyl)benzyl]-3'-[1-ethyl-6-oxo-5-(quinolin-5-ylamino)-1,6-dihydropyridazin-3-yl]biphenyl-3-carboxamide;
3'-[1-ethyl-6-oxo-5-(quinolin-5-ylamino)-1,6-dihydropyridazin-3-yl]-N-[3-(2-oxopyrrolidin-1-yl)propyl]biphenyl-3-carboxamide;
3'-[1-ethyl-6-oxo-5-(quinolin-5-ylamino)-1,6-dihydropyridazin-3-yl]-N-[3-(2-oxopyrrolidin-1-yl)propyl]biphenyl-3-carboxamide;
N-({3'-[1-ethyl-6-oxo-5-(quinolin-5-ylamino)-1,6-dihydropyridazin-3-yl]biphenyl-3-yl}carbonyl)glycine;
-{3'-[1-ethyl-5-(isoquinolin-4-ylamino)-6-oxo-1,6-dihydropyridazin-3-yl]biphenyl-4-yl}-N-(2-pyrrolidin-1-ylethyl)acetamide;
2-{3'-[1-ethyl-6-oxo-5-(quinolin-5-ylamino)-1,6-dihydropyridazin-3-yl]biphenyl-4-yl}-N-(2-pyrrolidin-1-ylethyl)acetamide;
N-{2-[1-(dimethylamino)cyclopentyl]ethyl}-3'-[1-ethyl-6-oxo-5-(quinolin-5-ylamino)-1,6-dihydropyridazin-3-yl]biphenyl-3-carboxamide;
4-[({3'-[1-ethyl-6-oxo-5-(quinolin-5-ylamino)-1,6-dihydropyridazin-3-yl]biphenyl-3-yl}carbonyl)amino]piperidine-1-carboxamide;
2-{3'-[1-ethyl-6-oxo-5-(quinolin-5-ylamino)-1,6-dihydropyridazin-3-yl]biphenyl-4-yl}-N-(2-piperidin-1-ylethyl)acetamide;
3'-[1-ethyl-6-oxo-5-(quinolin-5-ylamino)-1,6-dihydropyridazin-3-yl]-N-{2-[(2R)-1-methylpyrrolidin-2-yl]ethyl}biphenyl-3-carboxamide;
6-(3'-{[4-(dimethylamino)piperidin-1-yl]carbonyl}biphenyl-3-yl)-2-ethyl-4-(quinolin-5-ylamino)pyridazin-3(2H)-one;
3'-[1-ethyl-6-oxo-5-(quinolin-5-ylamino)-1,6-dihydropyridazin-3-yl]-N-{2-[(2S)-1-methylpyrrolidin-2-yl]ethyl}biphenyl-3-carboxamide;
3-{3'-[1-ethyl-6-oxo-5-(quinolin-5-ylamino)-1,6-dihydropyridazin-3-yl]biphenyl-4-yl}-N-(2-piperidin-1-ylethyl)propanamide;
3-{3'-[1-ethyl-6-oxo-5-(quinolin-5-ylamino)-1,6-dihydropyridazin-3-yl]biphenyl-4-yl}-N-(1-methylpiperidin-4-yl)propanamide;
3-{3'-[1-ethyl-6-oxo-5-(quinolin-5-ylamino)-1,6-dihydropyridazin-3-yl]biphenyl-4-yl}-N-(2-morpholin-4-ylethyl)propanamide;
N,N'-dicyclopropyl-3'-[1-ethyl-6-oxo-5-(quinolin-5-ylamino)-1,6-dihydropyridazin-3-yl]biphenyl-3,5-dicarboxamide;
N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide;
N-cyclopropyl-3'-[1-ethyl-6-oxo-5-(quinolin-5-ylamino)-1,6-dihydropyridazin-3-yl]-N'-{2-[(2S)-2-(methoxymethyl)pyrrolidin-1-yl]ethyl}biphenyl-3,5-dicarboxamide;
and pharmaceutically acceptable salts, solvates, N-oxides or deuterated derivatives thereof.

Of outstanding interest are:
3'-[1-ethyl-6-oxo-5-(quinolin-5-ylamino)-1,6-dihydropyridazin-3-yl]biphenyl-3-carboxamide;
3'-[1-ethyl-5-(isoquinolin-4-ylamino)-6-oxo-1,6-dihydropyridazin-3-yl]biphenyl-4-carboxylic acid;
N-[2-(dimethylamino)ethyl]-3'-[1-ethyl-6-oxo-5-(quinolin-5-ylamino)-1,6-dihydropyridazin-3-yl]biphenyl-3-carboxamide;
N-cyclopropyl-3'-[1-ethyl-6-oxo-5-(quinolin-5-ylamino)-1,6-dihydropyridazin-3-yl]biphenyl-3-carboxamide;
3'-[1-ethyl-6-oxo-5-(quinolin-5-ylamino)-1,6-dihydropyridazin-3-yl]-N-(2-piperidin-1-ylethyl)biphenyl-3-carboxamide;
3'-[1-ethyl-5-(isoquinolin-4-ylamino)-6-oxo-1,6-dihydropyridazin-3-yl]-N-(2-morpholin-4-ylethyl)biphenyl-3-carboxamide;
N-cyclobutyl-3'-[1-ethyl-5-(isoquinolin-4-ylamino)-6-oxo-1,6-dihydropyridazin-3-yl]biphenyl-3-carboxamide;
3'-[1-ethyl-5-(isoquinolin-4-ylamino)-6-oxo-1,6-dihydropyridazin-3-yl]-N-(2-piperidin-1-ylethyl)biphenyl-4-carboxamide;
N-cyclobutyl-3'-[1-ethyl-6-oxo-5-(quinolin-5-ylamino)-1,6-dihydropyridazin-3-yl]biphenyl-3-carboxamide;
N-cyclopentyl-3'-[1-ethyl-6-oxo-5-(quinolin-5-ylamino)-1,6-dihydropyridazin-3-yl]biphenyl-3-carboxamide;
3'-[1-ethyl-6-oxo-5-(quinolin-5-ylamino)-1,6-dihydropyridazin-3-yl]-N-{7-[(2-hydroxyethyl)(methyl)amino]heptyl}biphenyl-3-carboxamide;
3'-[1-ethyl-5-(isoquinolin-4-ylamino)-6-oxo-1,6-dihydropyridazin-3-yl]-N-(1-methylpiperidin-4-yl)biphenyl-3-carboxamide;
N-cyclopropyl-3-{3'-[1-ethyl-6-oxo-5-(quinolin-5-ylamino)-1,6-dihydropyridazin-3-yl]biphenyl-3-yl}propanamide;
2-{3'-[1-ethyl-6-oxo-5-(quinolin-5-ylamino)-1,6-dihydropyridazin-3-yl]biphenyl-4-yl}-N-(2-piperidin-1-ylethyl)acetamide;
N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide;
and pharmaceutically acceptable salts, solvates, N-oxides or deuterated derivatives thereof.

The invention is also directed to a compound of the invention as described herein for use in the treatment of the human or animal body by therapy.

According to another embodiment the present invention covers pharmaceutical compositions comprising at least a compound of formula (I), as hereinabove described, in admixture with pharmaceutically acceptable diluents or carriers.

In still another embodiment the present invention covers a combination product comprising
(i) at least a compound of formula (I), as hereinabove described, and
(ii) one or more active ingredients selected from the group consisting of (a) β2-adrenergic agonists, (b) anti-cholinergics such as antagonists of M3 muscarinic receptors (c) antiinflammatory agents, (d) anti-allergic agents, (e) immunosuppressants and (f) anti-infectives;
for simultaneous, separate or sequential use in the treatment of the human or animal body.

The invention is also directed to the compounds of the invention as described herein, for use in the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention also provides the use of the compounds of the invention as described herein, for the manufacture of a medicament for the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention is also directed to a method of treatment of a pathological condition or disease susceptible to amelioration by inhibiton of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease; comprising administering a therapeutically effective amount of the compounds of the invention or a pharmaceutical composition of the invention to a subject in need of such treatment.

The compounds of the present invention may be prepared by methods such as those illustrated in the following Schemes. Solvents, temperatures, pressures and other reaction conditions may readily be selected by one of ordinary skill in the art. Starting materials are commercially available or can be readily prepared by one of ordinary skill in the art using known methods.

Compounds of general formula (I) may be prepared following the synthetic scheme depicted in figure 1.

### Figure 1

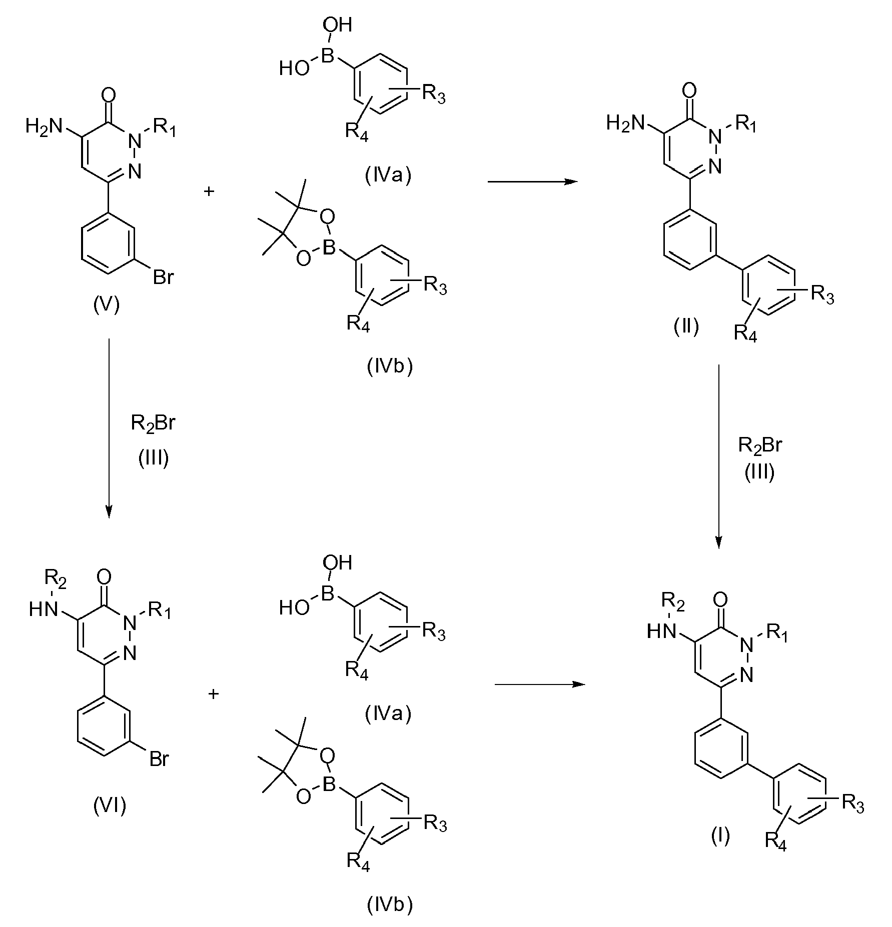

Compounds of general formula (I) may be prepared by condensation of an amine of formula (II), wherein R₁, R₃ and R₄ are as defined above, with an heteroaryl bromide of formula (III) wherein R₂ is as defined above. The reaction is carried out in the presence of a copper salt such as cuprous iodide and an inorganic base such as potassium phosphate, potassium carbonate or sodium carbonate and can also be performed in the presence of an organic base, preferably a diamine base such as N, N'-dimethylethylenediamine in an inert solvent such as toluene, dioxane or dimethylformamide, at a temperature from -20°C to the boiling point of the solvent. It can also be performed neat.

Compounds of formula (II) may be prepared by a coupling reaction between intermediate of formula (V) and a boronic acid (IVa) or a borolane derivative (IVb). The reaction is carried out using typical Suzuki-Miyaura reaction conditions (Miyaura, N.; Suzuki, A. Chem. Rev. 1995, 95, 2457) such as in the presence of tetrakis(triphenylphosphine)palladium(0) or [1,1'-bis(diphenylphosphino)ferrocene] palladium(II) dichloride dichloromethane complex (1:1) in solvents such as toluene or dioxane in an aqueous solution of a base such as sodium or cesium carbonate.

Alternatively, compounds of formula (I) may be prepared by condensation of a bromide of formula (VI), wherein R₁ and R₂ are as defined above, a boronic acid (IVa) or a borolane derivative (IVb). The reaction is carried out using typical Suzuki-Miyaura reaction conditions (Miyaura, N.; Suzuki, A. Chem. Rev. 1995, 95, 2457) such as in the presence of tetrakis(triphenylphosphine)palladium(0) or [1,1'-bis(diphenylphosphino)ferrocene] palladium(II) dichloride dichloromethane complex (1:1) in solvents such as toluene or dioxane in an aqueous solution of a base such as sodium or cesium carbonate.

Compounds of formula (VI) may be prepared by condensation of an amine of formula (V), wherein R₁ is as defined above, with an heteroaryl bromide of formula (III) wherein R₂ is as defined above. The reaction is carried out in the presence of a copper salt such as cuprous iodide and an inorganic base such as potassium phosphate, potassium carbonate or sodium carbonate and can also be performed in the presence of an organic base, preferably a diamine base such as N, N'-dimethylethylenediamine in an inert solvent such as toluene, dioxane or dimethylformamide, at a temperature from -20°C to the boiling point of the solvent. It can also be performed neat.

In some particular cases wherein R₃ represents a -(CH₂)ₙC(O)NH2 group, a -(CH₂)ₙC(O)NH-(CH₂)ₘ-R₅ group, a -(CH₂)ₙC(O)NH-(CH₂)ₘ-C(O)R₆ group or a -(CH₂)ₙC(O)NH-(CH₂)ₘ-NR₅R₇ group; compounds of formula (Ia), (Ib) and (Ic) may be prepared by reaction of the acid derivatives of formula (VII), wherein R₃ represents a -(CH₂)ₙ-CO₂H group, with an amine derivative of formula (VIIIa), (VIIIb) or (VIIIc) respectively following the synthetic route depicted in Figure 2.

### Figure 2

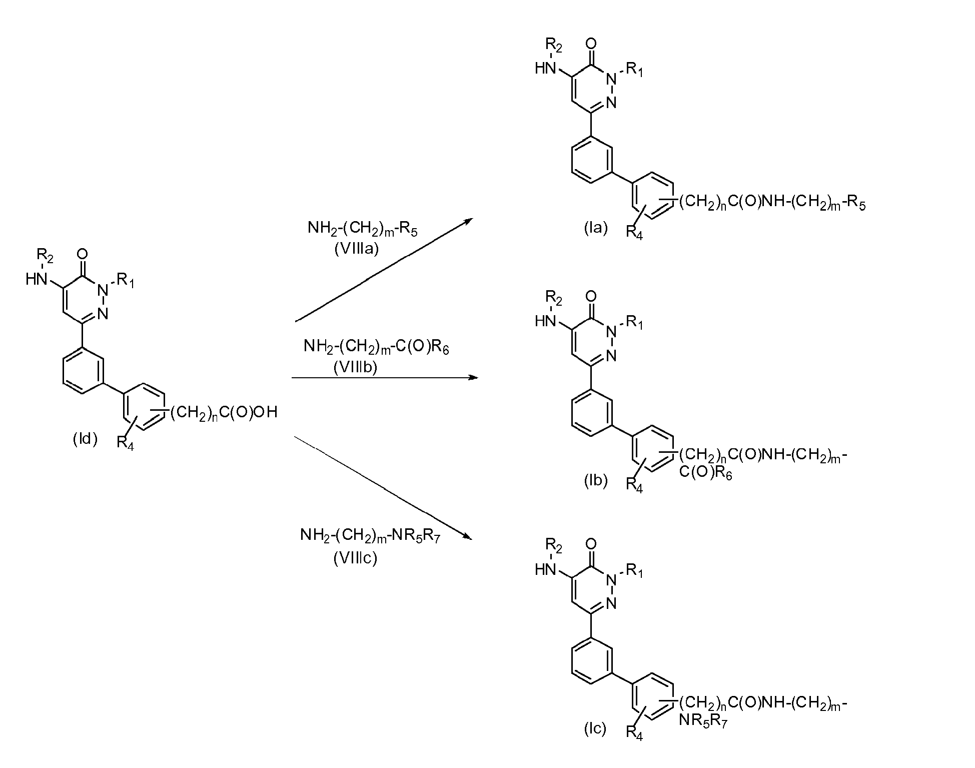

The reaction may be conducted using known amide bond formation procedures, for example by activation of the carboxylic acid of formula (Id) with a coupling agent such as N-ethyl-N'-(3-dimethyl-aminopropyl)carbodiimide hydrochloride in the presence of 1-hydroxybenzotriazole or O-(benzotriazol-1-yl)-N,N,N',N'-tetramethyluronium hexafluorphosphate in the presence of a base, such as diisopropylethylamine in a solvent such as N,N-dimethylformamide followed by addition of intermediates of formula (VIIIa), (VIIIb) or (VIIIc), wherein R₅, R₆ and R₇ are as defined above, to yields compounds of formula (Ia), (Ib) or (Ic), respectively.

Intermediates of formula (V) may be prepared by a variety of methods. For example, as shown in figure 3, reaction of 1,3-dicarbonylic compounds of general formula (IX) with ethyl 2-chloro-2-(hydroxyimino)acetate following methods known per se, e. g. G. Renzi et al., Gazz. Chim. Ital. 1965, 95, 1478, yields to isoxazole derivatives of formula (X).

### Figure 3

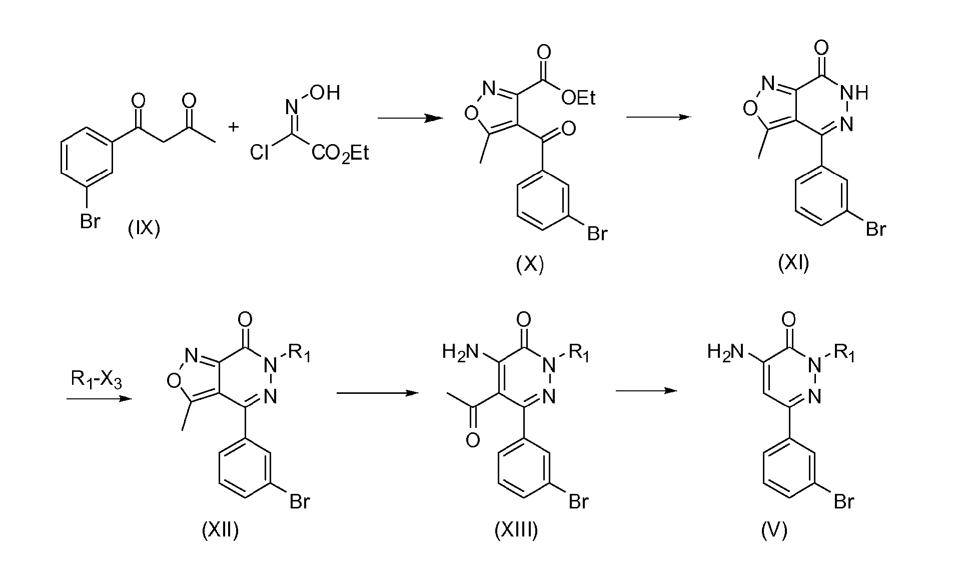

Isoxazole derivatives of formula (X) are condensed with hydrazine, by methods known per se, e. g. G. Renzi et al., Gazz. Chim. Ital. 1965, 95, 1478 and V. Dal Piaz et al. Heterocycles 1991, 32, 1173, to give isoxazolo[3,4-d]pyridazin-7(6H)-ones of formula (XI). Intermediates of formula (XI) are reacted with an alkylating agent of formula R₁-X₃, wherein R₁ is as hereinbefore defined and X₃ is a leaving group such as a chlorine or a bromine atom or a methanesulfonate, p-toluenesulfonate or a benzenesulfonate group, by methods known per se, e. g. V. Dal Piaz et al. Drug Des. Discovery 1996, 14, to yield isoxazolo[3,4-d]pyridazin-7(6H)-ones derivatives of formula (XII). Intermediates of formula (XII) are hydrogenated to yield 5-acetyl-4-amino(3-oxo)pyridazin-4-ylurea derivatives of formula (XIII). The hydrogenation may be performed using for example hydrogen in the presence of a catalyst by methods known per se, e. g. V. Dal Piaz et al. Heterocycles, 1991, 32, 1173. Alternatively, the reaction may be accomplished by transfer hydrogenation using an organic hydrogen donor and a transfer agent, such as ammonium formate or hydrazine by methods known per se, e. g. V. Dal Piaz et al. Heterocycles, 1991, 32, 1173. Finally, treatment of 4-amino(3-oxo)pyridazin-4-ylurea derivatives (XIII) with hydrobromic acid at reflux, gives Intermediate compounds of formula (V).

When the defined R groups are susceptible to chemical reaction under the conditions of the hereinbefore described processes or are incompatible with said processes, conventional protecting groups may be used in accordance with standard practice, for example see T. W. Greene and P. G. M. Wuts in 'Protective Groups in Organic Chemistry', 3rd Edition, John Wiley & Sons (1999). It may be that deprotection will form the last step in the synthesis of compounds of formula (I).

The syntheses of the compounds of the invention and of the intermediates for use therein are illustrated by the following Examples (1-63) (including Preparation Examples (Preparations 1 to 54) which do not limit the scope of the invention in any way.

¹H Nuclear Magnetic Resonance Spectra were recorded on Varian Gemini 200, 300 and 400 spectrometers.

LCMS analysis was carried out using the following method:
Waters Alliance 2795HT coupled to a Waters 2996 DAD detector and Waters ZQ mass detector.
Column: Symmetry C18 (100x2.1 mm) 3.5mm

| | |
|---|---|
| Mobile phase: | A: 0.05% Ammonium formate Water |
| | B: 0.04% Ammonium formate Acetonitrile:Methanol (1:1) |

| Gradient: t (min) | %B |
|---|---|
| 0 | 0 |
| 20 | 95 |
| 24 | 95 |
| 26 | 0 |
| 30 | 0 |

Flow: 0.4 ml/min
Temperature: Ambient

| | |
|---|---|
| Detection: | UV DAD (210) |
| | MS (ESI+ and ESI-) |

Sample concentration: 1 mg/ml
Injection volume: 5 mL

### Preparation Examples

### PREPARATION 1

### 1-(3-Bromophenyl)butane-1,3-dione

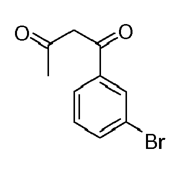

50 g sodium hydride (60% dispersion in oil, previously washed with pentane, 1.25 mol) was suspended in 920 mL dry tetrahydrofuran. 122.5 mL ethyl acetate (1.25 mol) was added followed by 15 drops of absolute ethanol. The mixture was cooled in an ice-water bath. A solution of 125 g 1-(3-bromophenyl)ethanone (0.63 mol) dissolved in 330 mL tetrahydrofuran was added with stirring, maintaining the reaction temperature below 20 °C. Upon completion of addition 3.5 g 18-crown-6 (0.01 mol) was added. The mixture was stirred at room temperature for 15 min, at 40 °C for 15 min, then at reflux for 5.5 h and then overnight, cooling to room temperature.

The mixture was evaporated and the residue taken up in 1.5 L water. The aqueous mixture was extracted five times with 250 mL ether and was then cooled in an ice-bath and acidified to pH 1-2 with concentrated hydrochloric acid. The aqueous was extracted three times with 250 mL ether, the combined organics were washed twice with water, once with brine. The organics were dried over sodium sulphate, filtered and evaporated to give 124.9 g (82%) of the crude title compound as an oil, contaminated with several small impurities.
¹H NMR (300 MHz, CHLOROFORM-d) δ ppm 8.01 (1 H, s), 7.80 (1 H, d, *J*=7.7 Hz), 7.64 (1 H, d, *J*=7.7 Hz), 7.33 (1 H, t, *J*=8.0 Hz), 6.15 (1 H, s), 2.22 (3 H, s)

### PREPARATION 2

### Ethyl 4-(3-bromobenzoyl)-5-methylisoxazole-3-carboxylate

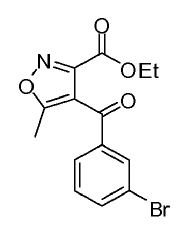

13 g Sodium metal (previously washed with pentane, 0.57 mol) was mechanically stirred in 50 mL absolute ethanol until completely dissolved. The solution was cooled to between 0 and -5 °C with a ice-salt bath and a solution of 124 g of the crude title compound from Preparation 1 (0.51 mol) dissolved in 325 ethanol was added drop-wise with stirring, maintaining the reaction temperature below 0 °C. The mixture was stirred for 15 min and then a solution of 85 g ethyl 2-chloro-2-(hydroxyimino)acetate (0.56 mol) dissolved in 180 mL ethanol was added drop-wise, maintaining the reaction temperature below 0 °C. The mixture was stirred for 30 min at 0 °C and then overnight, warming to room temperature. The solution (at pH 7) was acidified to pH 5 with 25 mL acetic acid and was evaporated to dryness. The residue was taken up in 750 mL ice-water and was extracted with 3 x 300 mL ether. The combined organics were washed successively with 4% sodium bicarbonate solution, water and brine. The organics were dried over sodium sulphate, decolourised at reflux with activated carbon and filtered. Evaporation under high vacuum gave 155.4 g (89%) of the crude title compound as an oil which solidified upon standing.
¹H NMR (300 MHz, CHLOROFORM-d) δ ppm 7.90 (1 H, s), 7.74 (1 H, d, *J*=7.7 Hz), 7.66 (1 H, d, *J*=7.4 Hz), 7.36 (1 H, t, *J*=7.8 Hz), 4.18 (2 H, q, *J*=7.0 Hz), 2.57 (3 H, s), 1.14 (3 H, t)

### PREPARATION 3

### 4-(3-Bromophenyl)-3-methylisoxazolo[3,4-d]pyridazin-7(6H)-one

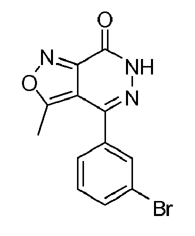

155.4 g of the crude title compound from Preparation 2 (0.46 mol) was suspended in 1.15 L ethanol. 34.1 mL hydrazine hydrate (0.7 mol) was slowly added with stirring, dissolving the remaining solid. The mixture was stirred at room temperature overnight, precipitating a solid.

The mixture was cooled in an ice-water bath for 15 min and then filtered. The solid was washed with ethanol, then with ether and was dried in a stream of air and then at 50 °C under vacuum to give 101.5 g (72%) of the title compound.
¹H NMR (300 MHz, DMSO-*d*₆) δ ppm 7.83 (1 H, s), 7.77 (1 H, d, *J*=7.9 Hz), 7.66 (1 H, d, *J*=7.6 Hz), 7.53 (1 H, t, *J*=7.9 Hz), 3.35 (3 H, s)

### PREPARATION 4

### 4-(3-Bromophenyl)-6-ethyl-3-methylisoxazolo[3,4-d]pyridazin-7(6H)-one

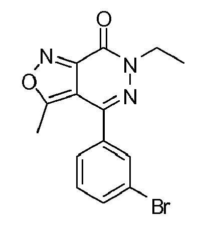

18.9 g of the title compound from Preparation 3 (61.8 mol) was dissolved in 200 mL anhydrous DMF. 25.7 g potassium carbonate (186 mmol) was added and the mixture stirred for 20 min. 13.9 mL bromoethane (186 mmol) was added drop-wise with stirring, maintaining the reaction temperature below 30 °C, and the mixture was stirred at room temperature overnight.

The mixture was evaporated to dryness, the residue taken up in 200 g ice-water and extracted several times with ethyl acetate. The combined organics were washed successively with water, 4% sodium bicarbonate solution, water, brine-water and brine. The organics were dried over sodium sulphate, decolourised at reflux with activated carbon and filtered. Evaporation gave a solid which was broken up in ether and collected by filtration. The solid was washed with ether and was dried in a stream of air and then at 50 °C under vacuum to give 17.6 g (85%) of the title compound.
¹H NMR (300 MHz, CHLOROFORM-*d*) δ ppm 7.72 (1 H, s), 7.67 (1 H, d, *J*=8.0 Hz), 7.50 (1 H, d, *J*=7.7 Hz), 7.43 (1 H, d, *J*=8.0 Hz), 4.28 (2 H, q, *J*=7.1 Hz), 2.58 (3 H, s), 1.42 (3 H, t, *J*=7.1 Hz)
HPLC/MS retention time 8.83 min.
LRMS: *m*/*z* 334/336 (M+H⁺), 351/353 (M+NH₄⁺)

### PREPARATION 5

### 5-Acetyl-4-amino-6-(3-bromophenyl)-2-ethylpyridazin-3(2H)-one

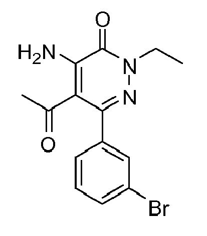

34.4 g of the title compound from Preparation 4 (103 mmol) was dissolved in 500 mL tetrahydrofuran and 100 mL ethanol. A heaped spoonful of Raney nickel was added and the mixture agitated under 14 psi hydrogen overnight.

The mixture was filtered and the catalyst washed with tetrahydrofuran. The combined filtrate was evaporated and the solid residue was broken up in 100 mL hot ethanol. Upon cooling to room temperature, the solid was collected by filtration, was washed with ether and was dried in a stream of air and then at 50 °C under vacuum to give 31.2 g (90%) of the title compound.
¹H NMR (300 MHz, CHLOROFORM-*d*) δ ppm 7.66 (1 H, s), 7.60 (1 H, d, *J*=7.4 Hz), 7.29 - 7.40 (2 H, m), 4.25 (2 H, q, *J*=7.2 Hz), 1.83 (3 H, s), 1.42 (3 H, t, *J*=7.1 Hz)
HPLC/MS retention time 7.77 min.
LRMS: *m*/*z* 336/338 (M+H⁺)

### PREPARATION 6

### 4-Amino-6-(3-bromophenyl)-2-ethylpyridazin-3(2H)-one

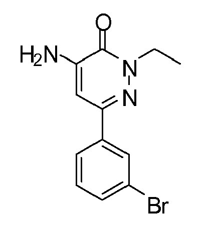

29.4 g of the title compound from Preparation 5 (87.5 mmol) was suspended in 215 mL 48% hydrobromic acid (3.96 mol) and was heated at 130 °C for 1.25 h. the mixture was allowed to cool and was carefully diluted with ice-water and basified with solid sodium carbonate. Once basic, the aqueous was extracted with 2 x 300 mL ethyl acetate. The combined organics were washed successively with water and brine. The organics were dried over sodium sulphate, decolourised at reflux with activated carbon and filtered. Evaporation gave a residue which was broken up in 100 mL hot diisopropyl ether. Upon cooling, the solid was collected by filtration, washed with diisopropyl ether and was dried in a stream of air and then at 50 °C under vacuum to give 21.1 g (82%) of the title compound.
¹H NMR (CDCl₃) δ ppm 7.93 (1 H, s), 7.67 (1 H, d, *J*=8.0 Hz), 7.52 (1 H, d, *J*=8.0 Hz), 7.30 (1 H, t, *J*=7.7 Hz), 6.67 (1 H, s), 5.02 (2 H, br. s.), 4.30 (2 H, q, *J*=7.1 Hz), 1.45 (3 H, t, *J*=7.3 Hz)
HPLC/MS retention time 8.58 min.
LRMS: *m*/*z* 294/296 (M+H⁺)

### PREPARATION 7

### 5-Bromoquinoline

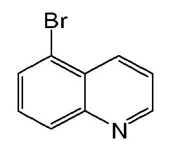

Quinolin-5-amine (3.37 g, 23.38 mmol) was dissolved in 9 ml of water and 11 ml of hydrogen bromide (48% in water); the resulting solution was cooled at 0°C. Sodium nitrite (1.94 g, 28.12 mmol) dissolved in 9 ml of water was dropwise added. The resultant solution was stirred at room temperature for 5 minutes. This solution was dropwise added to a solution of copper(I) bromide (4.02 g, 28.02 mmol) in 23 mL of HBr (48% in water) at 75°C. The resulting mixture was stirred at room temperature for 2 h. Then the reaction mixture was basified with sodium hydroxide and extracted twice with ethyl acetate. The organic phase is washed with brine, filtered and dried over sodium sulfate. After filtration and evaporation of the solvent, 2.98 g (61 %) of the final product were obtained.
¹H NMR (CDCl3) δ ppm: 7.44 - 7.63 (m, 2 H) 7.84 (d, *J*=7.42 Hz, 1 H) 8.10 (d, *J*=8.24 Hz, 1 H) 8.56 (d, *J*=8.51 Hz, 1 H) 8.94 (br.s., 1H) HPLC/MS (9 min) retention time 5.68 min.
LRMS: *m*/*z* 208 (M)/21 0 (M+2)

### PREPARATION 8

### 6-(3-Bromophenyl)-2-ethyl-4-(quinolin-5-ylamino)pyridazin-3(2H)-one

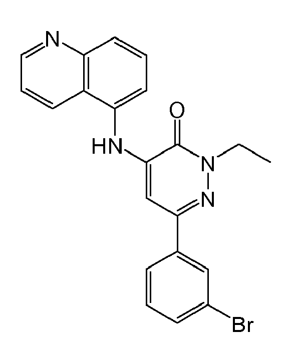

A Schlenck reactor with magnetic stirring and argon atmosphere was charged with 4-amino-6-(3-bromophenyl)-2-ethylpyridazin-3(2H)-one (200 mg, 0.68 mmol), 5-bromoquinoline (141.5 mg, 0.68 mmol), cessium carbonate (310 mg, 0.95 mmol), Xantphos (79 mg, 0.14 mmol) and dioxane (5 ml). After three cycles of vacuum-argon filling, Pd(dba)3 (62 mh, 0.07 mmol) was added and the reaction mixture was heated overnight at 120°C. Ethyl acetate was added, the mixture was filtered and the liquid phase was concentrated to dryness. The obtained residue was dissolved in dichloromethane and washed twice with water. 350 mg (39%) of the final compound were obtained.
HPLC/MS (9 min) retention time 7.19 min.
LRMS: *m*/*z* 423 (M+2H⁺)

### PREPARATION 9

### Ethyl 3'-(1-ethyl-6-oxo-5-(quinolin-5-ylamino)-1 ,6-dihydropyridazin-3-yl)biphenyl-3-carboxylate

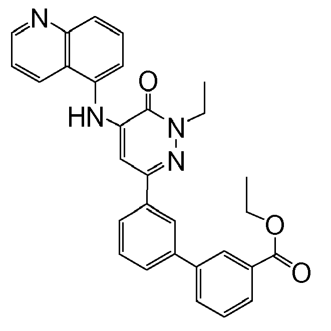

In a Schlenk tube, a mixture of 6-(3-bromophenyl)- 2-ethyl-4- (quinolin-5-ylamino) pyridazin-3(2H)-one (3.48g, 8.26 mmol), 3-(ethoxycarbonyl)phenylboronic acid (2.7 g, 13.92 mmol) and cessium carbonate (13.60 g, 3.29 mmol) was dissolved in dioxane (80ml). The mixture was purged (vacuum-argon three times) and [1,1-bis(diphenylphosphino)ferrocene]dichloropalladium(II)dichloromethanecomplex(0.41g, 0.51 mmol) was added. The mixture was purged again (vacuum-argon three times) and stirred at 90 °C for 18 h. The suspension was filtered off and the filtrated diluted with water and extracted three times with ethyl acetate. The combined organic layers were washed with water and brine, and dried over anhydrous sodium sulphate. Solvent was removed in vacuum and the residue was purified by the SP1® automated purification system to give 1.36g (34%) of the desired compound.
HPLC/MS (9 min) retention time 7.52 min.
LRMS: *m*/*z* 491 (M+H⁺)

### PREPARATION 10

### 6-(3-Bromophenyl)-2-ethyl-4-(quinolin-4-ylamino)pyridazin-3(2H)-one

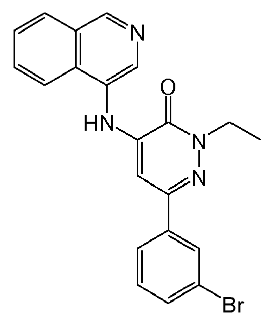

A Schlenck reactor with magnetic stirring and argon atmosphere was charged with 4-amino- 6- (3 -bromophenyl)-2 -ethylpyridazin -3(2H) -one (3 g, 10.2 mmol), 4-bromoisoquinoline (2.12 g, 10.19 mmol), BINAP (0.32g, 0.51 mmol), potasium tert-butoxide (2.29 g, 20.41 mmol), and toluene (100 mL). After three cycles of vacuum-argon filling, Pd(dba)₃ (0.47 g, 0.51 mmol) was added and the reaction mixture was heated overnight at 110°C. Once the reaction was over, the mixture was filtered thorugh diatomaceous earth (Celite®) and the liquid phase was washed twice with water, brine and dried over anhydrous magnesium sulphate. Solvent was removed in vacuum and the residue was purified by the SP1® automated purification system to give 1.39 g (29%) of the desired compound.
HPLC/MS (9 min) retention time 7.18 min.
LRMS: *m*/*z* 422 (M+H⁺)

### PREPARATION 11

### Ethyl 3'-(1-ethyl-5-(isoquinolin-4-ylamino)-6-oxo-1,6-dihydropyridazin-3-yl)biphenyl-3-carboxylate

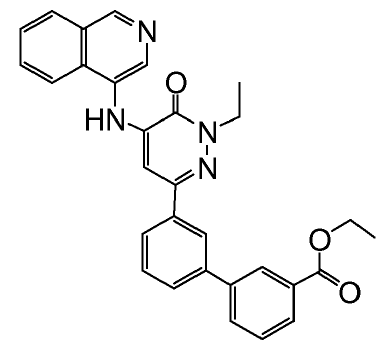

In a Schlenk tube, a mixture of 6-(3-bromophenyl)-2-ethyl-4-(quinolin-4-ylamino)pyridazin-3(2H)-one (640 mg, 1.23 mmol, see Preparation 10), 3-(ethoxycarbonyl)phenylboronic acid (320 mg, 1.65 mmol) and cessium carbonate (1.2 g, 3.74 mmol) was dissolved in dioxane (6 mL). The mixture was purged (vacuum-argon three times) and [1,1-bis (diphenylphosphino) ferrocene] dichloropalladium (II) dichloromethane complex (65 mg, 0.08 mmol) was added. The mixture was purged again (vacuum-argon three times) and stirred at 90 °C for 18 h. The suspension was filtered off and the filtrated diluted with water and extracted three times with ethyl acetate. The combined organic layers were washed with water and brine, and dried over anhydrous sodium sulphate. Solvent was removed in vacuum and the residue was soaked with ethyl ether and subsequently filtered to give 0.29 g (38%) of the desired compound.
HPLC/MS (9 min) retention time 7.55 min.
LRMS: *m*/*z* 604 (M+H⁺)

### PREPARATION 12

### Ethyl 3'-(1-ethyl-5-(isoquinolin-4-ylamino)-6-oxo-1,6-dihydropyridazin-3-yl)biphenyl-4-carboxylate

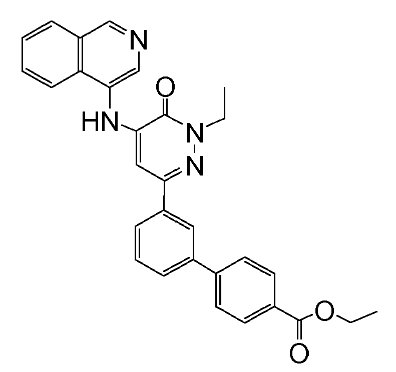

Reaction of 6- (3-bromophenyl) -2- ethyl-4- (isoquinolin -4-ylamino) pyridazin -3(2H)-one (0.66 g, 1.57 mmol) with 4-(ethoxycarbonyl)phenylboronic acid (0.46 g, 2.37 mmol) according to the method described in Preparation 11 gave 1.04 g (100%) of the title compound.
HPLC/MS (9 min) retention time 7.54 min.
LRMS: *m*/*z* 491 (M+1)

### PREPARATION 13

### Ethyl [4-(4,4,5,5-tetramethyl-1,3,2-dioxaborolan-2-yl)phenyl]acetate

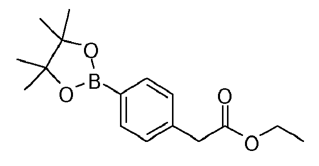

In a Schlenk tube, a mixture of (4-bromophenyl)acetate (1.0 g, 4.11mmol), bis(pinacolato)diboron (2.37 g, 9.34 mmol) and potassium acetate (1.38 g, 14 mmol) was dissolved in dioxane (40ml). The mixture was purged (vacuum-argon three times) and [1,1-bis (diphenylphosphino) ferrocene] dichloropalladium (II) dichloromethane complex (0.19 g, 0.23 mmol) and 1,1'-bis(diphenylphospheno)ferrocene (0.13 g, 0.23 mmol) were added. The mixture was purged again (vacuum-argon three times) and stirred at 90 °C for 18 h.

The suspension was filtered off and the filtrated diluted with water and extracted three times with ethyl acetate. The combined organic layers were washed with water and brine, and dried over anhydrous sodium sulphate. Solvent was removed in vacuum and the residue was purified by the SP1® automated purification system to give 0.86 g mmol (72%) of the desired compound.
¹H NMR (200 MHz, CHLOROFORM-*d*) δ ppm 1.23 (t, *J*=6.83 Hz, 3 H), 1.34 (s, 12 H), 3.62 (s, 2 H), 4.13 (q, *J*=7.03 Hz, 2 H), 7.22 - 7.34 (m, 2 H), 7.77 (d,
*J*=7.42 Hz, 2 H),
HPLC/MS (9 min) retention time 6.86 min.
LRMS: *m*/*z* 308 (M+1)

### PREPARATION 14

### Ethyl 2-(3'-(1-ethyl-6-oxo-5-(quinolin-5-ylamino)-1,6-dihydropyridazin-3-yl)biphenyl-4-yl)acetate

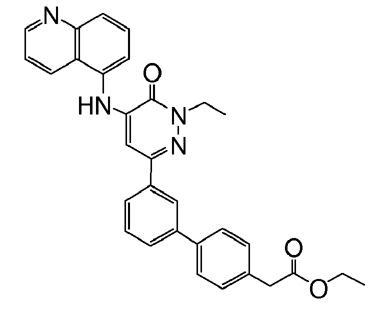

Reaction of 6-(3-bromophenyl)-2-ethyl-4-(quinolin-5-ylamino)pyridazin-3(2H)-one (0.6g, 1.42 mmol, see Preparation 8) with ethyl [4-(4,4,5,5-tetramethyl-1,3,2-dioxaborolan-2-yl)phenyl]acetate (0.45 g, 1.55 mmol, see Preparation 13) according to the method described in Preparation 11 gave 0.23 g (32%) of the title compound.
HPLC/MS (9 min) retention time 7.73 min.
LRMS: *m*/*z* 505 (M+1)

### PREPARATION 15

### Methyl [3-(4,4,5,5-tetramethyl-1,3,2-dioxaborolan-2-yl)phenyl]acetate

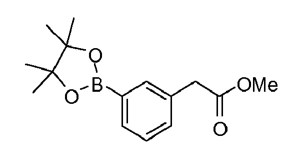

Reaction of 2.05 g methyl (3-bromophenyl)acetate with 4.55 g bis(pinacolato)diboron according to the method described in Preparation 13 gave 2.00 g (75%) of the title compound.
HPLC/MS (9 min) retention time 6.59 min.
LRMS: *m*/*z* 277 (M+1)

### PREPARATION 16

### 2-(3-(4,4,5,5-Tetramethyl-1,3,2-dioxaborolan-2-yl)phenyl)acetic acid

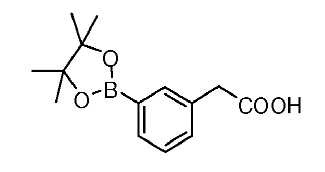

To a suspension of 3-bromophenylacetic acid (1.00 g, 4.65 mmol), bispinacolato diboron (2.40 g, 9.30 mmol) and AcOK (1.40 g, 13.95 mmol) in dioxane (30 mL) were added PdCl₂dppf·DCM (0.19 g, 0.23 mmol) and dppf (0.13 g, 0.23 mmol). The mixture was stirred at reflux for 16 h. It was filtered through Celite, washed with AcOEt, and concentrated. The residue was redissolved in AcOEt (30 mL) and water (30 mL) and the aqueous phase was extracted with AcOEt (3x20 mL). The combined organic phases were dried over Na₂SO₄ (anhydrous), filtered and concentrated. The crude residue was flash chromatographed on SiO₂ (60%-100% AcOEt/hexanes) to afford the titled compound (0.94 g, white solid, yield: 78%).
¹H NMR (CDCl₃, 250 MHz) δ ppm: 7.73 (m, 2H), 7.36 (m, 2H), 3.66 (s, 2H), 1.34 (s, 12H)

### PREPARATION 17

### Ethyl 3'-(1-ethyl-6-oxo-5-(quinolin-5-ylamino)-1,6-dihydropyridazin-3-yl)biphenyl-4-carboxylate

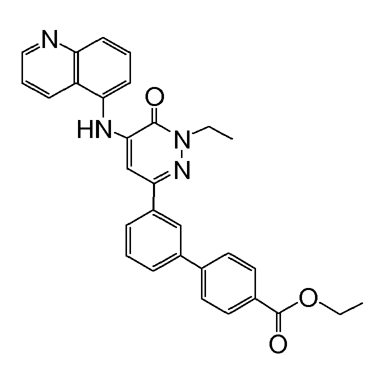

Reaction of 6-(3-bromophenyl)-2-ethyl-4-(quinolin-5-ylamino)pyridazin-3(2H)-one (1 g, 2.37 mmol, see Preparation 8) with 4-(ethoxycarbonyl)phenylboronic acid (0.69 g, 3.56 mmol) according to the method described in Preparation 9 gave 0.66 g (56 %) of the title compound.
¹H NMR (200 MHz, DMSO-d6) δ ppm 1.27 - 1.51 (m, 6 H) 4.20 - 4.43 (m, 4 H) 6.61 (s, 1 H) 7.42 - 7.67 (m, 3 H) 7.67 - 7.88 (m, 5 H) 7.89 - 8.10 (m, 4 H) 8.34 (d, J=8.20 Hz, 1 H) 8.96 (d, J=2.34 Hz, 1 H) 9.16 (s, 1 H)
HPLC/MS (9 min) retention time 7.56 min.
LRMS: *m*/*z* 491 (M+1)

### PREPARATION 19

### N-[2-(Dimethylamino)ethyl]-3-(4,4,5,5-tetramethyl-1,3,2-dioxaborolan-2-yl)benzamide

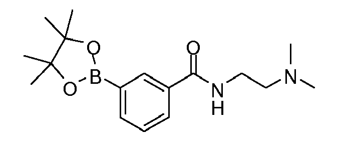

N,N-dimethylethane-1,2-diamine (0.71 g, 8.06 mmol) was added to a solution of 3-(4,4,5,5-tetramethyl-1,3,2-dioxaborolan-2-yl)benzoic acid (1.00 g, 4.03 mmol), 1-hydroxybenzotriazole hydrate (0.82 g, 6.05 mmol) and N-ethyl-N'-(3-dimethylaminopropyl)carbodiimide hydrochloride (1.16 g, 6.05 mmol) in 8 mL dimethylformamide. The mixture was stirred at room temperature for 3 h.

The solvent was removed under reduced pressure and the residue dissolved in 4% sodium bicarbonate solution. The aqueous phase was extracted three times with ethyl acetate. The combined organic layers were washed with water and brine, and dried over anhydrous sodium sulphate. Solvent was removed in vacuum to give 0.81 g (2.55 mmol, 63%) of the title compound.

### PREPARATION 20

### 3-bromo-5-nitrobenzoic acid

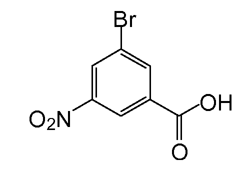

A mixture of 3-nitrobenzoic acid (10 g, 0.06 mols), concentrated sulphuric acid (120 mL), silver sulphate (9.33 g, 0.03 mols) and bromine (3.68 mL, 0.07 mols) was stirred at 100°C for 6h. The mixture was poured on ice, and the solid filtered off and extracted with sodium carbonate solution. The title compound was precipitated on acidification from dilute alcohol giving 7.11 g (47%).
HPLC/MS (10 min) retention time 5.69 min
LRMS: *m*/*z* 244 (M-1)

### PREPARATION 21

### 3-Amino-5-bromobenzoic acid

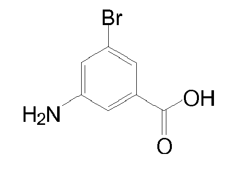

Concentrated hydrochloric acid (30 mL) was gradually added to a solution of the title compound of Preparation 20 (7.02 g, 28.54 mmols) and ethanol (150 mL), the solution was stirred at room temperature during five minutes. Then Tin (II) chloride was added and the solution was stirred at 50°C for two hours.

The solution was cooled at room temperature. The solution basified to pH 9 with 8M sodium hydroxide precipitating a solid. The solid was collected by filtration and discarded. The filtrate was acidified with hydrochloric acid to pH 5 and precipitation from this solution gave 5.8 g (94%) of the title compound.
HPLC/MS (10 min) retention time 4.67 min
LRMS: *m*/*z* 216 (M-1)

### PREPARATION 22

### 3-Bromo-5-hydroxybenzoic acid

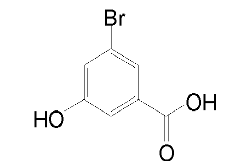

The title compound of Preparation 21 (5.80 g, 26.85 mmols), in 10% sulphuric acid (55 mL), was diazotised with sodium nitrite (1.85 g), and the solution boiled with dilute sulphuric acid (1:1) (30 mL) for one hour and forty-five minutes.

The solution was cooled at room temperature. The suspension was filtered and the solid give 3.83 g (66%) of the title compound.
HPLC/MS (10 min) retention time 5.06 min
LRMS: *m*/*z* 215 (M-1)

### PREPARATION 23

### Methyl 3-bromo-5-hydroxybenzoate

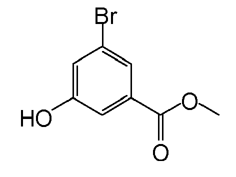

Reaction of 3.93 g (18.11 mmols) of the title compound of Preparation 22 following W02006/051375 gave 4.00 g (96%) of the title compound.
HPLC/MS (10 min) retention time 5.87 min
LRMS: *m*/*z* 230 (M-1)

### PREPARATION 24

### Methyl 3-hydroxy-5-(4,4,5,5-tetramethyl-1,3,2-dioxaborolan-2-yl)benzoate

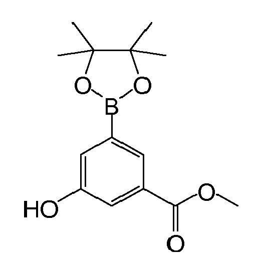

Reaction of 4.00 g (17.31 mmols) of the title compound of Preparation 23 with 8.79 g (34.63 mmol) bis(pinacolato)diboron according to the method described in Preparation 13 gave 5.19 g (98%) of the title compound.
¹H NMR (200 MHz, DMSO-*d*₆) δ ppm 0.94 - 1.44 (m, 12 H) 3.84 (s, 3 H) 7.30 (s, 1 H) 7.43 (s, 1 H) 7.71 (s, 1 H) 9.47 (s, 1 H)
HPLC/MS (10 min) retention time 5.53 min
LRMS: *m*/*z* 277 (M-1)

### PREPARATION 25

### Methyl 3'-(1-ethyl-6-oxo-5-(quinolin-5-ylamino)-1,6-dihydropyridazin-3-yl)-5-hydroxybiphenyl-3-carboxylate

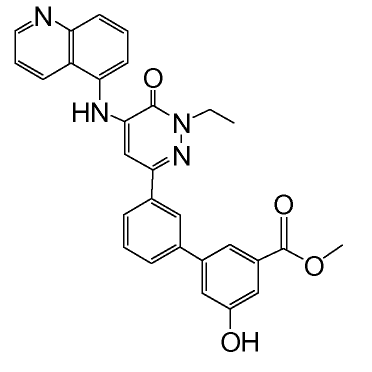

Reaction of 600 mg (1.42 mmols) of the title compound of Preparation 8 with 475 mg (1.71 mmol) methyl 3 -hydroxy- 5- (4,4,5,5-tetramethyl - 1,3,2-dioxaborolan - 2 - yl) benzoate according to the method described in Preparation 11 gave 170 mg (22%) of the title compound.
HPLC/MS (9 min) retention time 6.73 min.
LRMS:*m*/*z* 493 (M+1)

### PREPARATION 26

### 3'-(1-Ethyl-6-oxo-5-(quinolin-5-ylamino)-1,6-dihydropyridazin-3-yl)-5-hydroxybiphenyl-3-carboxylic acid

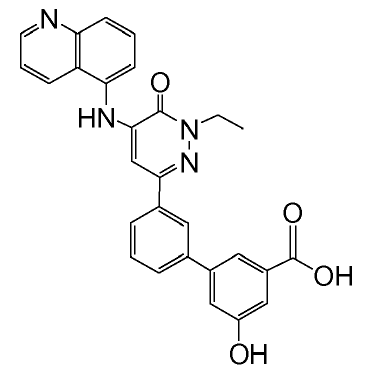

Methyl 3'-(1-ethyl-6-oxo-5-(quinolin-5-ylamino)-1,6-dihydropyridazin-3-yl)-5-hydroxybiphenyl-3-carboxylate (170 mg, 0.31 mmol, see Preparation 25) is suspended in 4 ml of water, 4 ml of THF and 3 ml of methanol. Lithium hydroxide monohydrate (70 mg, 1.67 mmol) is added and the mixture is stirred at room temperature for 18h. The solvent is evaporated under reduced pressure and the resulting solution is acidified with 5N chlorhydric acid. A solid precipitates, which is filtered, washed with water and dried under reduced pressure at 40°C. 110 mg (74%) of the final compound were obtained.
HPLC/MS (9 min) retention time 6.25 min.
LRMS: *m*/*z* 479 (M+1)

### PREPARATION 27

### 7-[(2-hydroxyethyl)(methyl)amino]heptanenitrile

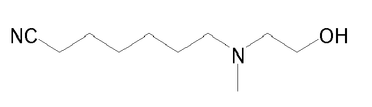

1.90 mL of 2-(methylamino)ethanol (23.67 mmol) was dissolved in 90 ml of methylisobutylketone and 3.27 g of potassium carbonate (23.67 mmol) were added. To this mixture was added a solution of 3.00 g of 7-bromoheptanenitrile (15.78 mmol) in 10 mL of methylisobutylketone. The reaction was kept overnight at 70 °C.

The solvent was evaporated under vacuum and the residue taken up in ethyl acetate. The organics were washed with water and brine, dried over magnesium sulphate, filtered and evaporated. 2.81 g (97%) of the title compound were obtained as a yellow oil.
HPLC/MS (10 min) retention time 0.67 min.
LRMS: *m*/*z* 185 (M+1)

### PREPARATION 28

### 2-[(7-aminoheptyl)(methyl)amino]ethanol

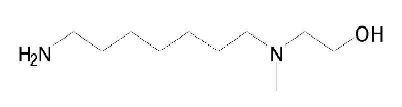

1.74 g of lithium aluminum hydride (45.75 mmol) were suspended in 40 mL of dry tetrahydrofuran under nitrogen atmosphere. Then a solution of 2.81 g of the title compound of Preparation 69 (15.25 mmol) in 10 mL of dry tetrahydrofuran was added, followed by additional 50 mL of solvent. The reaction was kept for 3 h at room temperature.

The reaction was quenched with successive additions of 1.75 mL of water, 1.75 mL of aqueous solution of sodium hydroxide 4M and 5.25 mL of water. This mixture was filtered though diatomaceous earth (Celite ®) and the filtrates were evaporated under vacuum. The residue was dissolved in methylene chloride and the organics were dried over magnesium sulphate, filtered and evaporated. 2.32 g (81%) of the title compound were obtained with no need of futher purification.
HPLC/MS (10 min) retention time 0.64 min.
LRMS: *m*/*z* 189 (M+1)

### PREPARATION 29

### 3-[4-(4,4,5,5-Tetramethyl-1,3,2-dioxaborolan-2-yl)phenyl]propanoic acid

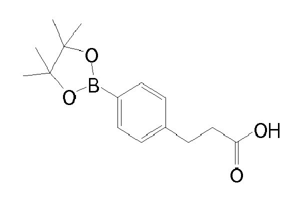

Reaction of 1.5 g (6.42 mmols) of 3-(4-bromophenyl)propanoic acid with 2.44 g (9.61 mmol) bis(pinacolato)diboron according to the method described in Preparation 13 gave 1.28 g (72%) of the title compound.
HPLC/MS (10 min) retention time 6.33 min
LRMS: *m*/*z* 294 (M+18)

### PREPARATION 30

### Ethyl 3-(3-bromophenyl)propanoate

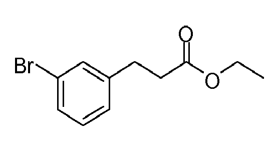

2 g (8.38 mmols) of 3-(3-bromophenyl)propanoic acid was dissolved in 25 mL ethanol. 2.10 mL sulphuric acid (39.4 mmols) was added and the mixture was heated at 90°C for 5 hours. The mixture was allowed to cool and was evaporated. The residue was taken up in water and basified to pH 9 with NaOH 32%. The aqueous was then extracted five times with dichloromethane. The organic phase was dried over magnesium sulphate. Purification by chromatography (hexane 100% to ethyl acetate 100%) gave 2.04g (92%) of the title compound.
HPLC/MS (10 min) retention time 6.81 min
LRMS: *m*/*z* 257 (M+)

### PREPARATION 31

### Ethyl 3-[3-(4,4,5,5-tetramethyl-1,3,2-dioxaborolan-2-yl)phenyl]propanoate

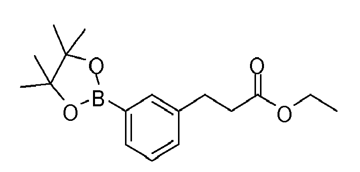

Reaction of 2.04 g (5.33 mmols) of the title compound of Preparation 30 with 2.98 g (11.5 mmol) bis(pinacolato)diboron according to the method described in Preparation 13 gave after purification by chromatography (hexane 100% to ethyl acetate 100%) 2.26g (97%) of the title compound.
HPLC/MS (10 min) retention time 6.5 min
LRMS: *m*/*z* 305 (M+1)

### PREPARATION 32

### Methyl 3'-(1-ethyl-6-oxo-5-(quinolin-5-ylamino)-1,6-dihydropyridazin-3-yl)-5-hydroxybiphenyl-3-carboxylate

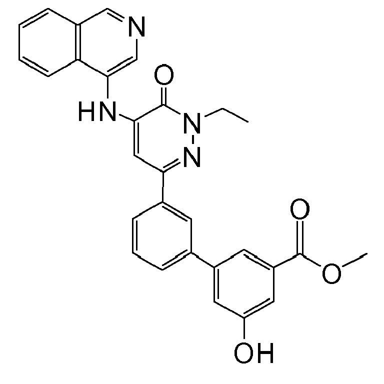

Reaction of 600 mg (1.42 mmols) of the title compound of Preparation 10 with 515 mg (1.85 mmol) methyl 3- hydroxy- 5- (4,4,5,5- tetramethyl- 1,3,2- dioxaborolan- 2-yl) benzoate according to the method described in Preparation 11 gave 129 mg (18%) of the title compound.
HPLC/MS (10 min) retention time 6.77 min
LRMS: *m*/*z* 493 (M+1)

### PREPARATION 33

### 3'-(1-Ethyl-5-(isoquinolin-4-ylamino)-6-oxo-1,6-dihydropyridazin-3-yl)-5-hydroxybiphenyl-3-carboxylic acid

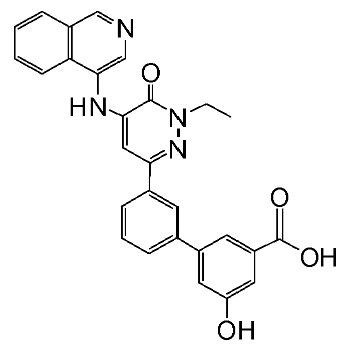

Methyl 3'-(1-ethyl-5-(isoquinolin-4-ylamino)-6-oxo-1,6-dihydropyridazin-3-yl)-5-hydroxybiphenyl-3-carboxylate (129 mg, 0.26 mmol, see Preparation 32) was suspended in 3.5 mL of water, 3.5 mL of THF and 2 mL of methanol. Lithium hydroxide monohydrate (55 mg, 1.31 mmol) was added and the mixture was stirred at room temperature for 18h. The solvent was evaporated under reduced pressure and the resulting solution was acidified with 5N chlorhydric acid. A solid precipitates, which was filtered, washed with water and dried under reduced pressure at 40°C. 127 mg (99%) of the final compound were obtained.
HPLC/MS (9 min) retention time 6.24 min.
LRMS: *m*/*z* 479 (M+1)

### PREPARATION 34

### 2-(3'-(5-Amino-1-ethyl-6-oxo-1,6-dihydropyridazin-3-yl)biphenyl-3-yl)acetate

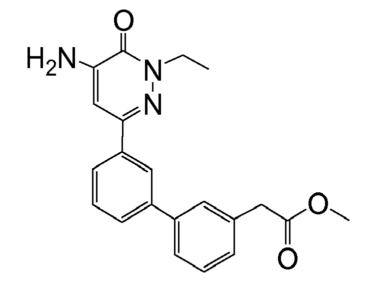

Reaction of 1 g (3.4 mmols) of the title compound of Preparation 6 with the title compound of Preparation 15 (1.03 g, 3.73 mmol) according to the method described in Preparation 11 gave 0.83 g (67%) of the title compound.
HPLC/MS (10 min) retention time 6.61 min
LRMS: *m*/*z* 363 (M⁺)

### PREPARATION 35

### 2-(3'-(5-Amino-1-ethy)-6-oxo-1,6-dihydropyridazin-3-yl)bipheny)-3-yl)acetic acid

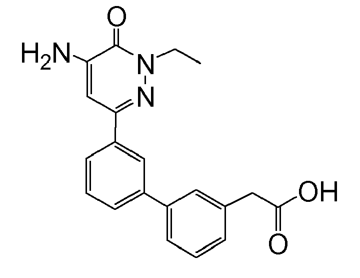

2.11 g (5.81 mmol) of the title compound of Preparation 34 were dissolved in 50 mL of THF and 8.7 mL of 2N NaOH are added. The reaction mixture was heated at 60 °C for 2h. The solvent was evaporated under reduced pressure and the residue was diluted with more 2N NaOH. This liquid was poured into a separation funnel, where this aqueous phase was washed with chloroform. The organic phase was acidified with 5N HCl and a solid was formed, which was extracted with chloroform. The organic phase was washed with brine, dried over magnesium sulphate, filtered and evaporated, to yield 1.88 g (83%) the final compound as a solid.
HPLC/MS (10 min) retention time 6.05 min
LRMS: *m*/*z* 350 (M⁺+1)

### PREPARATION 36

### 2-(3'-(5-Amino-1-ethyl-6-oxo-1,6-dihydropyridazin-3-yl)biphenyl-3-yl)-N-(2-(pyrrolidin-1-yl)ethyl)acetamide

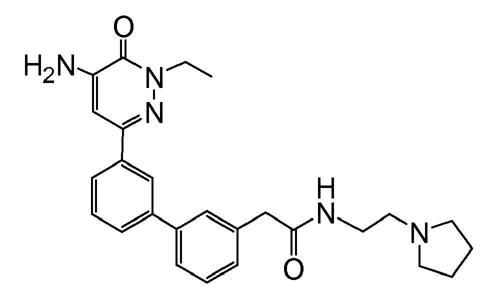

Reaction of 610 mg (1.75 mmols) of the title compound of Preparation 35 with 2-(pyrrolidin-1-yl)ethanamine (440 µL, 3.49 mmol) according to the method described in Example 15 gave 620 mg (80%) of the title compound.
HPLC/MS (10 min) retention time 4.56 min
LRMS: *m*/*z* 446 (M⁺)

### PREPARATION 37

### Methyl 2-(3'-(1-ethyl-6-oxo-5-(quinolin-5-ylamino)-1,6-dihydropyridazin-3-yl)biphenyl-3-ylcarboxamido)acetate

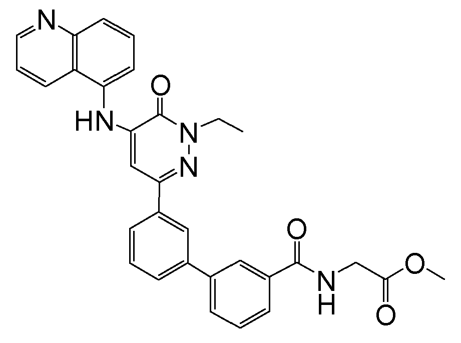

Reaction of 150 mg (0.32 mmols) of the title compound of Example 1 with methyl 2-aminoacetate (58 mg, 0.65 mmol) according to the method described in Example 15 gave 103 mg (48%) of the title compound.
HPLC/MS retention time 6.32 min
LRMS: *m*/*z* 533 (M⁺)

### PREPARATION 38

### ethyl 2-(3'-(5-amino-1-ethyl-6-oxo-1,6-dihydropyridazin-3-yl)biphenyl-4-yl)acetate

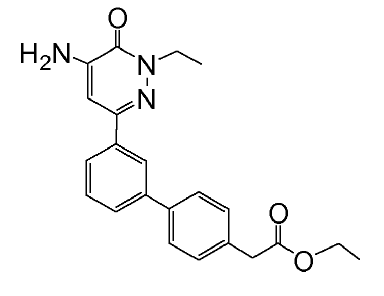

Reaction of 1 g (3.4 mmols) of the title compound of Preparation 6 with the title compound of Preparation 13 (1.09 g, 3.76 mmol) according to the method described in Preparation 11 gave 1.08 g (84%) of the title compound.
HPLC/MS (10 min) retention time 6.83 min
LRMS: *m*/*z* 377 (M⁺)

### PREPARATION 39

### 2-(3'-(5-Amino-l-ethyl -6-oxo-1,6-dihyd ropyridazin-3-yl) biphenyl-4-yl)acetic acid

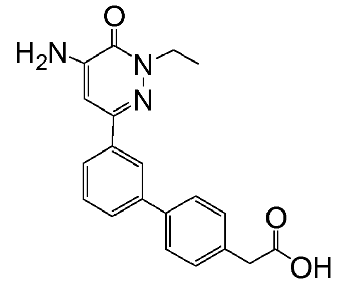

Reaction of 1080 mg (2.86 mmols) of the title compound of Preparation 38 with 2N NaOH (4.29 mL, 8.58 mmol) according to the method described in Preparation 35 gave 1000 mg (100%) of the title compound.
HPLC/MS (10 min) retention time 6.0 min
LRMS: *m*/*z* 349 (M⁺)

### PREPARATION 40

### 2-(3'-(5-Amino-1-ethyl-6-oxo-1,6-dihydropyridazin-3-yl)biphenyl-4-yl)-N-(2-(pyrrolidin-1-yl)ethyl)acetamide

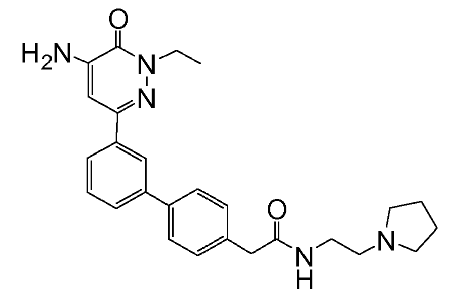

Reaction of 500 mg (1.43 mmols) of the title compound of Preparation 38 with 2-(pyrrolidin-1-yl)ethanamine (327 mg, 2.86 mmol) according to the method described in Example 15 gave 422 mg (66%) of the title compound.
HPLC/MS (10 min) retention time 4.44 min
LRMS: *m*/*z* 446 (M⁺+1)

### PREPARATION 41

### 2-Cyclopentylideneacetonitrile

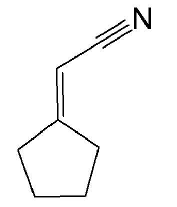

Diethyl cyanomethylphosphonate (0.94 mL, 6.0 mmol) dissolved in 2 mL of diethyl ether is dropwise added to a cooled suspension of 60% sodium hydride (0.24 g, 6.0 mmol), followed by the dropwise addition of cyclopentanone (0.53 mL, 5.99 mmol) in 2 mL of ethyl ether. The mixture is stirred 10 minutes in the ice bath and then overnight at room temperature. Water is cauteously added and it is stirred for half an hour. After extraction with diethyl ether, the organic phase is washed with brine, dried over sodium sulphate and the solvent evaporated under reduced pressure. 0.74 g (99%) of the final product were obtained, pure enough to be used in the next synthetic step without further purification.
¹H NMR (200 MHz, DMSO-*d*₆) δ ppm 1.6-2.0 (m, 4H) 2.4-2.8 (m, 4H) 5.2 (s, 1 H).

### PREPARATION 42

### 2-(1-(Methylamino)cyclopentyl)acetonitrile

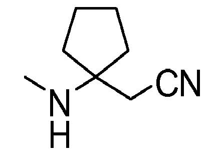

The title compound of Preparation 41 (2.54 g, 23.71 mmol) was placed in glass tube with methanamine (33% in ethanol, 60 ml, 236.8 mmol). The tube was sealed and heated overnight at 60°C. Once the reaction was over, the solvent was evaporated under reduced pressure. 2.82 g (79%) of the final compound were obtained as an oil, pure enough to be used in the next synthetic step without further purification.
¹H NMR (200 MHz, DMSO-*d*₆) δ ppm 1.6-2.0 (m, 8H) 2.4 (s, 3H) 2.6 (s, 2H).

### PREPARATION 43

### 2-(1-(Dimethylamino)cyclopentyl)acetonitrile

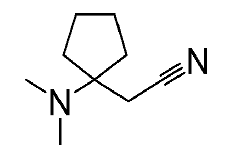

The title product of Preparation 42 (2.82 g, 18.57 mmol) was dissolved in dichloromethane (100 mL). 37% Formaldehide (2.77 mL, 37.20 mmol), sodium cyanoborohydride (1.28 g, 20.37 mmol) and acetic acid (3.19 mL, 55.73 mmol) were added and stirred overnight at room temperature. This solution was neutralized with 8N NaOH and extracted with dichloromethane. The organic phase was washed water and brine, dried over sodium sulfate, filtered and the solvent evaporated under reduced pressure. 2.16 g (76%) of the final product were obtained, pure enough to be used in the next synthetic step without further purification.
¹H NMR (200 MHz, DMSO*-d*₆) δ ppm 1.6-2.0 (m, 8H) 2.4 (s, 6H) 2.5 (s, 2H).

### PREPARATION 44

### 1-(2-Aminoethyl)-N, N-dimethylcyclopentanamine

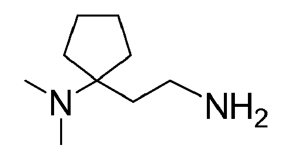

To a cooled suspension of lithium aluminium hydride (1.62 g, 42.56 mmol) in THF (25 ml) concentrated sulfuric acid (1.06 mL, 19.86 mmol) was dropwise added under an atmosphere of argon. After 1 h of stirring at low temperature, the title compound of Preparation 43 (2.16 g, 14.19 mmol) in THF (5 mL) was dropwise added. The ice bath was removed and the reaction mixture was stirred for 4h at room temperature. Then was cooled again and water (1.6 mL) and 4N NaOH (4.8 mL) were successively dropwise added. The reaction mixture was filtered through a pad of diatomaceous earth (Celite ®) and the resulting liquid was dried over magnesium sulfate and the solvent removed under reduced pressure. 1.38 g (62%) of the final compound were obtained, pure enough to be used in the next synthetic step without further purification.
¹H NMR (200 MHz, DMSO*-d*₆) δ ppm 1.4-1.9 (m, 8H) 2.2 (s, 6H) 2.8 (m, 2H).

### PREPARATION 45

### tert-Butyl 4-(3'-(1-ethyl-6-oxo-5-(quinolin-5-ylamino)-1,6-dihydropyridazin-3-yl)biphenyl-3-ylcarboxamido)piperidine-1-carboxylate

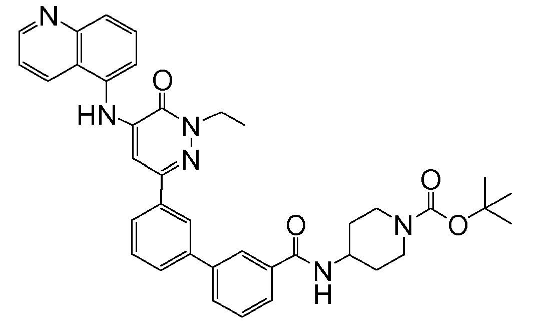

Reaction of the title compound of Example 1 (150 mg, 0.32 mmol) with tert-butyl 4-aminopiperidine-1-carboxylate (130 mg, 0.65 mmol) according to the method described in Example 15 gave 119 mg (55%) of the title compound.
HPLC/MS (9 min) retention time 7.22 min
LRMS: *m*/*z* 573 (M⁺)

### PREPARATION 46

### 3'-(1-Ethyl-6-oxo-5-(quinolin-5-ylamino)-1,6-dihydropyridazin-3-yl)-N-(piperidin-4-yl)biphenyl-3-carboxamide

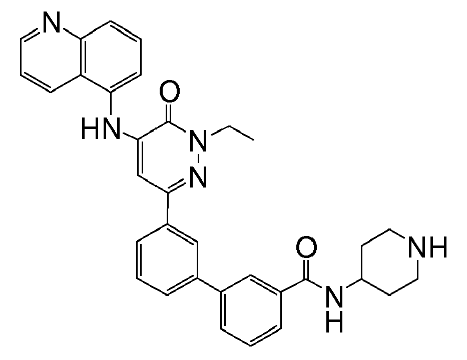

The title compound of Preparation 45 (119 mg, 0.18 mmol) was dissolved in 4M HCl in dioxane (1.19 mL) and stirred overnight at room temperature. The solvent was removed under reduced pressure and the residue was suspended in water. After basification with 2N NaOH, this aqueous phase was extracted with dichloromethane. The organic phase was washed with water and brine, dried over magnesium sulfate, filtered and evaporated under reduced pressure. 88 mg (81 %) of the final product as a solid were obtained.
HPLC/MS (9 min) retention time 5.05 min
LRMS: *m*/*z* 545 (M⁺+1)

### PREPARATION 47

### 3-bromo-5-(methoxycarbonyl)benzoic acid

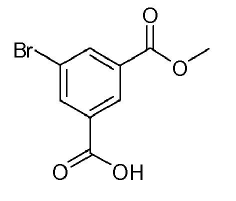

To a solution of dimethyl 5-bromoisophthalate (2.80 g, 10.25mmols) in methanol (18.5 mL) was added a solution of potassium hydroxide (0.29 g, 5.13mmols) in 3.7 mL of water. The mixture was stirred overnight under reflux.

The mixture was partitioned between water and ethyl ether. The aqueous phase was extracted twice with ether. The aqueous was then acidified to acid pH with 5N HCl and was extracted three times with ethyl ether. The combined organics were washed with brine and dried over sodium sulphate. Purification by chromatography (hexane 100% to ethyl acetate 100%) gave 0.700 g (2.70 mmol, 26%) of the title compound.
HPLC/MS (10 min) retention time 5.79 min
LRMS: *m*/*z* 259 (M-1)

### PREPARATION 48

### Methyl 3-bromo-5-[(cyclopropylamino)carbonyl]benzoate

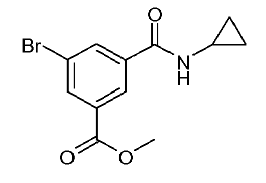

Reaction of 0.6 g (3.22 mmol) of title compound of preparation 47 with 322 µL (4.63 mmol) of cyclopropanamine according to the method described in Example 15 gave 0.66 g (96%) of the title compound.
HPLC/MS (10 min) retention time 5.64 min
LRMS: *m*/*z* 296 (M-1)

### PREPARATION 49

### Methyl 3-[(cyclopropylamino)carbonyl]-5-(4,4,5,5-tetramethyl-1,3,2-dioxaborolan-2-yl)benzoate

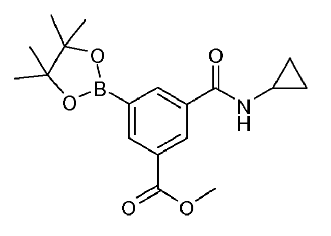

In a Schlenk tube, a mixture of methyl 3-bromo-5-[(cyclopropylamino)carbonyl]benzoate (0.66 g, 2.21 mmols, see Preparation 48), bis(pinacolato)diboron (1.12 g, 4.43 mmol) and potassium acetate (1.38 g, 14 mmol) was dissolved in dioxane (40 mL). The mixture was purged (vacuum-argon three times) and [1,1-Bis (diphenylphosphino) ferrocene] dichloropalladium (II) dichloromethane complex (0.19 g, 0.23 mmol) and 1,1'-bis (diphenylphospheno) ferrocene (0.13 g, 0.23 mmol) were added. The mixture was purged again (vacuum-argon three times) and stirred at 90 °C for 18 h.

The suspension was filtered off and the filtrated diluted with water and extracted three times with ethyl acetate. The combined organic layers were washed with water and brine, and dried over anhydrous sodium sulphate. Solvent was removed in vacuum and the residue was purified by the SP1® automated purification system to give 0.572 g (75%) of the desired compound.
HPLC/MS (10 min) retention time 6.31 min
LRMS: m/z 346 (M+1)

### PREPARATION 50

### 5-Bromo-N,N'-dicyclopropylisophthalamide

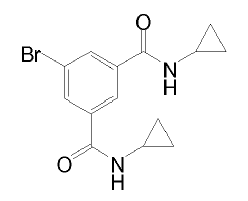

Cyclopropanamine (1.01ml, 14.49 mmol) was added to a solution of 5-bromoisophthalic acid (710 mg, 2.90 mmol), 1-hydroxybenzotriazole hydrate (1.17 g, 8.69 mmol) and N-ethyl-N'-(3-dimethylaminopropyl)carbodiimide hydrochloride (1.66 g, 8.69 mmol) in 20 mL dimethylformamide. The mixture was stirred at room temperature for 2 h.

The solvent was removed under reduced pressure and the residue dissolved in 4% sodium bicarbonate solution precipitating a solid. The solid was collected by filtration and the solid give 0.65 g (70%) of the title compound.
HPLC/MS (10 min) retention time 5.03 min
LRMS: *m*/*z* 323 (M-1)

### PREPARATION 51

### N,N'-Dicyclopropyl-5-(4,4,5,5-tetramethyl-1,3,2-dioxaborolan-2-yl)isophthalamide

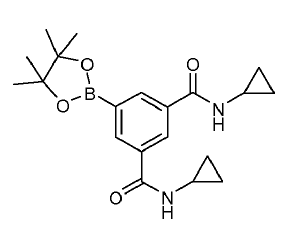

Reaction of 0.780 g (2.41 mmols) of the title compound of Preparation 50 with 1.22 g (4.83 mmol) bis(pinacolato)diboron according to the method described in Preparation 13 gave 0.640 g (72%) of the title compound.
HPLC/MS (10 min) retention time 5.77 min
LRMS: *m*/*z* 371 (M+1)

### PREPARATION 53

### Methyl 5-(cyclopropylcarbamoyl)-3'-(1-ethyl-6-oxo-5-(quinolin-5-ylamino)-1,6-dihydropyridazin-3-yl)biphenyl-3-carboxylate

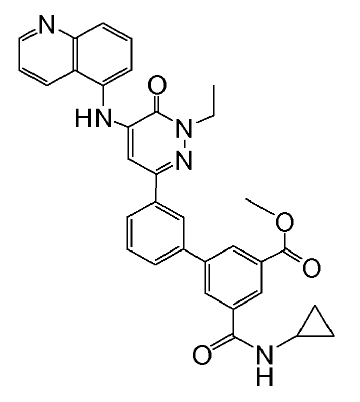

Reaction of 0.90 g (2.14 mmols) of the title compound of Preparation 8 with 0.89 g (2.58 mmol) of the title compound of Preparation 49 according to the method described in Preparation 11 gave 0.41 g (34%) of the title compound.
HPLC/MS (10 min) retention time 9.22 min
LRMS: *m*/*z* 559 (M+1)

### PREPARATION 54

### 5-(Cyclopropylcarbamoyl)-3'-(1-ethyl-6-oxo-5-(quinolin-5-ylamino)-1,6-dihydropyridazin-3-yl)biphenyl-3-carboxylic acid

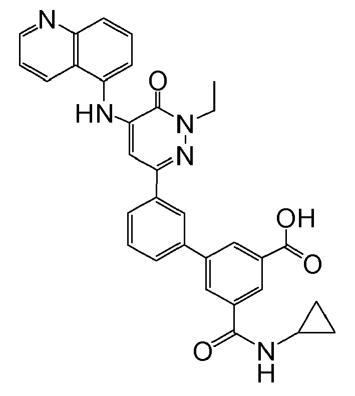

Reaction of 410 mg (0.73 mmols) of the title compound of Preparation 53 with 154 mg (3.67 mmol) of the lithium hydroxide monohydrate according to the method described in Preparation 26 gave 290 mg (73%) of the title compound.
HPLC/MS (9 min) retention time 6.25 min
LRMS: *m*/*z* 546 (M+1)

### EXAMPLE 1

### 3'-[1-ethyl-6-oxo-5-(quinolin-5-ylamino)-1,6-dihydropyridazin-3-yl]biphenyl-3-carboxylic acid

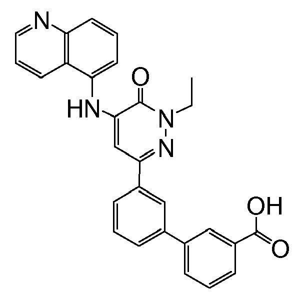

Ethyl 3'-(1-ethyl-6-oxo-5-(quinolin-5-ylamino)-1,6-dihydropyridazin-3-yl)biphenyl-3-carboxylate (1.36 g, 2.77 mmol, see Preparation 9) was suspended in 15 mL of water and 30 mL of THF. Lithium hydroxide monohydrate (0.58 g, 13.86 mmol) dissolved in 15 mL of water was added and the mixture was stirred at room temperature for 18h. The solvent was evaporated under reduced pressure and the resulting solution was acidified with 2N chlorhydric acid. A solid precipitates, which was filtered, washed with water and dried under reduced pressure at 40°C. 1.19 g (93%) of the final compound were obtained.
HPLC/MS retention time 17.3 min.
LRMS: m/z 461 (M-1)

### EXAMPLE 2

### 3'-[1-ethyl-5-(isoquinolin-4-ylamino)-6-oxo-1,6-dihydropyridazin-3-yl]biphenyl-3-carboxylic acid

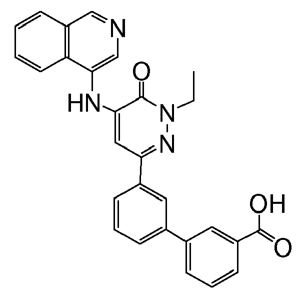

Reaction of the title compound of Preparation 11 (290 mg, 0.47 mmol) with lithium hydroxide monohydrate (100 mg, 2.34 mmol) according to the method described in Example 1 gave 20 mg (9%) of the title compound.
HPLC/MS retention time 17.0 min
LRMS: *m*/*z* 463 (M+)

### EXAMPLE 3

### 3'-[1-ethyl-5-(isoquinolin-4-ylamino)-6-oxo-1,6-dihydropyridazin-3-yl]biphenyl-3-carboxamide

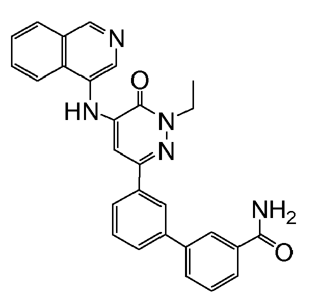

In a Schlenk tube, a mixture of 6-(3-bromophenyl)-2-ethyl-4-(isoquinolin-4-ylamino)pyridazin-3(2H)-one (430mg, 1.1 mmol, see Preparation 10), 3-carbamoylphenylboronic acid (250 mg, 1.52 mmol) and 2M cessium carbonate solution (1.52 ml, 3.03 mmol) was dissolved in dioxane (10 ml). The mixture was purged (vacuum-argon three times) and [1,1-bis (diphenylphosphino) ferrocene] dichloropalladium (II) dichloromethane complex (50 mg, 0.06 mmol) was added. The mixture was purged again (vacuum-argon three times) and stirred at 90 °C for 18 h. The suspension was filtered off and the filtrated diluted with water and extracted three times with ethyl acetate. The combined organic layers were washed with water and brine, and dried over anhydrous sodium sulphate. Solvent was removed in vacuum and the residue was recrystalized three times from a mixture CH₂Cl₂/MeOH 1:1 to yield 0.29 g (62%) of the desired compound.
HPLC/MS retention time 15.0 min.
LRMS: *m*/*z* 462 (M+H⁺)

### EXAMPLE 4

### 3'-[1-ethyl-6-oxo-5-(quinolin-5-ylamino)-1,6-dihydropyridazin-3-yl]biphenyl-3-carboxamide

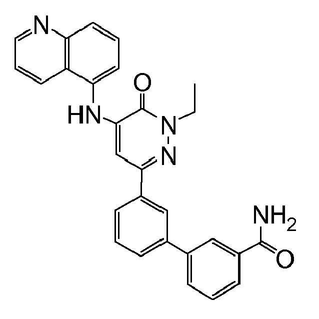

Reaction of the title compound of Preparation 8 (2.4 mg, 5.79 mmol) with 3-carbamoylphenylboronic acid (1.4g, 8.67 mmol) according to the method described in Example 3 gave 1.8 g (68%) of the title compound.
¹H NMR (400 MHz, DMSO-d₆) δ ppm: 1.42 (t, 3H), 4.30 (q, 2H), 6.61 (s, 1H), 7.3-8.2 (m, 14 H), 8.35 (d, 1H), 8.95(m, 1H), 9.17(s, 1H)
HPLC/MS retention time 14.9 min
LRMS: *m*/*z* 463 (M+)

### EXAMPLE 5

### 3'-[1-ethyl-5-(isoquinolin-4-ylamino)-6-oxo-1,6-dihydropyridazin-3-yl]biphenyl-4-carboxylic acid

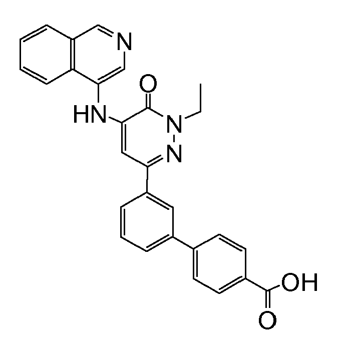

Reaction of the title compound of Preparation 12 (1.04 g, 2.12 mmol) with lithium hydroxide monohydrate (0.44 g, 10.60 mmol) according to the method described in Example 1 gave 0.46 g (57%) of the title compound.
¹H NMR (400 MHz, DMSO-d₆) δ ppm: 1.46 (t, 3H), 4.32 (q, 2H), 6.51 (s, 1H), 7.43 (t, 1H), 7.49 (m, 2H), 7.54 (d, 1H), 7.63 (d, 1H), 7.75 (t, 1H), 7.83 (m, 2H), 7.93 (m, 3H), 8.22 (d, 1H), 8.62 (d, 1H), 9.26 (s, 1H).
HPLC/MS retention time: 16.74
LRMS: *m*/*z* 463 (M+)

### EXAMPLE 6

### {3'-[1-ethyl-6-oxo-5-(quinolin-5-ylamino)-1,6-dihydropyridazin-3-yl]biphenyl-4-yl}acetic acid

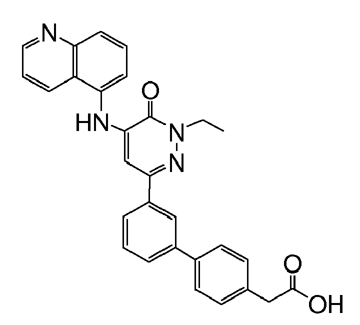

Reaction of the title compound of Preparation 14 (230 mg, 0.46 mmol) with lithium hydroxide monohydrate (96 mg, 2.29 mmol) according to the method described in Example 1 gave 177 mg (82%) of the title compound.
HPLC/MS retention time 16.7 min
LRMS: *m*/*z* 477 (M+)

### EXAMPLE 7

### 3'-{1-ethyl-5-[(1-oxidoquinolin-5-yl)amino]-6-oxo-1,6-dihydropyridazin-3-yl}biphenyl-3-carboxamide

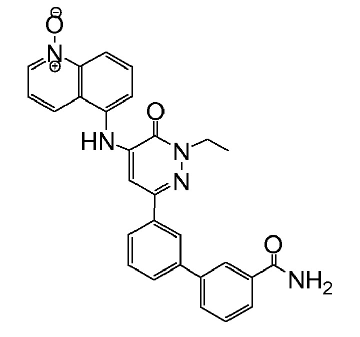

3'-(1-Ethyl-6-oxo-5-(quinolin-5-ylamino)-1,6-dihydropyridazin-3-yl)biphenyl-3-carboxamide (100 mg, 0.21 mmol, see Example 4) in dichloromethane (10 mL was added dropwise to a ice-bath cooled solution of 3-chloroperbenzoic acid (mCPBA) (0.06 g, 0.29 mmol) in dichloromethane (1.5 mL). Further portions of mCPBA are added until no starting material was detected. The reacion mixture was poured into a 25% KHSO₄ solution, the organic phase was separated and washed with 4% NaHCO₃ and water, dried over sodium sulfate, filtered and the solvent evaporated under reduced pressure. The residue was recristalized in 10 mL of a mixture CH₂Cl₂/MeOH 8/2 to yield 46 mg (44%) of the final compound.
HPLC/MS retention time 13.8 min
LRMS: *m*/*z* 478 (M+H⁺)

### EXAMPLE 8

### {3'-[1-ethyl-5-(isoquinolin-4-ylamino)-6-oxo-1,6-dihydropyridazin-3-yl]biphenyl-3-yl}acetic acid

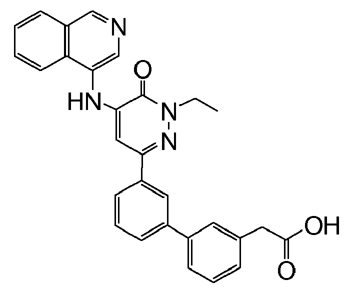

A suspension of the title compound of preparation 10 (0.15 g, 0.36 mmol), an the boronate of preparation 16 (0.11 g, 0.43 mmol), Pd(PPh₃)₄ (0.01 g, 0.01 mmol) and K₂CO₃ (0.08 g, 0.54 mmol) in dioxane/water (3 mL, 1:1) was microwave-irradiated (140 °C, 10 min, 70 W). The residue was filtered through Celite, washed with MeOH, and concentrated. The crude residue was chromatographed on reverse phase (System Biotage SP1; A: 50% MeOH/CH₃CN; B: 0.5 mL NH₃/L H₂O) to afford the titled compound (50 mg, yellow solid, yield: 29%).
¹H NMR (DMSO-d₆, 400 MHz, 80 °C) δ ppm: 9.22 (s, 1H), 8.60 (s, 1H), 8.21 (d, J = 7.9 Hz, 1H), 7.93 (d, J = 8.3 Hz, 1H), 7.79 (m, 3H), 7.57 (d, J = 7.9 Hz, 1H), 7.50 (d, J = 7.9 Hz, 1H), 7.42 (m, 2H), 7.24 (m, 3H), 6.47 (s, 1H), 4.32 (q, J = 7.5 Hz, 2H), 3.24 (s, 2H), 1.48 (t, J = 7.0 Hz, 3H)
HPLC/MS retention time 16.9 min
LRMS: *m*/*z* 477 (M+H⁺)

### EXAMPLE 9

### 3'-[1-ethyl-6-oxo-5-(quinolin-5-ylamino)-1,6-dihydropyridazin-3-yl]biphenyl-4-carboxylic acid

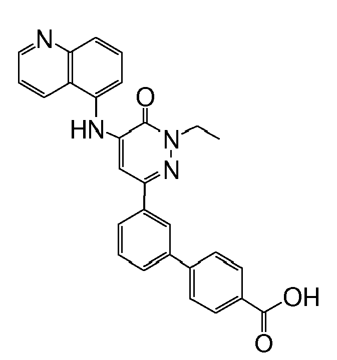

Reaction of the title compound of Preparation 17 (655 mg, 1.34 mmol) with lithium hydroxide monohydrate (280 mg, 6.68 mmol) according to the method described in Example 1 gave 609 mg (98 %) of the title compound.
¹H NMR (200 MHz, DMSO-d₆) δ ppm 1.43 (t, 3 H) 4.18 - 4.44 (m, 2 H) 7.50 - 7.82 (m, 9 H) 7.87 - 8.10 (m, 5 H) 8.38 (s, 1 H) 8.98 (s, 1 H) 9.17 (s, 1 H) HPLC/MS retention time 16.8 min
LRMS: *m*/*z* 463 (M+H⁺)

### EXAMPLE 10

### {3'-[1-ethyl-6-oxo-5-(quinolin-5-ylamino)-1,6-dihydropyridazin-3-yl]biphenyl-3-yl}acetic acid

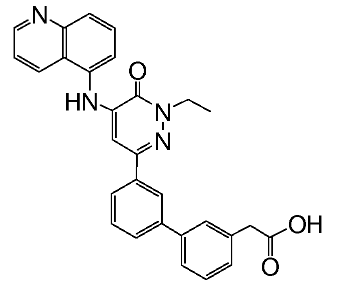

A suspension of preparation example 8 (0.15 g, 0.36 mmol), with the boronate of preparation 16 (0.11 g, 0.43 mmol), Pd(PPh₃)₄ (0.01 g, 0.01 mmol) and K₂CO₃ (0.08 g, 0.54 mmol) in dioxane/water (3 mL, 1:1) was microwave-irradiated (140 °C, 10 min, 70 W). The residue was filtered through Celite, washed with MeOH, and concentrated. The crude residue was chromatographed on reverse phase (System Biotage SP1; A: 50% MeOH/CH₃CN; B: 0.5 mL NH₃/L H₂O) to afford the titled compound (117 mg, yellow solid, yield: 69%).
¹H NMR (DMSO-d₆, 400 MHz, 80 °C) δ ppm: 8.95 (d, J = 5.7 Hz, 1H), 8.35 (d, J = 9.6 Hz, 1H), 7.97 (d, J = 8.3 Hz, 1H), 7.83 (m, 2H), 7.69 (d, J = 7.5 Hz, 1H), 7.50 (m, 6H), 7.26 (m, 3H), 4.32 (q, J = 7.0 Hz, 2H), 3.27 (s, 2H), 1.45 (t, J = 7.0 Hz, 3H)
HPLC/MS retention time 16.6 min
LRMS: *m*/*z* 477 (M+H⁺)

### EXAMPLE 11

### 3'-{1-ethyl-5-[(1-oxidoquinolin-5-yl)amino]-6-oxo-1,6-dihydropyridazin-3-yl}biphenyl-3-carboxylic acid

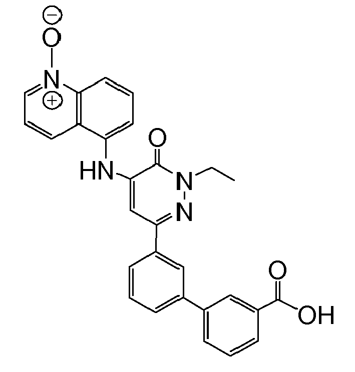

Reaction of the title compound of Example 1 with mCPBA according to the method described in Example 7 gave the title compound.
HPLC/MS retention time 15.6 min
LRMS: *m*/*z* 479 (M+H⁺)

### EXAMPLE 12

### N-[2-(dimethylamino)ethyl]-3'-[1-ethyl-6-oxo-5-(quinolin-5-ylamino)-1,6-dihydropyridazin-3-yl]biphenyl-3-carboxamide

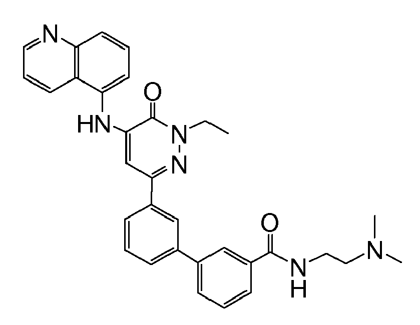

Reaction of the title compound of Preparation 8 (300 mg, 0.71 mmol) with N-[2-(dimethylamino) ethyl] -3-(4,4,5,5-tetramethyl -1,3,2-dioxaborolan-2-yl) benzamide (295 mg, 0.93 mmol, see Preparation 19) according to the method described in Example 3 gave 135 mg (36 %) of the title compound.
¹H NMR (200 MHz, DMSO-d₆) δ ppm: 1.43 (t, 3H), 2.21 (s, 6H), 2.44 (t, 2H), 3.39 (m, 2H), 4.30 (q, 2H), 6.60 (s, 1H), 7.4 - 8.0 (m, 10H), 8.06 (bs, 1H), 8.19 (s, 1H), 8.34 (d, 1H), 8.51 (m, 1H), 8.95 (dd, 1H), 9.15 (s, 1H).
HPLC/MS retention time 10.2 min
LRMS: *m*/*z* 533 (M+H⁺)

### EXAMPLE 13

### N-cyclopropyl-3'-[1-ethyl-6-oxo-5-(quinolin-5-ylamino)-1,6-dihydropyridazin-3-yl]biphenyl-3-carboxamide

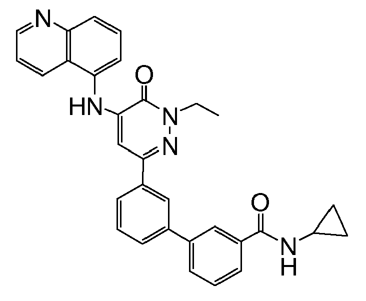

Reaction of the title compound of Preparation 8 (200 mg, 0.47 mmol) with 3-(cyclopropylcarbamoyl)phenylboronic acid (146 mg, 0.41 mmol) according to the method described in Example 3 gave 109.7 mg (46 %) of the title compound.
¹H NMR (200 MHz, DMSO-d₆) δ ppm: 0.58 (m, 2H), 0.72 (m, 2H), 1.42 (t, 3H), 2.86 (m, 1H), 4.30 (q, 2H), 6.60 (s, 1H), 7.4-8.1 (m, 12H), 8.34 (d, 1H), 8.54(d, 1H), 8.95 (dd, 1H), 9.17 (s, 1H)
HPLC/MS retention time 16.3 min
LRMS: *m*/*z* 502 (M+H⁺)

### EXAMPLE 14

### N-cyclopropyl-3'-[1-ethyl-5-(isoquinolin-4-ylamino)-6-oxo-1,6-dihydropyridazin-3-yl]biphenyl-3-carboxamide

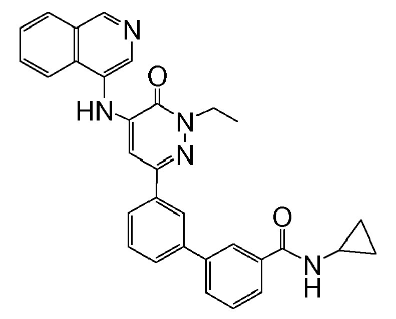

Reaction of the title compound of Preparation 10 (200 mg, 0.47 mmol) with 3-(cyclopropylcarbamoyl)phenylboronic acid (146 mg, 0.41 mmol) according to the method described in Example 3 gave 119 mg (50 %) of the title compound.
HPLC/MS retention time 16.4 min
LRMS: *m*/*z* 502 (M+H⁺)

### EXAMPLE 15

### 3'-[1-ethyl-6-oxo-5-(quinolin-5-ylamino)-1,6-dihydropyridazin-3-yl]-N-(2-morpholin-4-ylethyl)biphenyl-3-carboxamide

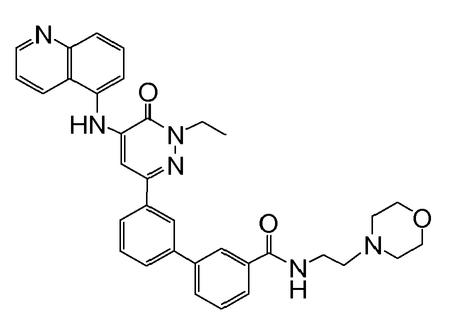

2-Morpholinoethanamine (68 mg, 0.52 mmol) was added to a solution of acid 3'-(1-ethyl-6-oxo-5-(quinolin-5-ylamino)-1,6-dihydropyridazin-3-yl)biphenyl-3-carboxylic (120 mg, 0.26 mmol), 1-hydroxybenzotriazole hydrate (53 mg, 0.39 mmol) and N-ethyl-N'-(3-dimethylaminopropyl)carbodiimide hydrochloride (75 mg, 0.39 mmol) in 3 mL of dimethylformamide. The mixture was stirred at room temperature for 3 h.

The solvent was removed under reduced pressure and the residue dissolved in 4% sodium bicarbonate solution. The aqueous phase was extracted three times with ethyl acetate. The combined organic layers were washed with water and brine, and dried over anhydrous sodium sulphate. Solvent was removed in vacuum to give 102 mg (176 mmol, 67%) of the title compound.
HPLC/MS retention time 11.0 min
LRMS: *m*/*z* 575 (M+H⁺)

### EXAMPLE 16

### 3'-[1-ethyl-6-oxo-5-(quinolin-5-ylamino)-1,6-dihydropyridazin-3-yl]-N-(2-piperidin-1-ylethyl)biphenyl-3-carboxamide

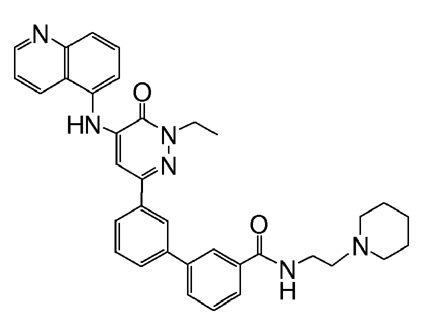

Reaction of the title compound of Example 1 (120 mg, 0.26 mmol) with 2-(piperidin-1-yl)ethanamine (67 mg, 0.52 mmol) according to the method described in Example 15 gave 101 mg (68 %) of the title compound.
¹H NMR (200 MHz, CDCl₃) δ ppm: 1.54 (t, 3H), 1.4-1.7 (m, 6H), 2.43 (m, 4H), 2.56 (t, 2H), 3.54 (q, 2H), 4.43 (q, 2H), 6.75 (s, 1H), 7.4 - 8.1 (m, 13H), 8.39 (m, 1H), 8.99(dd, 1H).
HPLC/MS retention time 11.4 min
LRMS: *m*/*z* 573 (M+H⁺)

### EXAMPLE 17

### 3'-[1-ethyl-5-(isoquinolin-4-ylamino)-6-oxo-1,6-dihydropyridazin-3-yl]-N-(2-morpholin-4-ylethyl)biphenyl-3-carboxamide

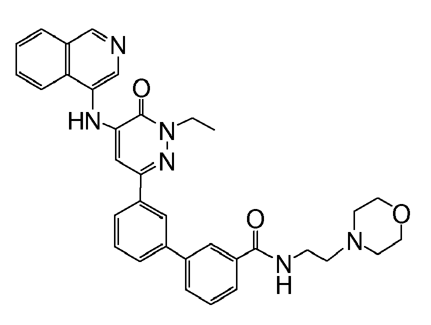

Reaction of the title compound of Example 2 (200 mg, 0.43 mmol) with 2-morpholinoethanamine (113 mg, 0.87 mmol) according to the method described in Example 15 gave 213 mg (84 %) of the title compound.
¹H NMR (200 MHz, CDCl₃) δ ppm: 1.55 (t, 3H), 2.51 (m, 4H), 2.62 (t, 2H), 3.58 (q, 2H), 3.72 (m, 4H), 4.43 (q, 2H), 6.82 (s, 1H), 7.4 - 8.2 (m, 13H), 8.68 (s, 1H), 9.19 (s, 1H).
HPLC/MS retention time 11.2 min
LRMS: *m*/*z* 575 (M+H⁺)

### EXAMPLE 18

### 3'-[1-ethyl-5-(isoquinolin-4-ylamino)-6-oxo-1,6-dihydropyridazin-3-yl]-N-(2-piperidin-1-ylethyl)biphenyl-3-carboxamide

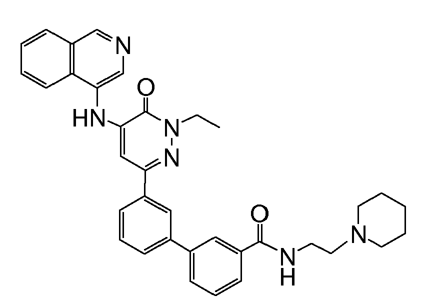

Reaction of the title compound of Example 2 (200 mg, 0.43 mmol) with 2-(piperidin-1-yl)ethanamine (111 mg, 0.87 mmol) according to the method described in Example 15 gave 213 mg (84 %) of the title compound.
HPLC/MS retention time 11.5 min
LRMS: *m*/*z* 573 (M+H⁺)

### EXAMPLE 19

### N-cyclobutyl-3'-[1-ethyl-5-(isoquinolin-4-ylamino)-6-oxo-1,6-dihydropyridazin-3-yl]biphenyl-3-carboxamide

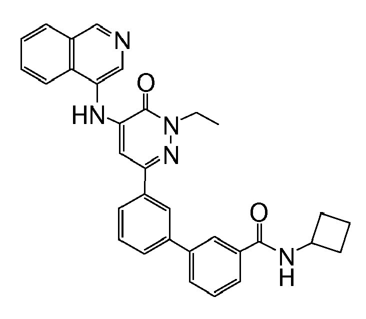

Reaction of the title compound of Example 2 (200 mg, 0.43 mmol) with cyclobutanamine (62 mg, 0.87 mmol) according to the method described in Example 15 gave 171 mg (75 %) of the title compound.
¹H NMR (200 MHz, CDCl₃) δ ppm: 1.54 (t, 3H), 1.6 - 2.1 (m, 4H), 1.94 (m, 2H), 4.42 (q, 2H), 4.62 (q, 1H), 6.81 (s, 1H), 7.4-8.2 (m, 13H), 8.68 (s, 1H), 9.18 (s, 1H).
HPLC/MS retention time 17.3 min
LRMS: *m*/*z* 516 (M+H⁺)

### EXAMPLE 20

### 3'-[1-ethyl-5-(isoquinolin-4-ylamino)-6-oxo-1,6-dihydropyridazin-3-yl]-N-(2-morpholin-4-ylethyl)biphenyl-4-carboxamide

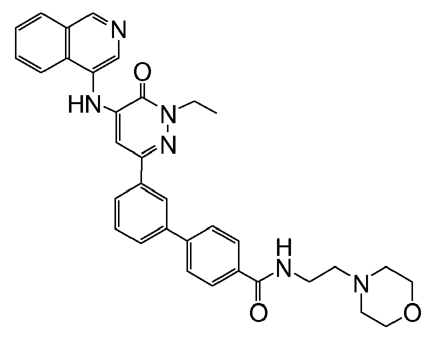

Reaction of the title compound of Example 5 (100 mg, 0.20 mmol) with 2-morpholinoethanamine (56 mg, 0.43 mmol) according to the method described in Example 15 gave 63 mg (64 %) of the title compound.
HPLC/MS retention time 11.4 min
LRMS: *m*/*z* 575 (M+H⁺)

### Example 21

### N-cyclopropyl-3'-[1-ethyl-6-oxo-5-(quinolin-5-ylamino)-1,6-dihydropyridazin-3-yl]-5-hydroxybiphenyl-3-carboxamide

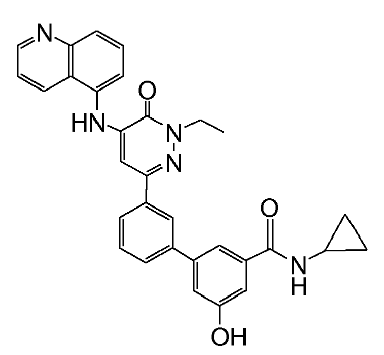

The title compound of Preparation 26 (116 mg, 0.24 mmol) is dissolved in dimethylformamide (4 mL) and cyclopropanamine (30 µL, 0.43 mmol) and diisopropylethylamine (DIEA) (150 µL, 0.86 mmol) were added. Under a nitrogen atmosphere, O - (7 - azabenzotriazol - 1 - yl) - *N, N, N', N'-* tetramethyl uronium hexafluoro phosphate (HATU) (138 mg, 0.36 mmol) are added and the mixture was stirred overnight. The solvent was removed under reduced pressure and the residue was suspended in 4% NaHCO₃. The resulting solid was filtered and purified by the SP1® automated purification system (reversed phase, 25S from C18 Waters columne, with a polarity gradient from 100% water to ACN/MeOH (1:1). 62.4 mg (49%) of the final compound were obtained.
HPLC/MS retention time 14.8 min
LRMS: *m*/*z* 516 (M-H⁺)

### EXAMPLE 22

### 3'-[1-ethyl-5-(isoquinolin-4-ylamino)-6-oxo-1,6-dihydropyridazin-3-yl]-N-(2-piperidin-1-ylethyl)biphenyl-4-carboxamide

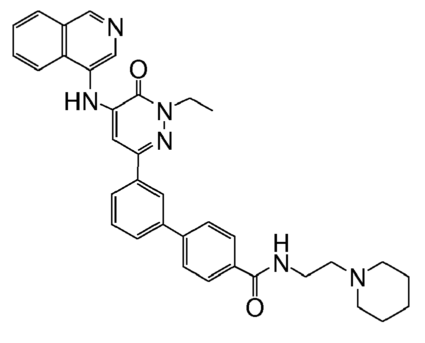

Reaction of the title compound of Example 5 (100 mg, 0.2 mmol) with 2-(piperidin-1-yl)ethanamine (56 mg, 0.44 mmol) according to the method described in Example 15 gave 57 mg (49 %) of the title compound.
¹H NMR (200 MHz, DMSO-d₆) δ ppm: 1.55 (t, 3H), 1.4-1.7 (m, 6H), 2.06 (m, 4H), 3.20 (t, 2H), 3.93 (m, 2H), 4.43 (q, 2H), 6.77 (s, 1H), 7.4 - 8.2 (m, 12H), 8.67 (m, 1H), 8.94 (m, 1H), 9.21 (s, 1H).
HPLC/MS retention time 11.8 min
LRMS: *m*/*z* 573 (M+H⁺)

### EXAMPLE 23

### N-cyclobutyl-3'-[1-ethyl-5-(isoquinolin-4-ylamino)-6-oxo-1,6-dihydropyridazin-3-yl]biphenyl-4-carboxamide

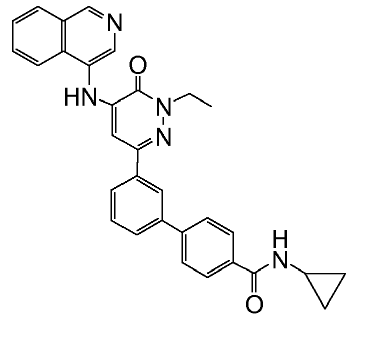

Reaction of the title compound of Example 5 (120 mg, 0.26 mmol) with cyclobutanamine (37 mg, 0.52 mmol) according to the method described in Example 15 gave 41 mg (30 %) of the title compound.
HPLC/MS (30 min) retention time 17.4 min
LRMS: *m*/*z* 516 (M+H⁺)

### EXAMPLE 24

### N-cyclopentyl-3'-[1-ethyl-5-(isoquinolin-4-ylamino)-6-oxo-1,6-dihydropyridazin-3-yl]biphenyl-4-carboxamide

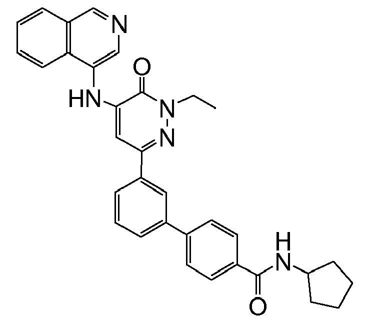

Reaction of the title compound of Example 5 (125 mg, 0.27 mmol) with cyclopentanamine (46 mg, 0.54 mmol) according to the method described in Example 15 gave 88 mg (61%) of the title compound.
HPLC/MS retention time 18.0 min
LRMS: *m*/*z* 530 (M+H⁺)

### EXAMPLE 25

### N-cyclobutyl-3'-[1-ethyl-6-oxo-5-(quinolin-5-ylamino)-1,6-dihydropyridazin-3-yl]biphenyl-3-carboxamide

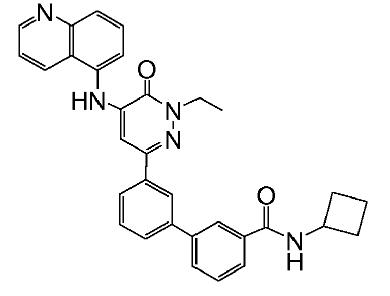

Reaction of the title compound of Example 1 (100 mg, 0.22 mmol) with cyclobutanamine (31 mg, 0.44 mmol) according to the method described in Example 15 gave 69 mg (61 %) of the title compound.
¹H NMR (200 MHz, DMSO-d₆) δ ppm: 1.43 (t, 3H), 1.6 - 2.4 (m, 6H), 4.30 (q, 2H), 4.45 (q, 1H), 6.60 (s, 1H), 7.4 - 8.1 (m, 12H), 8.35 (d, 1H), 8.69 (d, 1H), 8.95 (m, 1H), 9.15 (s, 1H).
HPLC/MS retention time 17.2 min
LRMS: *m*/*z* 516 (M+H⁺)

### EXAMPLE 26

### 3'-[1-ethyl-6-oxo-5-(quinolin-5-ylamino)-1,6-dihydropyridazin-3-yl]-N-(3-hydroxybenzyl)biphenyl-3-carboxamide

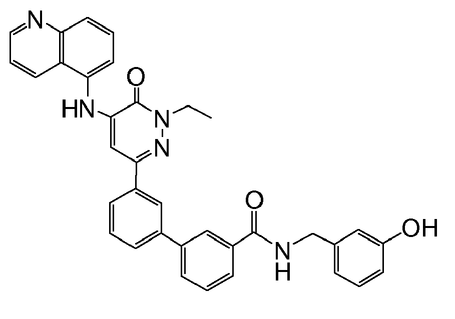

Reaction of the title compound of Example 1 (100 mg, 0.22 mmol) with 3-(aminomethyl)phenol (53 mg, 0.43 mmol) according to the method described in Example 15 gave 46 mg (37 %) of the title compound.
HPLC/MS retention time 16.5 min
LRMS: *m*/*z* 568 (M+H⁺)

### EXAMPLE 27

### N-cyclopentyl-3'-[1-ethyl-6-oxo-5-(quinolin-5-ylamino)-1,6-dihydropyridazin-3-yl]biphenyl-3-carboxamide

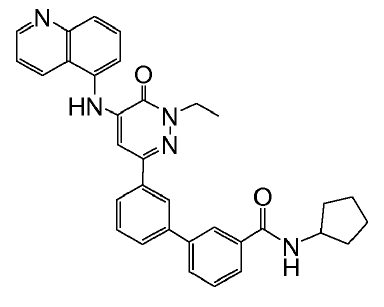

Reaction of the title compound of Example 1 (100 mg, 0.22 mmol) with cyclopentanamine (37 mg, 0.43 mmol) according to the method described in Example 15 gave 49 mg (43 %) of the title compound.
¹H NMR (200 MHz, DMSO-d₆) δ ppm: 1.43 (t, 3H), 1.3 - 2.0 (m, 8H), 4.1 - 4.4 (m, 3H), 6.60 (s, 1H), 7.4 - 8.1 (m, 12H), 8.34 (m, 2H), 8.94 (m, 1H), 9.16 (s, 1H).
HPLC/MS retention time 17.8 min
LRMS: *m*/*z* 530 (M+H⁺)

### EXAMPLE 28

### 1-({3'-[1-ethyl-6-oxo-5-(quinolin-5-ylamino)-1,6-dihydropyridazin-3-yl]biphenyl-3-yl}carbonyl)piperidine-4-carboxamide

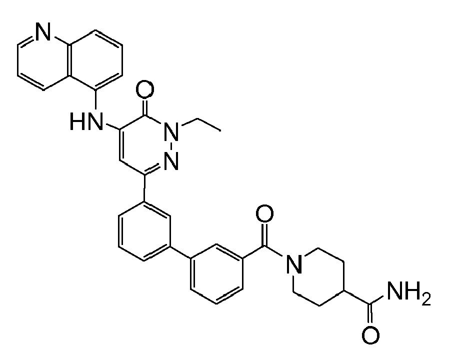

Reaction of the title compound of Example 1 (100 mg, 0.22 mmol) with piperidine-4-carboxamide (49 mg, 0.38 mmol) according to the method described in Example 15 gave 104 mg (95 %) of the title compound.
HPLC/MS retention time 14.4 min
LRMS: *m*/*z* 573 (M+H⁺)

### EXAMPLE 29

### 3'-[1-ethyl-6-oxo-5-(quinolin-5-ylamino)-1,6-dihydropyridazin-3-yl]-N-{7-[(2-hydroxyethyl)(methyl)amino]heptyl}biphenyl-3-carboxamide

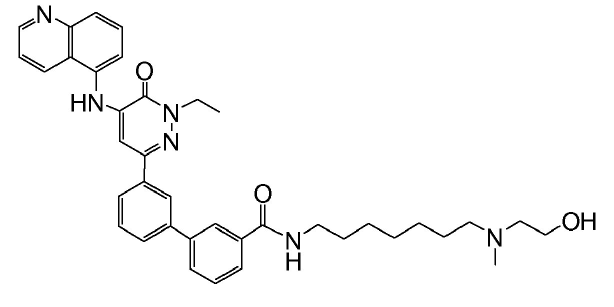

Reaction of the title compound of Example 1 (100 mg, 0.22 mmol) with 2-((7-aminoheptyl)(methyl)amino)ethanol (82 mg, 0.44 mmol, see Preparation 28) according to the method described in Example 15 gave 75 mg (55 %) of the title compound.
¹H NMR (200 MHz, CDCl3) δ ppm: 1.2-1.8 (m, 10H), 1.54 (t, 3H), 2.57 (s, 3H), 2.78 (m, 2H), 2.87 (m, 2H), 3.46 (q, 2H), 3.80 (m, 2H), 4.42 (q, 2H), 6.40 (t, 1H), 6.75 (s, 1H), 7.4-8.1 (m, 13H), 8.39 (dd, 1H), 8.52(s, 1H), 8.99(dd, 1H).
HPLC/MS retention time 12.3 min
LRMS: *m*/*z* 633 (M+H⁺)

### EXAMPLE 30

### N-cyclopentyl-3'-[1-ethyl-5-(isoquinolin-4-ylamino)-6-oxo-1,6-dihydropyridazin-3-yl]biphenyl-3-carboxamide

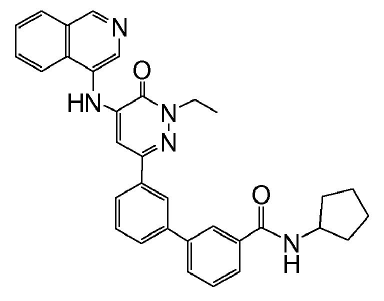

Reaction of the title compound of Example 2 (100 mg, 0.22 mmol) with cyclopentanamine (37 mg, 0.43 mmol) according to the method described in Example 15 gave 46 mg (40 %) of the title compound.
HPLC/MS retention time 17.9 min
LRMS: *m*/*z* 530 (M+H⁺)

### EXAMPLE 31

### 3'-[1-ethyl-5-(isoquinolin-4-ylamino)-6-oxo-1,6-dihydropyridazin-3-yl]-N-(3-hydroxybenzyl)biphenyl-3-carboxamide

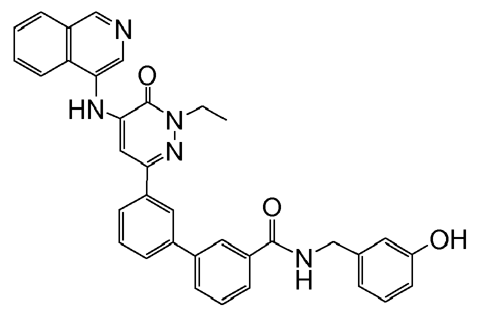

Reaction of the title compound of Example 2 (100mg, 0.22 mmol) with 3-(aminomethyl)phenol (53 mg, 0.43 mmol) according to the method described in Example 15 gave 46 mg (38 %) of the title compound.
HPLC/MS retention time 16.6 min
LRMS: *m*/*z* 568 (M+H⁺)

### EXAMPLE 32

### 3'-[1-ethyl-5-(isoquinolin-4-ylamino)-6-oxo-1,6-dihydropyridazin-3-yl]-N-{7-[(2-hydroxyethyl)(methyl)amino]heptyl}biphenyl-3-carboxamide

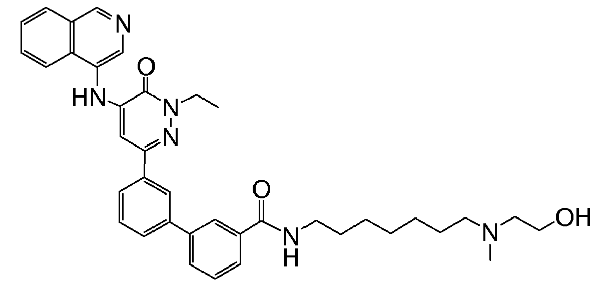

Reaction of the title compound of Example 2 (100 mg, 0.22 mmol) with 2-((7-aminoheptyl)(methyl)amino)ethanol (82 mg, 0.44 mmol, see Preparation 28) according to the method described in Example 15 gave 69 mg (50 %) of the title compound.
HPLC/MS retention time 12.5 min
LRMS: *m*/*z* 633 (M+H⁺)

### EXAMPLE 33

### 3-{3'-[1-ethyl-6-oxo-5-(quinolin-5-ylamino)-1,6-dihydropyridazin-3-yl]biphenyl-4-yl}propanoic acid

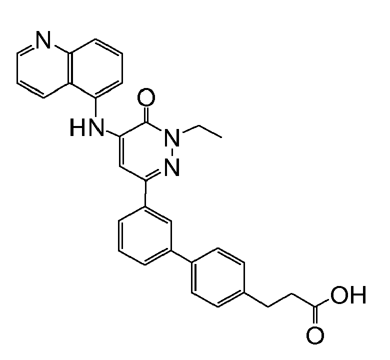

Reaction of 6-(3-bromophenyl)-2-ethyl-4-(quinolin-5-ylamino)pyridazin-3(2H)-one (500 mg, 1.51 mmol, see Preparation 8) with 3- (4- (4,4,5,5-tetramethyl-1,3,2- dioxaborolan -2-yl) phenyl) propanoic acid (414 mg, 1.50 mmol, see Preparation 29) according to the method described in Preparation 11 gave 145 mg (25%) of the title compound.
HPLC/MS retention time 17.3 min
LRMS: *m*/*z* 491 (M+H⁺)

### EXAMPLE 34

### 3-{3'-[1-ethyl-6-oxo-5-(quinolin-5-ylamino)-1,6-dihydropyridazin-3-yl]biphenyl-3-yl}propanoic acid

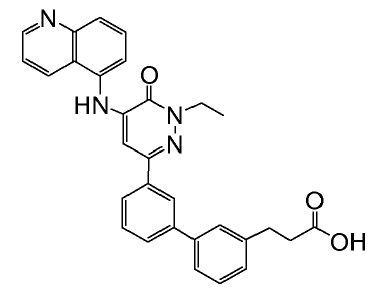

Reaction of 6-(3-bromophenyl)-2-ethyl-4-(quinolin-5-ylamino)pyridazin-3(2H)-one (500 mg, 1.19 mmol, see Preparation 8) with 3-(3-(4,4,5,5-tetramethyl-1,3,2-dioxaborolan-2-yl)phenyl)propanoic acid (425 mg, 1.54 mmol, see Preparation 31) according to the method described in Preparation 11 gave 110 mg (19%) of the title compound.
HPLC/MS retention time 17.4 min
LRMS: *m*/*z* 491 (M+H⁺)

### EXAMPLE 35

### N-cyclopropyl-3'-[1-ethyl-5-(isoquinolin-4-ylamino)-6-oxo-1,6-dihydropyridazin-3-yl]-5-hydroxybiphenyl-3-carboxamide

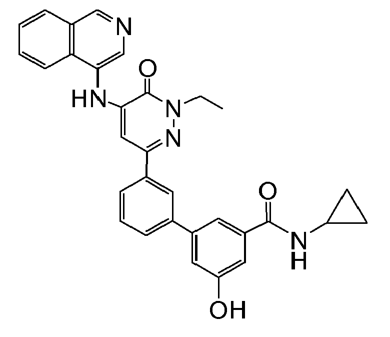

Reaction of the title compound of Preparation 33 (127 mg, 0.27 mmol) with cyclopropanamine (34 µL, 0.49 mmol) according to the method described in Example 21 gave 49 mg (34 %) of the title compound.
HPLC/MS retention time 14.9 min
LRMS: *m*/*z* 516 (M-H⁺)

### EXAMPLE 36

### 3'-[1-ethyl-5-(isoquinolin-4-ylamino)-6-oxo-1,6-dihydropyridazin-3-yl]-N-(1-methylpiperidin-4-yl)biphenyl-3-carboxamide

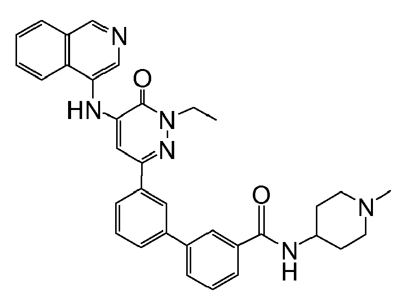

Reaction of the title compound of Example 2 (130 mg, 0.28 mmol) with 1-methylpiperidin-4-amine (64 mg, 0.56 mmol) according to the method described in Example 15 gave 40 mg (25 %) of the title compound.
¹H NMR (200 MHz, CDCl3) δ ppm: 1.5 - 2.3 (m, 6H), 1.55 (t, 3H), 2.34 (s, 3H), 2.86 (m, 2H), 4.02 (m, 1H), 4.43 (q, 3H), 6.15 (d, 1H), 6.82 (s, 1H), 7.4-8.2 (m, 13H), 8.68 (s, 1H), 9.19 (s, 1H).
HPLC/MS (30 min) retention time 11.5 min
LRMS: *m*/*z* 559 (M+H⁺)

### EXAMPLE 37

### N-cyclopropyl-3-{3'-[1-ethyl-6-oxo-5-(quinolin-5-ylamino)-1,6-dihydropyridazin-3-yl]biphenyl-4-yl}propanamide

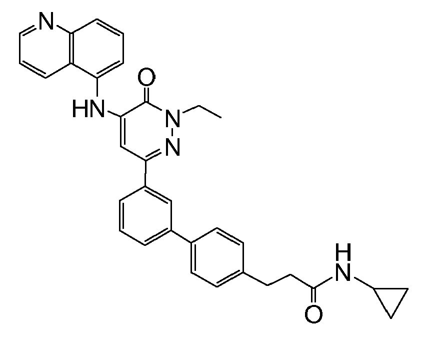

Reaction of the title compound of Example 33 (100 mg, 0.20 mmol) with cyclopropanamine (30 µL, 0.43 mmol) according to the method described in Example 15 gave 31 mg (28 %) of the title compound.
HPLC/MS retention time 16.8 min
LRMS: *m*/*z* 530 (M+H⁺)

### EXAMPLE 38

### N-cyclopropyl-3-{3'-[1-ethyl-6-oxo-5-(quinolin-5-ylamino)-1,6-dihydropyridazin-3-yl]biphenyl-3-yl}propanamide

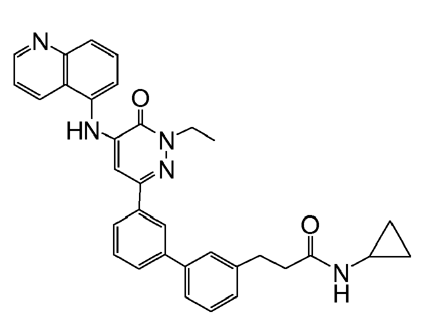

Reaction of the title compound of Example 34 (92 mg, 0.19 mmol) with cyclopropanamine (26 µL, 0.38 mmol) according to the method described in Example 15 gave 47 mg (47 %) of the title compound.
¹H NMR (200 MHz, CDCl3) δ ppm: 0.43 (m, 2H), 0.74 (m, 2H), 1.55 (t, 3H), 2.41 (t, 2H), 2.71 (m, 1H), 3.01 (t, 2H), 4.42 (q, 2H), 5.88 (bs, 1H), 6.67 (s, 1H), 7.2 - 7.9 (m, 13H), 8.07 (d, 1), 8.37 (d, 1H), 8.99 (m, 1H).
HPLC/MS retention time 16.9 min
LRMS: *m*/*z* 530 (M+H⁺)

### EXAMPLE 39

### N-cyclopentyl-3'-[1-ethyl-6-oxo-5-(quinolin-5-ylamino)-1,6-dihydropyridazin-3-yl]biphenyl-4-carboxamide

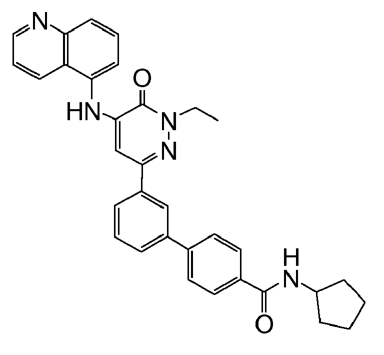

Reaction of the title compound of Example 9 (150 mg, 0.32 mmol) with cyclopentanamine (65 µL, 0.66 mmol) according to the method described in Example 15 gave 135 mg (79 %) of the title compound.
HPLC/MS retention time 17.9 min
LRMS: *m*/*z* 530 (M+H⁺)

### EXAMPLE 40

### 2-{3'-[1-ethyl-5-(isoquinolin-4-ylamino)-6-oxo-1,6-dihydropyridazin-3-yl]biphenyl-3-yl}-N-(2-pyrrolidin-1-ylethyl)acetamide

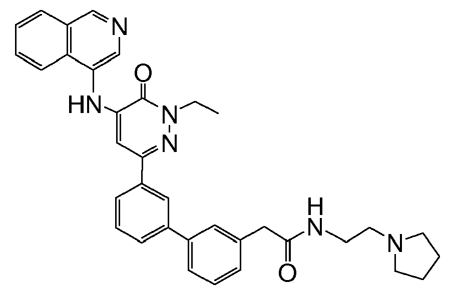

A reactor for high pressure was charged with the title compound of Preparation 36 (300 mg, 0.67g), 4-bromoisoquinoline (175 mg, 0.84 mmol), potassium carbonate (112 mg, 0.81 mmol) and copper(I)iodide (13 mg, 0.07 mmol), dimethylethylendiamine (14 µL, 0.13 mmol) and dioxane (3 mL). This reaction mixture was heated overnight under nitrogen at 130°C. The reaction mixture was diluted with water and ethyl acetate, phases were separated and the organic phase was washed with water and brine, dried over magnesium sulphate, filtered and evaporated under reduced pressure. The residue was purified by the SP1® automated purification system (reverse phase, 40g Merck column). 140 mg (36%) of the final compound were obtained.
HPLC/MS retention time 11.8 min
LRMS: *m*/*z* 572 (M+H⁺)

### EXAMPLE 41

### 2-{3'-[1-ethyl-6-oxo-5-(quinolin-5-ylamino)-1,6-dihydropyridazin-3-yl]biphenyl-3-yl}-N-(2-pyrrolidin-1-ylethyl)acetamide

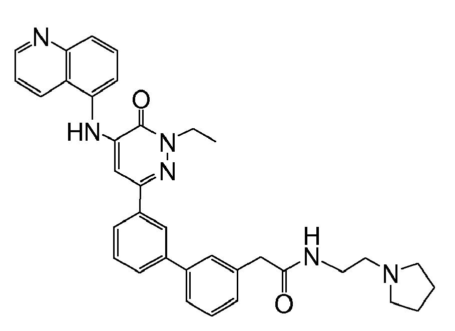

Reaction of the title compound of Preparation 36 (300 mg, 0.67 mmol) with 5-bromoquinoline (175 mg, 8.84 mmol) according to the method described in Example 40 gave 200 mg (52 %) of the title compound.
HPLC/MS retention time 11.5 min
LRMS: *m*/*z* 572 (M+H⁺)

### EXAMPLE 42

### 3'-[1-ethyl-6-oxo-5-(quinolin-5-ylamino)-1,6-dihydropyridazin-3-yl]-N-(2-morpholin-4-ylethyl)biphenyl-4-carboxamide

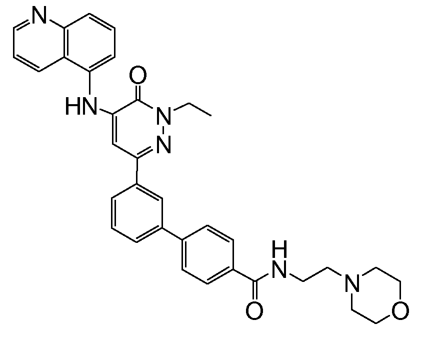

Reaction of the title compound of Example 9 (150mg, 0.32 mmol) with 2-morphplonoethanamine (84 mg, 0.65 mmol) according to the method described in Example 15 gave 159 mg (85 %) of the title compound.
¹H NMR (200 MHz, DMSO-d₆) δ ppm 1.43 (t, 3 H) 2.39 - 2.46 (m, 4 H) 3.34 - 3.48 (m, 3 H) 3.54 - 3.62 (m, 5 H) 4.30 (q, J=7.29 Hz, 2 H) 6.60 (s, 1 H) 7.42 - 7.64 (m, 3 H) 7.71 (d, J=8.20 Hz, 4 H) 7.78 - 8.01 (m, 5 H) 8.28 (s, 1 H) 8.35 (d, J=8.59 Hz, 1 H) 8.47 (t, J=5.47 Hz, 1 H) 8.96 (dd, J=4.29, 1.56 Hz, 1 H)
HPLC/MS retention time 11.0 min
LRMS: *m*/*z* 575 (M+H⁺)

### EXAMPLE 43

### 3'-[1-ethyl-6-oxo-5-(quinolin-5-ylamino)-1,6-dihydropyridazin-3-yl]-N-(2-piperidin-1-ylethyl)biphenyl-4-carboxamide

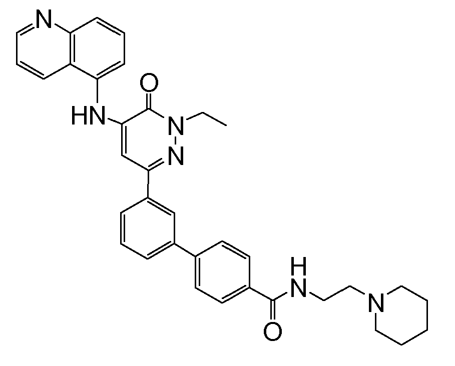

Reaction of the title compound of Example 9 (150 mg, 0.32 mmol) with 2-morpholinoethanamine (93 mg, 0.65 mmol) according to the method described in Example 15 gave 144 mg (77 %) of the title compound.
¹H NMR (200 MHz, DMSO-d₆) δ ppm 1.31 - 1.59 (m, 9 H) 2.39 - 2.46 (m, 4 H) 3.32 - 3.43 (m, 2 H) 4.30 (q, J=7.42 Hz, 2 H) 6.60 (s, 1 H) 7.43 - 7.64 (m, 3 H) 7.71 (d, J=8.59 Hz, 4 H) 7.80 - 8.03 (m, 6 H) 8.19 (s, 1 H) 8.35 (d, J=8.59 Hz, 1 H) 8.42-8.54 (m, 1 H) 8.96 (dd, J=4.29, 1.56 Hz, 1 H) 9.16(s, 1 H)
HPLC/MS retention time 11.5 min
LRMS: *m*/*z* 575 (M+H⁺)

### EXAMPLE 44

### N-(3,4-dimethoxybenzyl)-3'-[1-ethyl-5-(isoquinolin-4-ylamino)-6-oxo-1,6-dihydropyridazin-3-yl]biphenyl-3-carboxamide

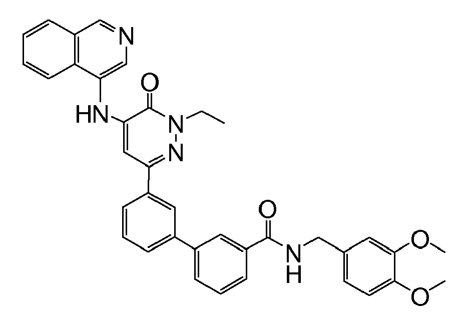

Reaction of the title compound of Example 2 (180 mg, 0.39 mmol) with (3,4-dimethoxyphenyl)methanamine (130 mg, 0.78 mmol) according to the method described in Example 15 gave 145 mg (61 %) of the title compound.
HPLC/MS retention time 17.2 min
LRMS: *m*/*z* 612 (M+H⁺)

### EXAMPLE 45

### N-cyclobutyl-3'-[1-ethyl-6-oxo-5-(quinolin-5-ylamino)-1,6-dihydropyridazin-3-yl]biphenyl-4-carboxamide

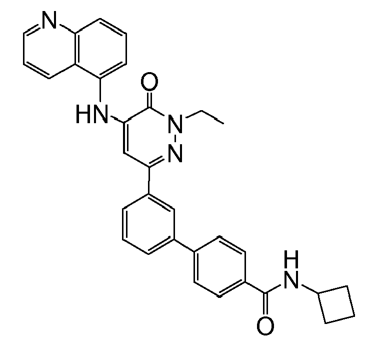

Reaction of the title compound of Example 9 (150 mg, 0.32 mmol) with cyclobutanamine (60 µL, 0.70 mmol) according to the method described in Example 15 gave 82 mg (47 %) of the title compound.
HPLC/MS retention time 17.3 min
LRMS: *m*/*z* 516 (M+H⁺)

### EXAMPLE 46

### N-[4-(aminosulfonyl)benzyl]-3'-[1-ethyl-6-oxo-5-(quinolin-5-ylamino)-1,6-dihydropyridazin-3-yl]biphenyl-3-carboxamide

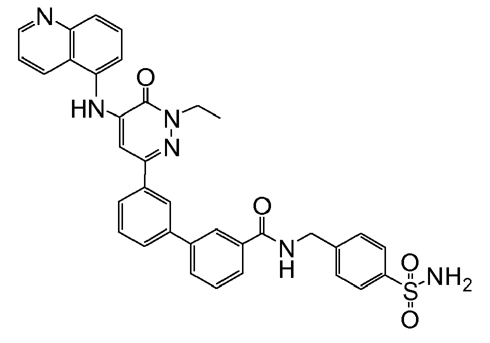

Reaction of the title compound of Example 1 (100mg, 0.22 mmol) with 4-(aminomethyl)benzenesulfonamide (81 mg, 0.70 mmol) according to the method described in Example 15 gave 30 mg (22 %) of the title compound.
HPLC/MS retention time 15.8 min
LRMS: *m*/*z* 631 (M+H⁺)

### EXAMPLE 47

### 3'-[1-ethyl-6-oxo-5-(quinolin-5-ylamino)-1,6-dihydropyridazin-3-yl]-N-[3-(2-oxopyrrolidin-1-yl)propyl]biphenyl-3-carboxamide

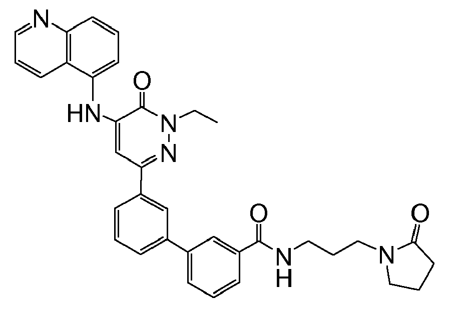

Reaction of the title compound of Example 1 (100 mg, 0.22 mmol) with 1-(3-aminopropyl)pyrrolidin-2-one (61 mg, 0.43 mmol) according to the method described in Example 15 gave 79 mg (62 %) of the title compound.
HPLC/MS retention time 15.9 min
LRMS: *m*/*z* 587 (M+H⁺)

### EXAMPLE 48

### N-(2-Amino-2-oxoethyl)-3'-(1-ethyl-6-oxo-5-(quinolin-5-ylamino)-1,6-dihydropyridazin-3-yl)biphenyl-3-carboxamide

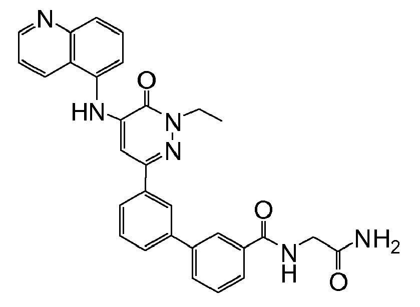

Reaction of the title compound of Example 1 (100 mg, 0.22 mmol) with 2-aminoacetamide chlorhydrate (48 mg, 0.43 mmol) and triethylamine (90 µL, 0.65 mmol) according to the method described in Example 15 gave 25 mg (22 %) of the title compound.
HPLC/MS retention time 14.1 min
LRMS: *m*/*z* 519 (M+H⁺)

### EXAMPLE 49

### N-({3'-[1-ethyl-6-oxo-5-(quinolin-5-ylamino)-1,6-dihydropyridazin-3-yl]biphenyl-3-yl}carbonyl)glycine

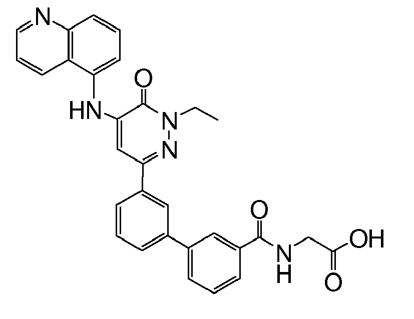

Reaction of the title compound of Preparation 37 (103 mg, 0.15 mmol) with lithium hydroxide monohydrate (32 mg, 0.76 mmol) according to the method described in Example 1 gave 49 mg (61 %) of the title compound.
HPLC/MS retention time 15.2 min
LRMS: *m*/*z* 520 (M+H⁺)

### EXAMPLE 50

### 2-{3'-[1-ethyl-5-(isoquinolin-4-ylamino)-6-oxo-1,6-dihydropyridazin-3-yl]biphenyl-4-yl}-N-(2-pyrrolidin-1-ylethyl)acetamide

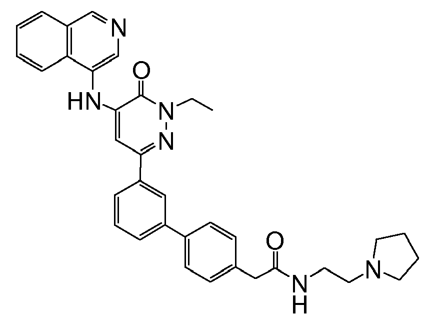

Reaction of the title compound of Preparation 40 (200mg, 0.45 mmol) with 4-bromoisoquinoline (112 mg, 0.54 mmol) according to the method described in Example 40 gave 73 mg (28 %) of the title compound.
HPLC/MS retention time 11.5 min
LRMS: *m*/*z* 572 (M+H⁺)

### EXAMPLE 51

### 2-{3'-[1-ethyl-6-oxo-5-(quinolin-5-ylamino)-1,6-dihydropyridazin-3-yl]biphenyl-4-yl}-N-(2-pyrrolidin-1-ylethyl)acetamide

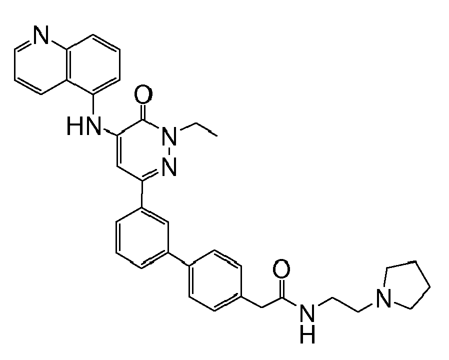

Reaction of the title compound of Preparation 40 (200mg, 0.45 mmol) with 5-bromoquinoline (112 mg, 0.54 mmol) according to the method described in Example 40 gave 96 mg (37 %) of the title compound.
HPLC/MS retention time 11.3 min
LRMS: *m*/*z* 572 (M+H⁺)

### EXAMPLE 52

### N-{2-[1-(dimethylamino)cyclopentyl]ethyl}-3'-[1-ethyl-6-oxo-5-(quinolin-5-ylamino)-1,6-dihydropyridazin-3-yl]biphenyl-3-carboxamide

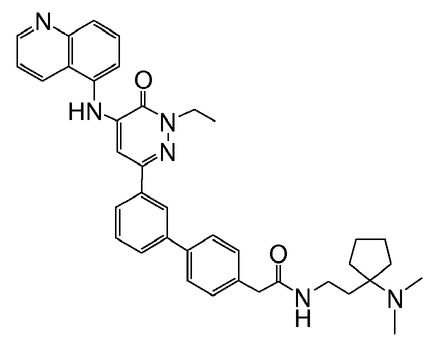

Reaction of the title compound of Example 1 (100 mg, 0.22 mmol) with 1-(2-aminoethyl)-N,N-dimethylcyclopentanamine (68 mg, 0.44 mmol, see Preparation 44) according to the method described in Example 15 gave 42 mg (32 %) of the title compound.
HPLC/MS retention time 12.0 min
LRMS: *m*/*z* 601 (M+H⁺)

### EXAMPLE 53

### 4-[({3'-[1-ethyl-6-oxo-5-(quinolin-5-ylamino)-1,6-dihydropyridazin-3-yl]biphenyl-3-yl}carbonyl)amino]piperidine-1-carboxamide

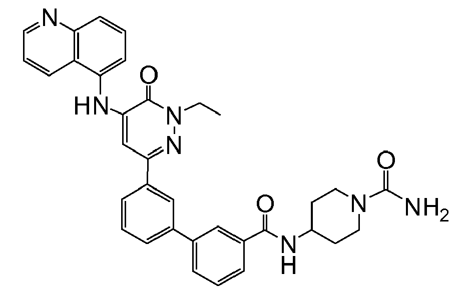

The title compound of Preparation 46 (88 mg, 0.15 mmol) was dissolved in THF (2 mL). diisopropylethylamine (34 µL, 0.2 mmol) and trimethylsilylisocyanate (24µL, 0.20 mmol) were added and the mixture was stirred for 2 h at room temperature. The solvent was evaporated under reduced pressure and water and dichloromethane were added to the residue. The organic phase was separated and the aqueous one further extracted. The organic phase was washed with water and brine, dried over sodium sulfate, filtered and evaporated under reduced pressure. The resulting solid (151 mg) was purified by the SP1® automated purification system to give 37 mg (42%) of the desired compound.
HPLC/MS retention time 14.7 min
LRMS: *m*/*z* 588 (M+H⁺)

### EXAMPLE 54

### 2-{3'-[1-ethyl-6-oxo-5-(quinolin-5-ylamino)-1,6-dihydropyridazin-3-yl]biphenyl-4-yl}-N-(2-piperidin-1-ylethyl)acetamide

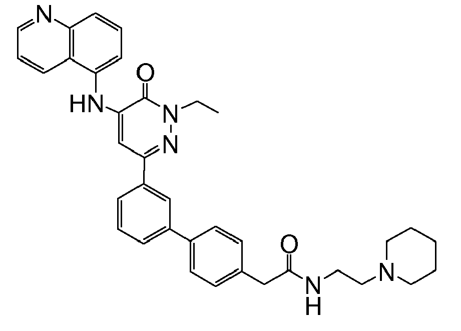

Reaction of the title compound of Example 6 (177mg, 0.37 mmol) with 2-(piperidin-1-yl)ethanamine (95 mg, 0.74 mmol) according to the method described in Example 15 gave 37 mg (17 %) of the title compound.
¹H NMR (200 MHz, DMSO-d₆) δ ppm 1.2-1.9 (m, 6H), 1.42 (t, 3 H), 2.85 (m, 2H), 3.10 (m, 2H), 3.3 - 3.5 (m, 4H), 3.49 (s, 2H), 4.29 (q, 2H), 6.56 (s, 1H), 7.3 - 8.1 (m, 13H), 8.34 (d, 1H), 8.48 (bs, 1H), 8.96 (m, 1H), 9.15 (s, 1H), 9.76 (bs, 1H).
HPLC/MS retention time 11.7 min
LRMS: *m*/*z* 588 (M+H⁺)

### EXAMPLE 55

### 3'-[1-ethyl-6-oxo-5-(quinolin-5-ylamino)-1,6-dihydropyridazin-3-yl]-N-{2-[(2R)-1-methylpyrrolidin-2-yl]ethyl}biphenyl-3-carboxamide

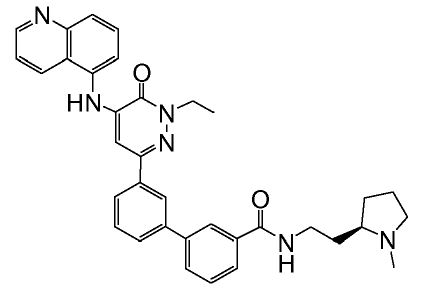

Reaction of the title compound of Example 1 (160 mg, 0.35 mmol) with (R)-2-(1-methylpyrrolidin-2-yl)ethanamine (103 mg, 0.69 mmol, prepared as described at EP1408030 A1) according to the method described in Example 15 gave 70 mg (35 %) of the title compound.
HPLC/MS retention time 11.4 min
LRMS: *m*/*z* 573 (M+H⁺)

### EXAMPLE 56

### 6-(3'-{[4-(dimethylamino)piperidin-1-yl]carbonyl}biphenyl-3-yl)-2-ethyl-4-(quinolin-5-ylamino)pyridazin-3(2H)-one

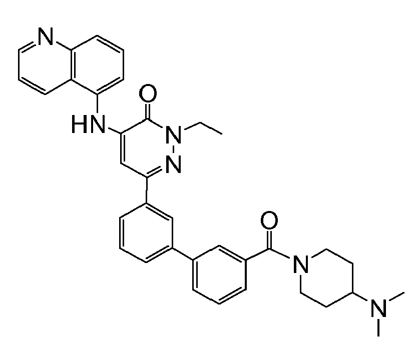

Reaction of the title compound of Example 1 (100mg, 0.22 mmol) with N,N-dimethylpiperidin-4-amine (55 mg, 0.43 mmol) according to the method described in Example 15 gave 63 mg (49 %) of the title compound.
HPLC/MS retention time 10.9 min
LRMS: *m*/*z* 573 (M+H⁺)

### EXAMPLE 57

### 3'-[1-ethyl-6-oxo-5-(quinolin-5-ylamino)-1,6-dihydropyridazin-3-yl]-N-{2-[(2S)-1-methylpyrrolidin-2-yl]ethyl}biphenyl-3-carboxamide

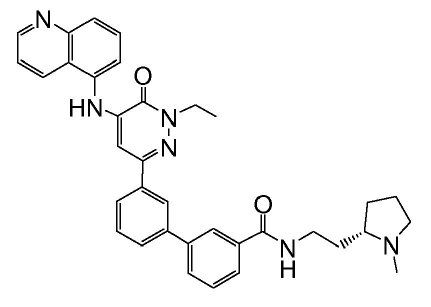

Reaction of the title compound of Example 1 (87.7 mg, 0.19 mmol) with (S)-2-(1-methylpyrrolidin-2-yl)ethanamine (49 mg, 0.38 mmol, prepared as described at EP1408030 A1) according to the method described in Example 15 gave 50 mg (46 %) of the title compound.
HPLC/MS (30 min) retention time 11.7 min
LRMS: *m*/*z* 573 (M+H⁺)

### EXAMPLE 58

### 3-{3'-[1-ethyl-6-oxo-5-(quinolin-5-ylamino)-1,6-dihydropyridazin-3-yl]biphenyl-4-yl}-N-(2-piperidin-1-ylethyl)propanamide

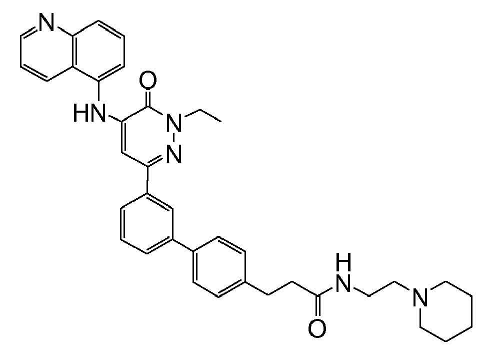

Reaction of the title compound of Example 33 (160mg, 0.33 mmol) with 2-(piperidin-1-yl)ethanamine (95 µL, 0.67 mmol) according to the method described in Example 15 gave 53 mg (27 %) of the title compound.
HPLC/MS retention time 12.5 min
LRMS: *m*/*z* 601 (M+H⁺)

### EXAMPLE 59

### 3-{3'-[1-ethyl-6-oxo-5-(quinolin-5-ylamino)-1,6-dihydropyridazin-3-yl]biphenyl-4-yl}-N-(1-methylpiperidin-4-yl)propanamide

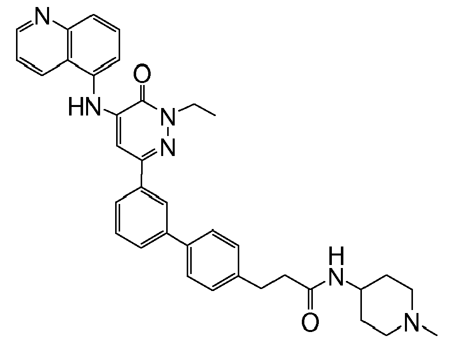

Reaction of the title compound of Example 33 (160 mg, 0.33 mmol) with 1-methylpiperidin-4-amine (75 mg, 0.66 mmol) according to the method described in Example 15 gave 70 mg (37 %) of the title compound.
HPLC/MS retention time 12.1 min
LRMS: *m*/*z* 587 (M+H⁺)

### EXAMPLE 60

### 3-{3'-[1-ethyl-6-oxo-5-(quinolin-5-ylamino)-1,6-dihydropyridazin-3-yl]biphenyl-4-yl}-N-(2-morpholin-4-ylethyl)propanamide

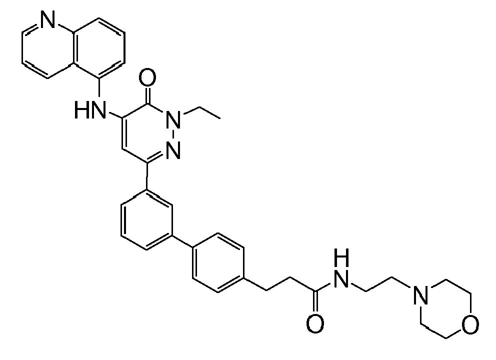

Reaction of the title compound of Example 33 (160 mg, 0.33 mmol) with 2-morpholinoethanamine (85 mg, 0.65 mmol) according to the method described in Example 15 gave 109 mg (55 %) of the title compound.
HPLC/MS retention time 12.2 min
LRMS: *m*/*z* 603 (M+H⁺)

### EXAMPLE 61

### N,N'-dicyclopropyl-3'-[1-ethyl-6-oxo-5-(quinolin-5-ylamino)-1,6-dihydropyridazin-3-yl]biphenyl-3,5-dicarboxamide

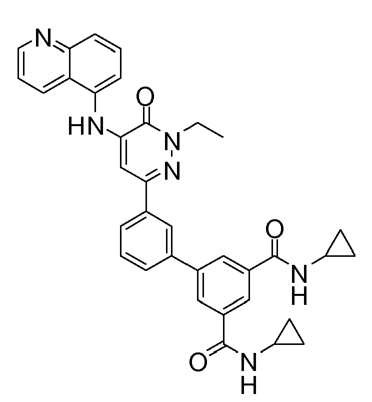

Reaction of the title compound of Preparation 8 (160 mg, 0.38 mmol) with 3,5-bis(cyclopropylcarbamoyl)phenylboronic acid (130 mg, 0.45 mmol, see Preparation 51) according to the method described in Example 3 gave 53 mg (24%) of the title compound.
HPLC/MS retention time 15.2 min
LRMS: *m*/*z* 584 (M+)

### EXAMPLE 62

### N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide

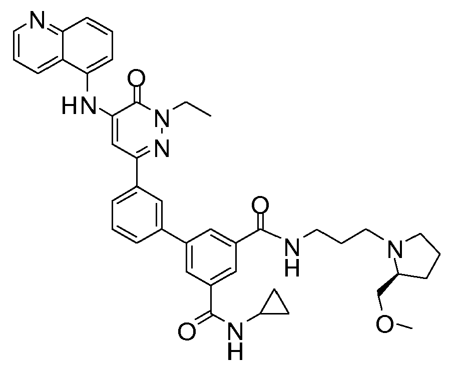

Reaction of the title compound of Preparation 54 (290 mg, 0.53 mmol) with (S)-3-(2-(methoxymethyl)pyrrolidin-1-yl)propan-1-amine (183 mg, 1.01 mmol, prepared as described at EP1408030 A1) according to the method described in Example 15 gave 155 mg (41 %) of the title compound.
¹H NMR (400 MHz, dmso-d6) δ ppm: 0.60 (m, 2H), 0.73 (m, 2H), 1.43 (t, 3H), 1.48 (m, 1H), 1.6 - 1.9 (m, 5H), 1.16 (m, 1H), 2.41 (m, 1H), 2.64 (m, 1H), 2.8 - 3.0 (m, 2H), 3.1 - 3.5 (m, 5H), 3.17 (s, 3H), 4.31 (q, 2H), 6.62 (s, 1H), 7.52 (t, 1H), 7.58 (dd, 1H), 7.63 (d, 1H), 7.73 (d, 1H), 7.75 (d, 1H), 7.83 (t, 1H), 7.94 (s, 1H), 7.96 (t, 1H), 8.1 - 8.4 (m, 4H), 8.63 (m, 1H), 8.72 (m, 1H), 8.94 (m, 1H), 9.14(s, 1H).
HPLC/MS retention time 11.0 min
LRMS: *m*/*z* 700 (M⁺+1)

### EXAMPLE 63

### N-cyclopropyl-3'-[1-ethyl-6-oxo-5-(quinolin-5-ylamino)-1,6-dihydropyridazin-3-yl]-N'-{2-[(2S)-2-(methoxymethyl)pyrrolidin-1-yl]ethyl}biphenyl-3,5-dicarboxamide

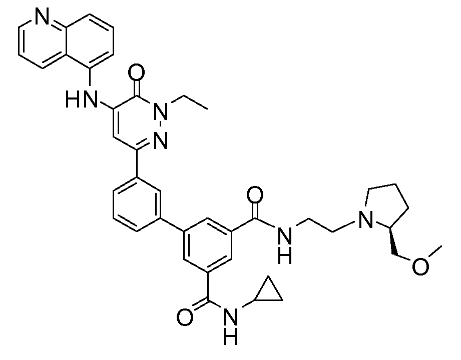

Reaction of the title compound of Preparation 54 (80 mg, 0.15 mmol) with (S)-2-(2-(methoxymethyl)pyrrolidin-1-yl)ethanamine (68 mg, 0.29 mmol, prepared as described at EP1408030 A1) according to the method described in Example 15 gave 51 mg (51%) of the title compound.
HPLC/MS retention time 10.9 min
LRMS: *m*/*z* 686 (M⁺+1)

### PHARMACOLOGICAL ACTIVITY

### PDE4 Assay Procedure

Measurement of Phosphodiesterase Activity was done using a [3H] cAMP Scintillation Proximity Assay (SPA) (GE Healthcare). Compounds to be tested were disolved in dimethylsulfoxide (DMSO) at a stock concentration of 1 mM and serial dilutions were prepared in 50% DMSO to determine IC50s.

The reactions were conducted in 96-well plates (Corning, Ref.3604) at room temperature, in 0.1 mL of reaction buffer containing (final concentrations): 50 mM Tris-HCl, pH 7.5, 8.3 mM MgCl₂, 1.7 mM EGTA, 30 nM [3H] cAMP (approximately 150000 dpm/well) with or without the inhibitors. The reaction was initiated by adding yeast extract of recombinant PDE4 enzyme.

Plates were shaken for 1 h at room temperature and the incubation was terminated by adding 50 µL (0.5 mg /well) of SPA ytrium silicate beads (RPNQ 0150; GE Healthcare) in the presence of zinc sulfate. Plates were stored overnight in the dark and read on a TRILUX microtiter plate reader (Perkin Elmer).

The results are shown in Table 1.

| **Example** | **IC₅₀ PDE4 (nM)** |
|---|---|
| **4** | 0.037 |
| **5** | 0.445 |
| **7** | 0.016 |
| **12** | 0.125 |
| **13** | 0.037 |
| **15** | 0.019 |
| **16** | 0.045 |
| **17** | 0.043 |
| **19** | 0.223 |
| **22** | 0.128 |
| **25** | 0.209 |
| **27** | 0.016 |
| **29** | 0.025 |
| **36** | 0.172 |
| **38** | 0.101 |
| **48** | 0.041 |
| **49** | 0.042 |
| **54** | 0.090 |
| **62** | 0.023 |
| **63** | 0.037 |

It can be seen from Table 1 that the compounds of formula (I) are potent inhibitors of phosphodiesterase 4 (PDE 4). Preferred pyridazin-3(2H)-one derivatives of the invention possess an IC₅₀ value for the inhibition of PDE4 (determined as defined above) of less than 1 nM, preferably less than 0.5 nM and most preferably less than 0.2 nM. The compounds are also capable of blocking the production of some pro-inflammatory cytokines such as, for example, TNFα.

Thus, they can be used in the treatment of allergic, inflammatory and immunological diseases, as well as those diseases or conditions where the blockade of pro-inflammatory cytokines or the selective inhibition of PDE 4 could be of benefit. These disease states include asthma, chronic obstructive pulmonary disease (COPD), allergic rhinitis, rheumatoid arthritis, osteoarthritis, osteoporosis, bone-formation disorders, glomerulonephritis, multiple sclerosis, ankylosing spondylitis, Graves ophtalmopathy, myasthenia gravis, diabetes insipidus, graft rejection, gastrointestinal disorders such as irritable bowel disease, ulcerative colitis or Crohn disease, septic shock, adult distress respiratory syndrome, and skin diseases such as atopic dermatitis, contact dermatitis, acute dermatomyositis and psoriasis. They can also be used as improvers of cerebrovascular function as well as in the treatment of other CNS related diseases such as dementia, Alzheimer's disease, depression, and as nootropic agents.

Like other PDE4 inhibitors (see references above) the compounds of the invention can also be used for blocking, after preventive and/or curative treatment, the erosive and ulcerogenic effects induced by a variety of etiological agents, such as antiinflammatory drugs (steroidal or non-steroidal antiinflammatory agents), stress, ammonia, ethanol and concentrated acids.

They can be used alone or in combination with antacids and/or antisecretory drugs in the preventive and/or curative treatment of gastrointestinal pathologies like drug-induced ulcers, peptic ulcers, H. Pylori-related ulcers, esophagitis and gastro-esophageal reflux disease.

They can also be used in the treatment of pathological situations where damage to the cells or tissues is produced through conditions like anoxia or the production of an excess of free radicals. Examples of such beneficial effects are the protection of cardiac tissue after coronary artery occlusion or the prolongation of cell and tissue viability when the compounds of the invention are added to preserving solutions intended for storage of transplant organs or fluids such as blood or sperm. They are also of benefit on tissue repair and wound healing.

### Combinations

The compounds of formula (I) of the present invention can also be used in combination with other drugs known to be effective in the treatment of the diseases or the disorders indicated above. For example the compounds of the present invention can be combined with (a) β2-adrenergic agonists, (b) anti-cholinergics, (c) anti-allergic agents, (d) anti-inflammatory agents, (e) immunosuppressants, and (f) anti-infectives; for simultaneous, separate or sequential use in the treatment of the human or animal body.

Accordingly, another embodiment of the invention is the use of the compounds of formula (I) of the present invention for the manufacture of a medicament for treatment or prevention of pathological conditions, diseases and disorders known to be susceptible of amelioration by inhibition of PDE4, as well as a method for treating a subject afflicted with a pathological condition or disease susceptible to amelioration by inhibition of PDE4, which comprises administering to said subject an effective amount of a compound of formula (I).

The compounds or pharmaceutically acceptable salts, solvates, N-oxides or deuterated derivatives thereof according to the invention may also be used in combination with another therapeutically active agent, for example a) a β2-adrenoreceptor agonist, b) an anti-cholinergic agent, c) an anti-allergic, d) an anti-inflammatory agent, e) an immunosuppressant, or f) an anti-infective agent.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day, most preferably once a day.

Examples of suitable β2-agonists that can be combined with the PDE4 inhibitors of the invention are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in international patent applications Nos. WO2007/124898, W02006/122788A1, WO2008/046598 and WO2008095720.

Examples of suitable corticosteroids and glucocorticoids that can be combined with the PDE4 inhibitors of the invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, Desisobutyrylciclesonide, hydrocortisone acetate, hydrocortisone sodium succinate, prednisolone sodium phosphate and hydrocortisone probutate, Prednisolone sodium metasulfobenzoate and clobetasol propionate.

Examples of suitable M3 antagonists (anticholinergics) that can be combined with the PDE4 inhibitors of the invention are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-allergic agents that can be combined with the PDE4 inhibitors of the invention are anti-histamines (e.g. Methapyrilene, Mequitazine, Azelastine hydrochloride, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1004723D, GSK-835726, SUN-1334H), CRTH2 antagonists (e.g. Ramatroban, AMG-009, OC-000459), or mast cell stabilizers (e.g. nedocromil, pemirolast potassium, chromoglycate sodium, suplatast tosilate)

Examples for suitable immunosupressants that can be combined with the PDE4 inhibitors of the invention are picremolimus, tacromilus, cyclosporine A, leflunomide, methotrexate, anti-TNF agents and compounds described in International patent applications Nos. WO2009/021696 and WO2008/077639.

Examples for suitable anti-infectives that can be combined with the PDE4 inhibitors of the invention are mupiricin, retapamulin, clotrimazole, ketoconazole and terbinafine.

Other possible combinations include, for example, a combination comprising a PDE4 inhibitor of the invention with another anti-inflammatory agent such as a non-steroidal anti-inflammatory drug (NSAID) such as a leukotriene antagonist (e.g. Ibudilast, Pranlukast hydrate, Zafirlukast, Montelukast, Tipelukast), a FLAP inhibitor, a lipoxygenase inhibitor, a cyclooxygenase inhibitor (e.g. diclofenac, ibuprofen, celecoxib); an elastase inhibitor, a Syk kinase inhibitor, a PI3Kδγ inhibitor or another PDE inhibitor (e.g. theophylline).

Examples of suitable Syk kinase inhibitors that can be combined with the PDE4 inhibitors of the invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Examples of suitable antagonists of the PI3Kδγ inhibitors that can be combined with the PDE4 inhibitors of the invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

Particularly preferred pharmaceutical compositions according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, compounds described in International patent applications Nos. WO2009/021696 and WO2008/077639, methapyrilene, cetirizine, loratadine, ebastine, desloratadine, fexofenadine, azelastine, levocabastine, olopatadine, Montelukast, picremolimus, tacromilus, mupiricin, retapamulin, clotrimazole, ketoconazole, terbinafine.

Thus, in one aspect of the invention, the composition comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the composition comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. The composition may further comprise a corticosteroid selected from the group consisting of mometasone furoate, ciclesonide, budesonide, fluticasone furoate and fluticasone propionate.

In a still other aspect of the invention, the composition comprises a compound of formula (I) and a suitable β2-agonist. Particularly preferred β2-agonists are those selected from the group consisting of formoterol, salmeterol, indacaterol, carmoterol and compound described in International patent applications Nos. WO2007/124898, W02006/122788A1, WO2008/046598 and WO2008095720. The composition may further comprise a corticosteroid selected from the group consisting of mometasone furoate, ciclesonide, budesonide, fluticasone furoate, and fluticasone propionate. In addition to the salt of the invention and to the β2-agonist, the composition may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

The compounds of formula (I) and the combinations of the invention may be used in the treatment of respiratory, skin and inflammatory diseases, wherein the use of a PDE4 inhibitor is expected to have a beneficial effect, for example asthma, chronic obstructive pulmonary disease (COPD), allergic rhinitis, rheumatoid arthritis, multiple sclerosis, atopic dermatitis, psoriasis and inflammatory bowel disease.

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

### Pharmaceutical Compositions

Pharmaceutical compositions according to the present invention comprise the compounds of the invention in association with a pharmaceutically acceptable diluent or carrier.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by inhibition of phosphodiesterase IV.

The invention is also directed to pharmaceutical compositions of the invention for use in the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention also provides a method of treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001 % to 99% by weight, preferably 0.01 % to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

The pharmaceutically acceptable excipients which are admixed with the active compound or salts of such compound, to form the compositions of this invention are well-known per se and the actual excipients used depend inter alia on the intended method of administering the compositions.

Additional suitable carriers for formulations of the compounds of the present invention can be found in Remington: The Science and Practice of Pharmacy, 20th Edition, Lippincott Williams & Wilkins, Philadelphia, Pa., 2000. The following non-limiting examples illustrate representative pharmaceutical compositions of the invention.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 0.01-3000 mg, more preferably 0.5-1000 mg of active ingredient or the equivalent amount of a pharmaceutically acceptable salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuiar® (formerly known as Novolizer SD2FL), which is described the following patent applications Nos: WO97/000703, WO03/000325, WO03/061742 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetrafluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate. Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies one of the preferred application form, especially in the therapy of obstructive lung diseases or for the treatment of asthma. Other preferred application form is dermal and transdermal administration, especially in the therapy of atopic dermatitis or psoriasis.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

A gelatin cartridge for inhalation containing 0.2 mg of N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide and 25 mg of lactose was prepared using conventional methods.

### COMPOSITION EXAMPLE 2

A formulation for inhalation with a dry powder inhaler (DPI) containing 15 mg of N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide (micronized) and 3000 mg of lactose was prepared using conventional methods.

### COMPOSITION EXAMPLE 3

An oil-in-water emulsion cream is prepared containing N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide (active ingredient) and the ingredients listed above, using conventional methods.

| Formulation: | |
|---|---|
| Active ingredient | 1 % |
| Cetyl alcohol | 3 % |
| Stearyl alcohol | 4 % |
| Gliceryl monostearate | 4 % |
| Sorbitan monostearate | 0.8 % |
| Sorbitan monostearate POE | 0.8 % |
| Liquid vaseline | 5 % |
| Methylparaben | 0.18 % |
| Propylparaben | 0.02 % |
| Glycerine | 15 % |
| Purified water csp. | 100 % |

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, or solvate, or N-oxide, or stereoisomer or deuterated derivate thereof: 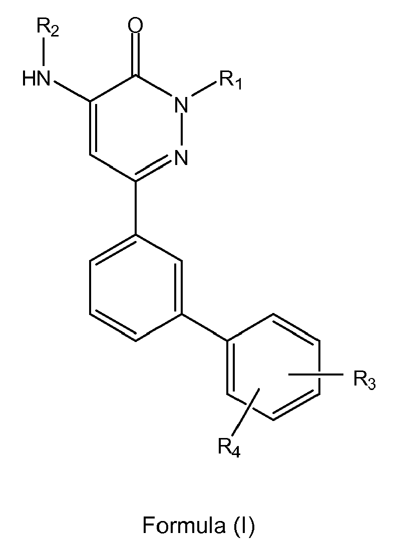 wherein
R₁ represents a linear or branched C₁-C₄ alkyl group;
R₂ represents a quinolyl or isoquinolyl group; wherein the quinolyl or isoquinolyl group is unsubstituted or substituted by one or more substituents selected from a halogen atom, a hydroxyl group, a cyano group, a linear or branched C₁-C₄ alkyl group, a C₁-C₄ haloalkyl group, a C₁-C₄ hydroxyalkyl group or a C₁-C₄ alkoxy group;
R₃ represents a -(CH₂)ₙC(O)OH group, a -(CH₂)ₙC(O)-R₅ group, a -(CH₂)ₙC(O)NH₂ group, a -(CH₂)ₙC(O)NH-(CH₂)ₘ-R₅ group, a -(CH₂)ₙC(O)NH-(CH₂)ₘ-C(O)R₆ group or
a -(CH₂)ₙC(O)NH-(CH₂)ₘ-NR₅R₇ group;
R₄ represents a hydrogen atom, a hydroxyl group, a linear or branched C₁-C₄ alkyl group or a -(CH₂)_{n'}C(O)NH-(CH₂)_{m'}-R₈ group;
R₅ and R₈ independently represent a linear or branched C₁-C₄ alkyl group, a C₁-C₄ hydroxyalkyl group, a (C₁-C₄ alkoxy)(C₁-C₂ alkylene) group, a C₃-C₁₀ cycloalkyl group, a monocyclic or polycyclic C₅-C₁₄ aryl group or a 5- to 14- membered heterocyclyl group containing at least one heteroatom selected from O, S and N,
wherein the cycloalkyl and heterocyclyl groups are unsubstituted or substituted by one or more substituents selected from a halogen atom, a hydroxyl group, a cyano group, an oxo group, a linear or branched C₁-C₄ alkyl group, aC₁-C₄ haloalkyl group, a C₁-C₄ hydroxyalkyl group, a C₁-C₄ alkoxy group, a (C₁-C₄ alkoxy)(C₁-C₂ alkylene) group, a carbamoyl group, a sulfamoyl group, an amino group, a mono-(C₁-C₄ alkyl)amino group or a di-(C₁-C₄ alkyl)amino group; and the aryl groups are unsubstituted or substituted by one or more substituents selected from a halogen atom, a hydroxyl group, a cyano group, a linear or branched C₁-C₄ alkyl group, aC₁-C₄ haloalkyl group, a C₁-C₄ hydroxyalkyl group, a C₁-C₄ alkoxy group, a (C₁-C₄ alkoxy)(C₁-C₂ alkylene) group, a carbamoyl group, a sulfamoyl group, an amino group, a mono-(C₁-C₄ alkyl)amino group or a di-(C₁-C₄ alkyl)amino group;
R₆ represents a hydroxyl group, an amino group, a linear or branched C₁-C₄ alkyl group or a C₁-C₄ hydroxyalkyl group;
R₇ represents a linear or branched C₁-C₄ alkyl group or a C₁-C₄ hydroxyalkyl group;
n and n' are independently 0 or an integer from 1 to 10; and
m and m' are independently 0 or an integer from 1 to 10.

2. A compound according to claim 1, wherein R₁ represents a methyl group or an ethyl group; preferably R₁ represents an ethyl group.

3. A compound according to claim 1 or claim 2, wherein R₂ represents an unsubstituted quinolyl or isoquinolyl group.

4. A compound according to any one of the preceding claims, wherein R₃ represents a -(CH₂)ₙC(O)OH group, a -(CH₂)ₙC(O)-R₅ group, a -(CH₂)ₙC(O)NH₂ group,
a -(CH₂)ₙC(O)NH-(CH₂)ₘ-R₅ group, a -(CH₂)ₙC(O)NH-(CH₂)ₘ-C(O)R₆ group or
a -(CH₂)ₙC(O)NH-(CH₂)ₘ-NR₅R₇ group; wherein
R₅ represents a linear or branched C₁-C₃ alkyl group, a C₁-C₃ hydroxyalkyl group, a C₃-C₇ cycloalkyl group, a phenyl group or a 5- to 10-membered heterocyclyl group containing 1, 2 or 3 heteroatoms selected from O, S and N,
wherein the cycloalkyl and heterocyclyl groups are unsubstituted or substituted by one or two substituents selected from a hydroxyl group, an oxo group, a linear or branched C₁-C₃ alkyl group, a C₁-C₃ alkoxy group, a (C₁-C₃ alkoxy)(C₁-C₂ alkylene) group, a carbamoyl group, a sulfamoyl group, an amino group, a mono-C₁-C₂ alkyl amino group or a di-C₁-C₂ alkyl amino group; and the phenyl groups are unsubstituted or substituted by one or two substituents selected from a hydroxyl group, a linear or branched C₁-C₃ alkyl group, a C₁-C₃ alkoxy group, a (C₁-C₃ alkoxy)(C₁-C₂ alkylene) group, a carbamoyl group, a sulfamoyl group, an amino group, a mono-C₁-C₂ alkyl amino group or a di-C₁-C₂ alkyl amino group;
R₆ represents a hydroxyl group or an amino group;
R₇ represents a linear or branched C₁-C₃ alkyl group or a C₁-C₃ hydroxyalkyl group;
n is 0 or an integer from 1 to 3; and
m is 0 or an integer from 1 to 8.

5. A compound according to any one of the preceding claims, wherein R₄ represents a hydrogen atom, a hydroxyl group or a -(CH₂)_{n'}C(O)NH-(CH₂)_{m'}-R₈ group, wherein n' and m' are 0, 1 or 2, and wherein R₈ is a C₃-C₇ cycloalkyl group, wherein the cycloalkyl group is unsubstituted or substituted by one or two substituents selected from a hydroxyl group, a linear or branched C₁-C₃ alkyl group, a C₁-C₃ hydroxyalkyl group, a C₁-C₃ alkoxy group, a (C₁-C₃ alkoxy)(C₁-C₂ alkylene) group, a carbamoyl group, a sulfamoyl group, an amino group, a mono-(C₁-C₂ alkyl)amino group or a di-(C₁-C₂ alkyl)amino group; preferably R₄ represents a hydrogen atom, a hydroxyl group or a -C(O)NH-R₈ group wherein R₈ is an unsubstituted C₃-C₇ cycloalkyl group; more preferably R₄ represents a hydrogen atom, a hydroxyl group or a -C(O)NH-R₈, wherein R₈ is a cyclopropyl group, a cyclobutyl group, a cyclopentyl group, a cyclohexyl group or a cycloheptyl group; most preferably R₄ represents a hydrogen atom, a hydroxyl group or a -C(O)NH-R₈ group, wherein R₈ is a cyclopropyl group.

6. A compound according to any one of the preceding claims, wherein R₃ is at the position 3 or position 4 of the phenyl group.

7. A compound according to any one of the preceding claims, wherein R₄ is at the position 3 or position 5 of the phenyl group.

8. A compound according to claim 1, wherein
R₁ represents an ethyl group;
R₂ represents an unsubstituted quinolyl or isoquinolyl group;
R₃ represents a -(CH₂)ₙC(O)OH group, a -(CH₂)ₙC(O)-R₅ group, a -(CH₂)ₙC(O)NH₂ group, a -(CH₂)ₙC(O)NH-(CH₂)ₘ-R₅ group, a -(CH₂)ₙC(O)NH-(CH₂)ₘ-C(O)R₆ group or
a -(CH₂)ₙC(O)NH-(CH₂)ₘ-NR₅R₇ group; wherein n is 0 or an integer from 1 to 3, and m is 0 or an integer from 1 to 8; and wherein
R₅ represents a methyl group, a C₁-C₂ hydroxyalkyl group, a C₃-C₅ cycloalkyl group, a phenyl group, a piperidinyl group, a piperidin-4-yl group, a morpholinyl group, a pyrrolidinyl group, a pyrrolidin-2-yl group or a pyrrolidin-2-one-yl group;
wherein the C₃-C₅ cycloalkyl, piperidinyl, piperidin-4-yl group, morpholinyl group, pyrrolidinyl group, pyrrolidin-2-yl group and pyrrolidin-2-one-yl groups are unsubstituted or substituted by one or two substituents selected from a hydroxyl group, an oxo group, a methyl group, a methoxy group, a methoxy(methylene) group, a carbamoyl group, a sulfamoyl group or a dimethyl amino group; and the phenyl groups are unsubstituted or substituted by one or two substituents selected from a hydroxyl group, a methyl group, a methoxy group, a methoxy(methylene) group, a carbamoyl group, a sulfamoyl group or a di-methyl amino group;
R₆ represents a hydroxyl group or an amino group;
R₇ represents a methyl group or a C₁-C₂ hydroxyalkyl group;
R₄ represents a hydrogen atom, a hydroxyl group or a -C(O)NH-cyclopropyl group;
or a pharmaceutically acceptable salt, or solvate, or N-oxide, or stereoisomer or deuterated derivate thereof.

9. A compound according to claim 1 which is one of:
3'-[1-ethyl-6-oxo-5-(quinolin-5-ylamino)-1,6-dihydropyridazin-3-yl]biphenyl-3-carboxylic acid;
3'-[1-ethyl-5-(isoquinolin-4-ylamino)-6-oxo-1,6-dihydropyridazin-3-yl]biphenyl-3-carboxylic acid;
3'-[1-ethyl-5-(isoquinolin-4-ylamino)-6-oxo-1,6-dihydropyridazin-3-yl]biphenyl-3-carboxamide;
3'-[1-ethyl-6-oxo-5-(quinolin-5-ylamino)-1,6-dihydropyridazin-3-yl]biphenyl-3-carboxamide;
3'-[1-ethyl-5-(isoquinolin-4-ylamino)-6-oxo-1,6-dihydropyridazin-3-yl]biphenyl-4-carboxylic acid;
{3'-[1-ethyl-6-oxo-5-(quinolin-5-ylamino)-1,6-dihydropyridazin-3-yl]biphenyl-4-yl}acetic acid;
3'-{1-ethyl-5-[(1-oxidoquinolin-5-yl)amino]-6-oxo-1,6-dihydropyridazin-3-yl}biphenyl-3-carboxamide;
{3'-[1-ethyl-5-(isoquinolin-4-ylamino)-6-oxo-1,6-dihydropyridazin-3-yl]biphenyl-3-yl}acetic acid;
3'-[1-ethyl-6-oxo-5-(quinolin-5-ylamino)-1,6-dihydropyridazin-3-yl]biphenyl-4-carboxylic acid;
{3'-[1-ethyl-6-oxo-5-(quinolin-5-ylamino)-1,6-dihydropyridazin-3-yl]biphenyl-3-yl}acetic acid;
3'-{1-ethyl-5-[(1-oxidoquinolin-5-yl)amino]-6-oxo-1,6-dihydropyridazin-3-yl}biphenyl-3-carboxylic acid;
N-[2-(dimethylamino)ethyl]-3'-[1-ethyl-6-oxo-5-(quinolin-5-ylamino)-1,6-dihydropyridazin-3-yl]biphenyl-3-carboxamide;
N-cyclopropyl-3'-[1-ethyl-6-oxo-5-(quinolin-5-ylamino)-1,6-dihydropyridazin-3-yl]biphenyl-3-carboxamide;
N-cyclopropyl-3'-[1-ethyl-5-(isoquinolin-4-ylamino)-6-oxo-1,6-dihydropyridazin-3-yl]biphenyl-3-carboxamide;
3'-[1-ethyl-6-oxo-5-(quinolin-5-ylamino)-1,6-dihydropyridazin-3-yl]-N-(2-morpholin-4-ylethyl)biphenyl-3-carboxamide;
3'-[1-ethyl-6-oxo-5-(quinolin-5-ylamino)-1,6-dihydropyridazin-3-yl]-N-(2-piperidin-1-ylethyl)biphenyl-3-carboxamide;
3'-[1-ethyl-5-(isoquinolin-4-ylamino)-6-oxo-1,6-dihydropyridazin-3-yl]-N-(2-morpholin-4-ylethyl)biphenyl-3-carboxamide;
3'-[1-ethyl-5-(isoquinolin-4-ylamino)-6-oxo-1,6-dihydropyridazin-3-yl]-N-(2-piperidin-1-ylethyl)biphenyl-3-carboxamide;
N-cyclobutyl-3'-[1-ethyl-5-(isoquinolin-4-ylamino)-6-oxo-1,6-dihydropyridazin-3-yl]biphenyl-3-carboxamide;
3'-[1-ethyl-5-(isoquinolin-4-ylamino)-6-oxo-1,6-dihydropyridazin-3-yl]-N-(2-morpholin-4-ylethyl)biphenyl-4-carboxamide;
N-cyclopropyl-3'-[1-ethyl-6-oxo-5-(quinolin-5-ylamino)-1,6-dihydropyridazin-3-yl]-5-hydroxybiphenyl-3-carboxamide;
3'-[1-ethyl-5-(isoquinolin-4-ylamino)-6-oxo-1,6-dihydropyridazin-3-yl]-N-(2-piperidin-1-ylethyl)biphenyl-4-carboxamide;
N-cyclobutyl-3'-[1-ethyl-5-(isoquinolin-4-ylamino)-6-oxo-1,6-dihydropyridazin-3-yl]biphenyl-4-carboxamide;
N-cyclopentyl-3'-[1-ethyl-5-(isoquinolin-4-ylamino)-6-oxo-1,6-dihydropyridazin-3-yl]biphenyl-4-carboxamide;
N-cyclobutyl-3'-[1-ethyl-6-oxo-5-(quinolin-5-ylamino)-1,6-dihydropyridazin-3-yl]biphenyl-3-carboxamide;
3'-[1-ethyl-6-oxo-5-(quinolin-5-ylamino)-1,6-dihydropyridazin-3-yl]-N-(3-hydroxybenzyl)biphenyl-3-carboxamide;
N-cyclopentyl-3'-[1-ethyl-6-oxo-5-(quinolin-5-ylamino)-1,6-dihydropyridazin-3-yl]biphenyl-3-carboxamide;
1-({3'-[1-ethyl-6-oxo-5-(quinolin-5-ylamino)-1,6-dihydropyridazin-3-yl]biphenyl-3-yl}carbonyl)piperidine-4-carboxamide;
3'-[1-ethyl-6-oxo-5-(quinolin-5-ylamino)-1,6-dihydropyridazin-3-yl]-N-{7-[(2-hydroxyethyl)(methyl)amino]heptyl}biphenyl-3-carboxamide;
N-cyclopentyl-3'-[1-ethyl-5-(isoquinolin-4-ylamino)-6-oxo-1,6-dihydropyridazin-3-yl]biphenyl-3-carboxamide;
3'-[1-ethyl-5-(isoquinolin-4-ylamino)-6-oxo-1,6-dihydropyridazin-3-yl]-N-(3-hydroxybenzyl)biphenyl-3-carboxamide;
3'-[1-ethyl-5-(isoquinolin-4-ylamino)-6-oxo-1,6-dihydropyridazin-3-yl]-N-{7-[(2-hydroxyethyl)(methyl)amino]heptyl}biphenyl-3-carboxamide;
3-{3'-[1-ethyl-6-oxo-5-(quinolin-5-ylamino)-1,6-dihydropyridazin-3-yl]biphenyl-4-yl}propanoic acid;
3-{3'-[1-ethyl-6-oxo-5-(quinolin-5-ylamino)-1,6-dihydropyridazin-3-yl]biphenyl-3-yl}propanoic acid;
N-cyclopropyl-3'-[1-ethyl-5-(isoquinolin-4-ylamino)-6-oxo-1,6-dihydropyridazin-3-yl]-5-hydroxybiphenyl-3-carboxamide
3'-[1-ethyl-5-(isoquinolin-4-ylamino)-6-oxo-1,6-dihydropyridazin-3-yl]-N-(1-methylpiperidin-4-yl)biphenyl-3-carboxamide;
N-cyclopropyl-3-{3'-[1-ethyl-6-oxo-5-(quinolin-5-ylamino)-1,6-dihydropyridazin-3-yl]biphenyl-4-yl}propanamide;
N-cyclopropyl-3-{3'-[1-ethyl-6-oxo-5-(quinolin-5-ylamino)-1,6-dihydropyridazin-3-yl]biphenyl-3-yl}propanamide;
N-cyclopentyl-3'-[1-ethyl-6-oxo-5-(quinolin-5-ylamino)-1,6-dihydropyridazin-3-yl]biphenyl-4-carboxamide;
2-{3'-[1-ethyl-5-(isoquinolin-4-ylamino)-6-oxo-1,6-dihydropyridazin-3-yl]biphenyl-3-yl}-N-(2-pyrrolidin-1-ylethyl)acetamide;
2-{3'-[1-ethyl-6-oxo-5-(quinolin-5-ylamino)-1,6-dihydropyridazin-3-yl]biphenyl-3-yl}-N-(2-pyrrolidin-1-ylethyl)acetamide;
3'-[1-ethyl-6-oxo-5-(quinolin-5-ylamino)-1,6-dihydropyridazin-3-yl]-N-(2-morpholin-4-ylethyl)biphenyl-4-carboxamide;
3'-[1-ethyl-6-oxo-5-(quinolin-5-ylamino)-1,6-dihydropyridazin-3-yl]-N-(2-piperidin-1-ylethyl)biphenyl-4-carboxamide;
N-(3,4-dimethoxybenzyl)-3'-[1-ethyl-5-(isoquinolin-4-ylamino)-6-oxo-1,6-dihydropyridazin-3-yl]biphenyl-3-carboxamide;
N-cyclobutyl-3'-[1-ethyl-6-oxo-5-(quinolin-5-ylamino)-1,6-dihydropyridazin-3-yl]biphenyl-4-carboxamide;
N-[4-(aminosulfonyl)benzyl]-3'-[1-ethyl-6-oxo-5-(quinolin-5-ylamino)-1,6-dihydropyridazin-3-yl]biphenyl-3-carboxamide;
3'-[1-ethyl-6-oxo-5-(quinolin-5-ylamino)-1,6-dihydropyridazin-3-yl]-N-[3-(2-oxopyrrolidin-1-yl)propyl]biphenyl-3-carboxamide;
3'-[1-ethyl-6-oxo-5-(quinolin-5-ylamino)-1,6-dihydropyridazin-3-yl]-N-[3-(2-oxopyrrolidin-1-yl)propyl]biphenyl-3-carboxamide;
N-({3'-[1-ethyl-6-oxo-5-(quinolin-5-ylamino)-1,6-dihydropyridazin-3-yl]biphenyl-3-yl}carbonyl)glycine;
-{3'-[1-ethyl-5-(isoquinolin-4-ylamino)-6-oxo-1,6-dihydropyridazin-3-yl]biphenyl-4-yl}-N-(2-pyrrolidin-1-ylethyl)acetamide;
2-{3'-[1-ethyl-6-oxo-5-(quinolin-5-ylamino)-1,6-dihydropyridazin-3-yl]biphenyl-4-yl}-N-(2-pyrrolidin-1-ylethyl)acetamide;
N-{2-[1-(dimethylamino)cyclopentyl]ethyl}-3'-[1-ethyl-6-oxo-5-(quinolin-5-ylamino)-1,6-dihydropyridazin-3-yl]biphenyl-3-carboxamide;
4-[({3'-[1-ethyl-6-oxo-5-(quinolin-5-ylamino)-1,6-dihydropyridazin-3-yl]biphenyl-3-yl}carbonyl)amino]piperidine-1-carboxamide;
2-{3'-[1-ethyl-6-oxo-5-(quinolin-5-ylamino)-1,6-dihydropyridazin-3-yl]biphenyl-4-yl}-N-(2-piperidin-1-ylethyl)acetamide;
3'-[1-ethyl-6-oxo-5-(quinolin-5-ylamino)-1,6-dihydropyridazin-3-yl]-N-{2-[(2R)-1-methylpyrrolidin-2-yl]ethyl}biphenyl-3-carboxamide;
6-(3'-{[4-(dimethylamino)piperidin-1-yl]carbonyl}biphenyl-3-yl)-2-ethyl-4-(quinolin-5-ylamino)pyridazin-3(2H)-one;
3'-[1-ethyl-6-oxo-5-(quinolin-5-ylamino)-1,6-dihydropyridazin-3-yl]-N-{2-[(2S)-1-methylpyrrolidin-2-yl]ethyl}biphenyl-3-carboxamide;
3-{3'-[1-ethyl-6-oxo-5-(quinolin-5-ylamino)-1,6-dihydropyridazin-3-yl]biphenyl-4-yl}-N-(2-piperidin-1-ylethyl)propanamide;
3-{3'-[1-ethyl-6-oxo-5-(quinolin-5-ylamino)-1,6-dihydropyridazin-3-yl]biphenyl-4-yl}-N-(1-methylpiperidin-4-yl)propanamide;
3-{3'-[1-ethyl-6-oxo-5-(quinolin-5-ylamino)-1,6-dihydropyridazin-3-yl]biphenyl-4-yl}-N-(2-morpholin-4-ylethyl)propanamide;
N,N'-dicyclopropyl-3'-[1-ethyl-6-oxo-5-(quinolin-5-ylamino)-1,6-dihydropyridazin-3-yl]biphenyl-3,5-dicarboxamide;
N-cyclopropyl-3'-[1-ethyl-6-oxo-5-(quinolin-5-ylamino)-1,6-dihydropyridazin-3-yl]-N'-{3-[(2S)-2-(methoxymethyl)pyrrolidin-1-yl]propyl}biphenyl-3,5-dicarboxamide;
N-cyclopropyl-3'-[1-ethyl-6-oxo-5-(quinolin-5-ylamino)-1,6-dihydropyridazin-3-yl]-N'-{2-[(2S)-2-(methoxymethyl)pyrrolidin-1-yl]ethyl}biphenyl-3,5-dicarboxamide;
and pharmaceutically acceptable salts, solvates, N-oxides or deuterated derivatives thereof.

10. A compound according to any one of claims 1 to 9 for use in the treatment of a pathological condition or disease susceptible to amelioration by inhibition of phosphodiesterase IV.

11. A compound according to claim 10, wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

12. A pharmaceutical composition comprising at least a compound as defined in any one of claims 1 to 9 in association with a pharmaceutically acceptable diluent or carrier.

13. Use of a compound as defined in any one of claims 1 to 9 for the manufacture of a medicament for the treatment of a pathological condition or disease as defined in claims 10 or 11.

14. A method for treating a subject afflicted with a pathological condition or disease as defined in claims 10 or 11, which comprises administering to said subject an effective amount of a compound as defined in any one of claims 1 to 9, or a pharmaceutical composition as defined in claim 12.

15. A combination product comprising
(i) at least a compound as defined in any one of claims 1 to 9, and
(ii) one or more active ingredients selected from the group consisting of (a) β2-adrenergic agonists, (b) anti-cholinergics (c) antiinflammatory agents, (d) anti-allergic agents, (e) immunosuppressants and (f) anti-infectives;
for simultaneous, separate or sequential use in the treatment of the human or animal body.
